# EUROPEAN PATENT SPECIFICATION

(11) **EP 1 625 199 B1**
(45) Date of publication and mention of the grant of the patent: **11.03.2015**
(21) Application number: 04734072.4
(22) Date of filing: 20.05.2004
(51) Int. Cl.: C12N 15/82, C12N 15/29, A01H 5/00

(54) **METHODS OF INCREASING ABIOTIC STRESS TOLERANCE AND/OR BIOMASS IN PLANTS AND PLANTS GENERATED THEREBY**
VERFAHREN ZUR ERHÖHUNG DER ABIOTISCHEN STRESSTOLERANZ UND/ODER BIOMASSE IN PFLANZEN UND DAMIT ERZEUGTE PFLANZEN
PROCEDES D'AUGMENTATION DE LA TOLERANCE DU STRESS ABIOTIQUE DES PLANTES ET PLANTES AINSI PRODUITES

(30) Priority: 22.05.2003 US 472433 P
(43) Date of publication of application: 15.02.2006
(60) Divisional application: 10194223.3
(73) Proprietor: Evogene Ltd., 76121 Rechovot (IL)
(72) Inventor: RONEN, Gil, 33870 Emeq-Hefer (IL); GOLAN, Ezekiel, 69703 Tel Aviv (IL); KARCHI, Hagai, 76 834 Doar Na Emek Soreq (IL); MEISSNER, Rafael, 76 350 Rehovot (IL)
(74) Representative: Vossius & Partner
(86) International application number: PCT/IL2004/000431
(87) International publication number: WO 2004/104162

(56) References cited:
- EP-A- 1 033 405
- EP-A1- 0 905 242
- EP-A1- 1 225 231
- WO-A-93/06710
- WO-A-94/17194
- WO-A-02/090557
- WO-A-03/020025
- WO-A-03/087313
- WO-A-2005/108422
- WO-A2-02/16655
- WO-A2-02/079403
- US-B1- 6 313 375
- US-B1- 6 313 376
- DATABASE EMBL [Online] 20 October 2000 (2000-10-20), "EST428934 tomato nutrient deficient roots Lycopersicon esculentum cDNA clone cLEW26B2 5' sequence, mRNA sequence." XP002446561 retrieved from EBI accession no. EMBL:BF098413 Database accession no. BF098413
- DATABASE EMBL [Online] 31 May 2000 (2000-05-31), "Panax ginseng mRNA for cytoplasmic ribosomal protein S13, complete cds." XP002446562 retrieved from EBI accession no. EMBL:AB043974 Database accession no. AB043974
- DATABASE EMBL [Online] 10 March 2004 (2004-03-10), "Glycine max ribosomal protein S13 (RPS13) mRNA, complete cds." XP002446563 retrieved from EBI accession no. EMBL:AY453393 Database accession no. AY453393
- KIM KEE-YOUNG ET AL: "Molecular cloning of low-temperature-inducible ribosomal proteins from soybean" JOURNAL OF EXPERIMENTAL BOTANY, vol. 55, no. 399, May 2004 (2004-05), pages 1153-1155, XP002446551 ISSN: 0022-0957
- TANAKA SATOSHI ET AL: "Enhanced tolerance against salt-stress and freezing-stress of Escherichia coli cells expressing algal bbc1 gene" CURRENT MICROBIOLOGY, vol. 42, no. 3, March 2001 (2001-03), pages 173-177, XP002446552 ISSN: 0343-8651
- SAEZ-VASQUEZ JULIO ET AL: "Accumulation and nuclear targeting of BnC24, a Brassica napus ribosomal protein corresponding to a mRNA accumulating in response to cold treatment" PLANT SCIENCE (SHANNON), vol. 156, no. 1, 14 July 2000 (2000-07-14), pages 35-46, XP002446553 ISSN: 0168-9452
- DATABASE EMBL [Online] 14 December 1999 (1999-12-14), "EST301294 tomato root during/after fruit set, Cornell University Lycopersicon esculentum cDNA clone cLEX1K11 similar to Vernicia fordii aquaporin, mRNA sequence." XP002455680 retrieved from EBI accession no. EMBL:AW218814 Database accession no. AW218814
- DATABASE EMBL [Online] 14 December 1999 (1999-12-14), "EST301295 tomato root during/after fruit set, Cornell University Lycopersicon esculentum cDNA clone cLEX1K11 similar to Vernicia fordii aquaporin, mRNA sequence." XP002455681 retrieved from EBI accession no. EMBL:AW218815 Database accession no. AW218815
- SMART LAWRENCE B ET AL: "MIP genes are down-regulated under drought stress in Nicotiana glauca" PLANT AND CELL PHYSIOLOGY, vol. 42, no. 7, July 2001 (2001-07), pages 686-693, XP002455676 ISSN: 0032-0781 -& DATABASE EMBL [Online] 2 January 2001 (2001-01-02), "Nicotiana glauca putative delta TIP (MIP2) mRNA, complete cds." XP002455682 retrieved from EBI accession no. EMBL:AF290618 Database accession no. AF290618
- ASPE M.P. ET AL: 'Engineering salt tolerance in plants.' CURRENT OPINION IN BIOTECHNOLOGY. vol. 13, no. 2, 01 April 2002, pages 146 - 150, XP003018468
- DATABASE GENBANK [Online] 18 May 2001 ALCALA J. ET AL: 'Gene sequence.', XP003018470 Database accession no. (AW932839)
- BRANDLE ET AL: 'Perspectives on the production of recombinant proteins in plants.' AGBIOTECHNET. vol. 3, no. ABN070, August 2001, pages 1 - 4, XP008064133

## Description

### FIELD AND BACKGROUND OF THE INVENTION

The present invention relates to methods of increasing abiotic stress tolerance in plants and, more particularly, to plants expressing exogenous abiotic stress-tolerance genes.

Abiotic stress (also referred to as "environmental stress") conditions such as salinity, drought, flood, suboptimal temperature and toxic chemical pollution, cause substantial damage to agricultural plants. Most plants have evolved strategies to protect themselves against these conditions. However, if the severity and duration of the stress conditions are too great, the effects on plant development, growth and yield of most crop plants are profound. Furthermore, most of the crop plants are very susceptible to abiotic stress (ABS) and thus necessitate optimal growth conditions for commercial crop yields. Continuous exposure to stress causes major alterations in the plant metabolism which ultimately lead to cell death and consequently yield losses. Thus, despite extensive research and the use of sophisticated and intensive crop-protection measures, losses due to abiotic stress conditions remain in the billions of dollars annually (1,2).

Developing stress-tolerant plants is a strategy that has the potential to solve or mediate at least some of these problems. However, traditional plant breeding strategies used to develop new lines of plants that exhibit tolerance to ABS are relatively inefficient since they are tedious, time consuming and of unpredictable outcome. Furthermore, limited germplasm resources for stress tolerance and incompatibility in crosses between distantly related plant species represent significant problems encountered in conventional breeding. Additionally, the cellular processes leading to ABS tolerance are complex in nature and involve multiple mechanisms of cellular adaptation and numerous metabolic pathways (4-7).

Genetic engineering efforts, aimed at conferring abiotic stress tolerance to transgenic crops, have been described in the prior art. Studies by Apse and Blumwald (Curr Opin Biotechnol. 13:146-150, 2002), Quesada et al. (Plant Physiol. 130:951-963, 2002), Holmström et al. (Nature 379: 683-684, 1996), Xu et al. (Plant Physiol 110: 249-257, 1996), Pilon-Smits and Ebskamp (Plant Physiol 107: 125-130, 1995) and Tarczynski et al. (Science 259: 508-510, 1993) have all attempted at generating stress tolerant plants.

In addition, several U.S. patents and patent applications also describe polynucleotides associated with stress tolerance and their use in generating stress tolerant plants. U.S. Pat. Nos. 5,296,462 and 5,356,816 describe transforming plants with polynucleotides encoding proteins involved in cold adaptation in *Arabidopsis thaliana,* to thereby promote cold tolerance in the transformed plants.

U.S. Pat. No. 6,670,528 describes transforming plants with polynucleotides encoding polypeptides binding to stress responsive elements, to thereby promote tolerance of the transformed plants to abiotic stress.

U.S. Pat. No. 6,720,477 describes transforming plants with a polynucleotide encoding a signal transduction stress-related protein, capable of increasing tolerance of the transformed plants to abiotic stress.

U.S Application Ser. Nos. 09/938842 and 10/342224 describe abiotic stress-related genes and their use to confer upon plants tolerance to abiotic stress.

U.S. Application Ser. No. 10/231035 describes overexpressing a molybdenum cofactor sulfurase in plants to thereby increase their tolerance to abiotic stress.

U.S. Pat. No. 6,313,375 describes nucleic acids and proteins relating to maize aquaporins and their use in the engineering of plants which are desiccation-, salt-, cold-, or drought-tolerant.

Although the above described studies were at least partially successful in generating stress tolerant plants, there remains a need for stress tolerant genes which can be utilized to generate plants tolerant of a wide range of abiotic stress conditions.

While reducing the present invention to practice, the present inventors have identified through bioinmformatic and laboratory studies several novel abiotic stress-tolerance genes, which can be utilized to increase tolerance to abiotic stress and/or biomass in plants.

### SUMMARY OF THE INTENTION

According to one aspect of the present invention there is provided a method of increasing tolerance of a plant to an abiotic stress. The method includes expressing within the plant an exogenous polynucleotide at least 90% homologous to the polynucleotide set forth by SEQ ID NO: 13.

Also described herein is a method of increasing biomass of a plant. The method includes expressing within the plant an exogenous polynucleotide at least 90% homologous to a polynucleotide selected from the group consisting of SEQ ID NOs: 1-18.

According to yet another aspect of the present invention there is provided a plant cell as characterised by the appended claims.

According to still another aspect of the present invention there is provided a nucleic acid construct, including a polynucleotide and a promoter capable of directing transcription of the polynucleotide in a host cell, as characterised by the appended claims.

Also described herein is an isolated polypeptide, including an amino acid sequence at least 90% homologous to the amino acid sequence encoded by a polynucleotide selected from the group consisting of SEQ ID NOs: 1-18.

According to further features in preferred embodiments of the invention described below, the expressing is effected by (i) transforming a cell of the plant with the exogenous polynucleotide; (ii) generating a mature plant from the cell; and (iii) cultivating the mature plant under conditions suitable for expressing the exogenous polynucleotide within the mature plant.

According to still further features in the described preferred embodiments the transforming is effected by introducing to the plant cell a nucleic acid construct including the exogenous polynucleotide and at least one promoter capable of directing transcription of the exogenous polynucleotide in the plant cell.

According to still further features in the described preferred embodiments the at least one promoter is a constitutive promoter.

According to still further features in the described preferred embodiments the constitutive promoter is CaMV 35S promoter.

According to still further features in the described preferred embodiments the constitutive promoter is At6669 promoter.

According to still further features in the described preferred embodiments the at least one promoter is an inducible promoter.

According to still further features in the described preferred embodiments the inducible promoter is an abiotic stress inducible promoter.

According to still further features in the described preferred embodiments the at least one promoter is a tissue-specific promoter.

According to still further features in the described preferred embodiments the expressing is effected by infecting the plant with a virus including the exogenous polynucleotide.

According to still further features in the described preferred embodiments the virus is an avirulent virus.

According to the invention, the abiotic stress is selected from the group consisting of salinity and water deprivation.

According to still further features in the described preferred embodiments the plant is a dicotyledonous plant.

According to still further features in the described preferred embodiments the plant is a monocotyledonous plant.

According to still further features in the described preferred embodiments the plant cell forms a part of a plant.

The present invention successfully addresses the shortcomings of the presently known configurations by providing methods of utilizing novel abiotic stress-tolerance genes to increase plants tolerance to abiotic stress.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

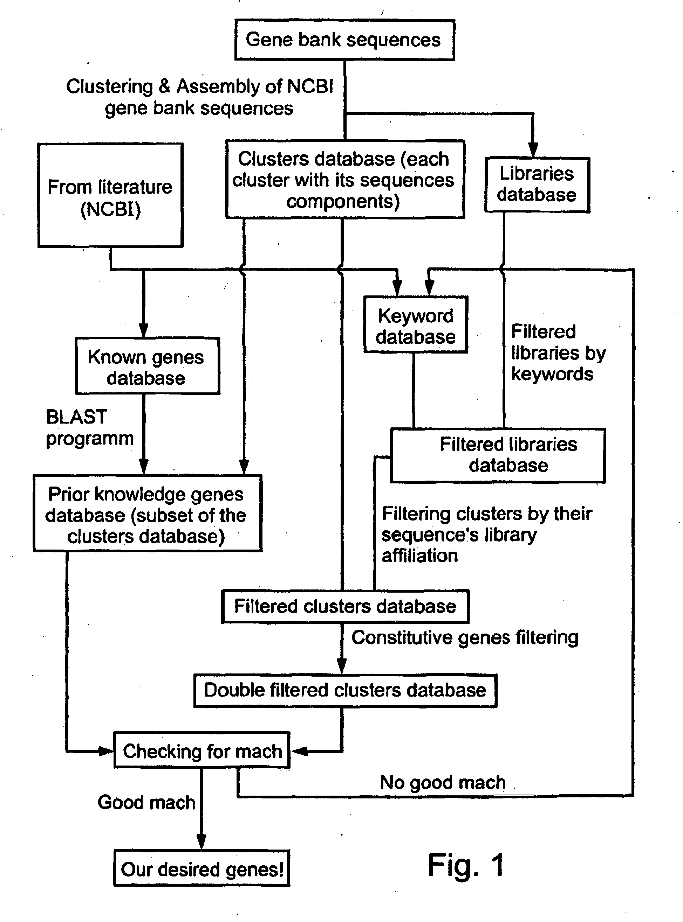

FIG. 1 is a flow chart illustrating a process of identifying putative plant stress-tolerance genes from nucleic-acid sequence databases.

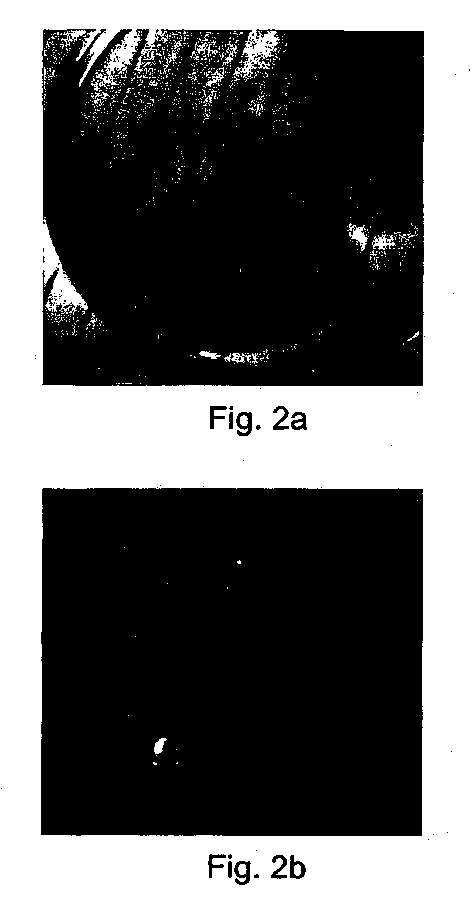

FIGs. 2A-B are photographs illustrating a T2 transgenic *Arabidopsis thaliana* mature plant at flowering stage, expressing erogenous luciferase transgene from the At6669 promoter. The same plant is shown under normal light conditions (Figure 2A) and in the dark (Figure 2B). Strong illumination indicative of luciferase expression is observed in the flower and root tissues.

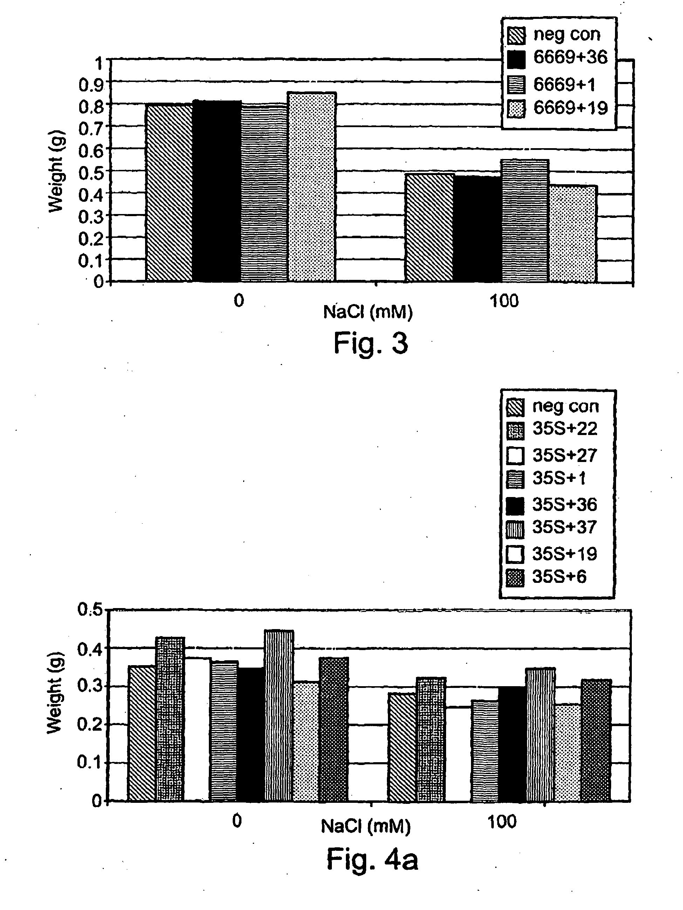

FIG. 3 illustrate the mean fresh weights of transgenic T₁ *A*. *thaliana* plants grown under normal or stress conditions (irrigated with 0 or 100 M NaCl solution, respectively). The plants were transformed with putative stress tolerance genes, or with luciferase reporter gene (control), positioned under the transcriptional control of the At6669 promoter. Means followed by the same letter are not significantly different according to a one way ANOVA T-Test.
FIG. 4A illustrates the mean fresh weights of T₂ *A*. *thaliana* plants grown under normal or stress conditions (irrigated with 0 or 100 M NaCl solution, respectively). The plants were transformed with the putative stress tolerance genes of the present invention, or with luciferase reporter gene (control), positioned under the transcriptional control of the 35S promoter. Means followed by the same letter are not significantly different according to a one way ANOVA T-Test.

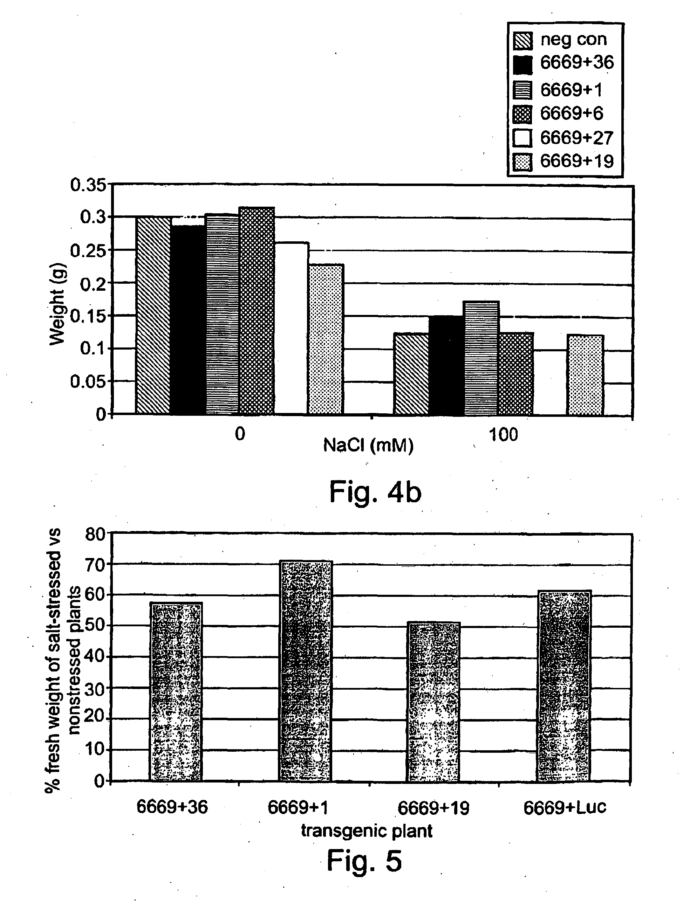

FIG. 4B illustrates the mean fresh weights of T₂ *A. thaliana* plants grown under normal or stress conditions (irrigated with 0 or 100 M NaCl solution, respectively). The plants were transformed with the putative stress tolerance genes of the present invention, or with luciferase reporter gene (control), positioned under the transcriptional control of the At6669 promoter. Means followed by the same letter are not significantly different according to a one way ANOVA T-Test.
FIG. 5 illustrates the relative (percent) fresh-weight of transgenic T₁ *A. thaliana* plants grown under salinity stress conditions (irrigated with 100 mM NaCl solution), as compared with similar plants grown under normal conditions (irrigated with water only). The plants were transformed with the putative stress tolerance genes of the present invention, or with luciferase reporter gene (control), positioned under the transcriptional control of the At6669 promoter.

### DESCRIPTION OF THE PREFERRED EMBODIMENTS

The present invention is of methods of increasing plants tolerance to abiotic stress by utilizing novel abiotic stress tolerance genes and of plants exhibiting increased tolerance to stress conditions.

The principles and operation of the present invention may be better understood with reference to the drawings and accompanying descriptions.

While reducing the present invention to practice, the present inventors while employing bioinformatic techniques, identified polynucleotide sequences which encode putative abiotic-stress tolerance (ABST) proteins (Example 1). Selected sequences were isolated (Example 2), cloned into expression vectors (Example 3-4) and introduced into *Ambidopsis thaliana* plants (Example 5). These plants, which were grown under salinity stress conditions, or under normal conditions, exhibited significantly higher biomass as compared with similar plants not carrying the exogenous ABST genes (Example 6).

Thus, according to one aspect of the present invention, there is provided a method of increasing tolerance of a plant to an abiotic stress as characterised by the appended claims. The method includes expressing within the plant an exogenous polynucleotide at least at least 90% homologous to a polynucleotide of SEQ ID NOs: 13. Also described herein is that, the exogenous polynucleotide encodes a polypeptide having an amino acid sequence selected from the group consisting of SEQ ID NOs: 39-92.

The phrase "abiotic stress" used herein refers to an adverse effect on metabolism, growth, reproduction and/or viability of a plant. Accordingly, abiotic stress can be induced by suboptimal environmental growth conditions such as salinity as water deprivation.

The phrase "abiotic stress tolerance" as used herein refers to the ability of a plant to endure an abiotic stress without suffering a substantial alteration in metabolism, growth, productivity and/or viability.

A suitable plant for use with the method of the present invention can be any monocotyledonous or dicotyledonous plant including, but not limited to, maize, wheat, barely, rye, oat, rice, soybean, peanut, pea, lentil and alfalfa, cotton, rapeseed, canola, pepper, sunflower, potato, tobacco, tomato, eggplant, eucalyptus, a tree, an ornamental plant, a perennial grass and a forage crop.

As used herein, the term "exogenous polynucleotide" refers to a nucleic acid sequence which is not naturally expressed within the plant but which, when introduced into the plant either in a stable or transient manner, produces at least one polypeptide product.

Expressing the exogenous polynucleotide within the plant can be effected by transforming one or more cells of the plant with the exogenous polynucleotide, followed by generating a mature plant from the transformed cells and cultivating the mature plant under conditions suitable for expressing the exogenous polynucleotide within the mature plant.

Preferably, the transformation is effected by introducing to the plant cell a nucleic acid construct which includes the exogenous polynucleotide of the present invention and at least one promoter capable of directing transcription of the exogenous polynucleotide in the plant cell. Further details of suitable transformation approaches are provided hereinbelow.

As used herein, the term "promoter" refers to a region of DNA which lies upstream of the transcriptional initiation site of a gene to which RNA polymerase binds to initiate transcription of RNA. The promoter controls where (e.g., which portion of a plant, which organ within an animal, etc.) and/or when (e.g., which stage or condition in the lifetime of an organism) the gene is expressed.

Any suitable promoter sequence can be used by the nucleic acid. Preferably the promoter is a constitutive promoter, a tissue-specific, or an abiotic stress-inducible promoter.

Suitable constitutive promoters include, for example, CaMV 35S promoter (SEQ ID NO: 19; Odell et al., Nature 313:810-812, 1985); Arabidopsis At6669 promoter (SEQ ID NO: 20); maize Ubi 1 (Christensen et al., Plant Sol. Biol. 18:675-689, 1992); rice actin (McElroy et al., Plant Cell 2:163-171,1990); pEMU (Last et al., Theor. Appl. Genet. 81:581-588, 1991); and Synthetic Super MAS (Ni et al., The Plant Journal 7: 661-76, 1995). Other constitutive promoters include those in U.S. Pat. Nos. 5,659,026, 5,608,149; 5.608,144; 5,604,121; 5.569,597: 5.466,785; 5,399,680; 5,268,463; and 5,608,142.

Suitable tissue-specific promoters include, but not limited to, leaf-specific promoters such as described, for example, by Yamamoto et al., Plant J. 12:255-265, 1997; Kwon et al., Plant Physiol. 105:357-67, 1994; Yamamoto et al., Plant Cell Physiol. 35:773-778, 1994; Gotor et al., Plant J. 3:509-18, 1993; Orozco et al., Plant Mol. Biol. 23:1129-1138, 1993; and Matsuoka et al., Proc. Natl. Acad. Sci. USA 90:9586-9590, 1993.

Suitable abiotic stress-inducible promoters include, but not limited to, salt-inducible promoters such as RD29A (Yamaguchi-Shinozalei et al., Mol. Gen. Genet. 236:331-340, 1993); drought-inducible promoters such as maize rab17 gene promoter (Pla et. al., Plant Mol. Biol. 21:259-266, 1993), maize rab28 gene promoter (Busk et. al., Plant J. 11:1285-1295,1997) and maize Ivr2 gene promoter (Pelleschi et. al., Plant Mol. Biol. 39:373-380, 1999); and heat-inducible promoters such as heat tomato hsp80-promoter from tomato (U.S. Pat. No. 5,187,267).

The nucleic acid construct of the present invention preferably further includes an appropriate selectable marker and/or an origin of replication. Preferably, the nucleic acid construct utilized is a shuttle vector, which can propagate both in *E. coli* (wherein the construct comprises an appropriate selectable marker and origin of replication) and be compatible for propagation in cells. The construct according to the present invention can be, for example, a plasmid, a bacmid, a phagemid, a cosmid, a phage, a virus or an artificial chromosome.

The nucleic acid construct of the present invention can be utilized to stably or transiently transform plant cells. In stable transformation, the exogenous polynucleotide is integrated into the plant genome and as such it represents a stable and inherited trait. In transient transformation, the exogenous polynucleotide is expressed by the cell transformed but it is not integrated into the genome and as such it represents a transient trait.

There are various methods of introducing foreign genes into both monocotyledonous and dicotyledonous plants (Potrykus, I., Annu. Rev. Plant. Physiol., Plant. Mol. Biol. (1991) 42:205-225; Shimamoto et al., Nature (1989) 338:274-276).

The principle methods of causing stable integration of exogenous DNA into plant genomic DNA include two main approaches:
(i) Agrobacterium-mediated gene transfer: Klee et al. (1987) Annu. Rev. Plant Physiol. 38:467-486; Klee and Rogers in Cell Culture and Somatic Cell Genetics of Plants, Vol. 6, Molecular Biology of Plant Nuclear Genes, eds. Schell, J., and Vasil, L. K., Academic Publishers, San Diego, Calif. (1989) p. 2-25; Gatenby, in Plant Biotechnology, eds. Kung, S. and Arntzen, C. J., Butterworth Publishers, Boston, Mass. (1989) p. 93-112.
(ii) Direct DNA uptake: Paszkowski et al., in Cell Culture and Somatic Cell Genetics of Plants, Vol. 6, Molecular Biology of Plant Nuclear Genes eds. Schell, J., and Vasil, L. K., Academic Publishers, San Diego, Calif. (1989) p. 52-68; including methods for direct uptake of DNA into protoplasts, Toriyama, K. et al. (1988) Bio/Technology 6:1072-1074. DNA uptake induced by brief electric shock of plant cells: Zhang et al. Plant Cell Rep. (1988) 7:379-384. Fromm et al. Nature (1986) 319:791-793. DNA injection into plant cells or tissues by particle bombardment, Klein et al. Bio/Technology (1988) 6:559-563; McCabe et al. Bio/Technology (1988) 6:923-926; Sanford, Physiol. Plant. (1990) 79:206-209; by the use of micropipette systems: Neuhaus et al., Theor. Appl. Genet. (1987) 75:30-36; Neuhaus and Spangenberg, Physiol. Plant. (1990) 79:213-217; glass fibers or silicon carbide whisker transformation of cell cultures, embryos or callus tissue, U.S. Pat. No. 5,464,765 or by the direct incubation of DNA with germinating pollen, DeWet et al. in Experimental Manipulation of Ovule Tissue, eds. Chapman, G. P. and Mantell, S. H. and Daniels, W. Longman, London, (1985) p. 197-209; and Ohta, Proc. Natl. Acad. Sci. USA (1986) 83:715-719.

The *Agrobacterium* system includes the use of plasmid vectors that contain defined DNA segments that integrate into the plant genomic DNA. Methods of inoculation of the plant tissue vary depending upon the plant species and the *Agrobacterium* delivery system. A widely used approach is the leaf disc procedure which can be performed with any tissue explant that provides a good source for initiation of whole plant differentiation. Horsch et al. in Plant Molecular Biology Manual A5, Kluwer Academic Publishers, Dordrecht (1988) p. 1-9. A supplementary approach employs the *Agrobacterium* delivery system in combination with vacuum infiltration. The *Agrobacterium* system is especially viable in the creation of transgenic dicotyledonous plants.

There are various methods of direct DNA transfer into plant cells. In electroporation, the protoplasts are briefly exposed to a strong electric field. In microinjection, the DNA is mechanically injected directly into the cells using very small micropipettes. In microparticle bombardment, the DNA is adsorbed on microprojectiles such as magnesium sulfate crystals or tungsten particles, and the microprojectiles are physically accelerated into cells or plant tissues.

Following stable transformation plant propagation is exercised. The most common method of plant propagation is by seed. Regeneration by seed propagation, however, has the deficiency that due to heterozygosity there is a lack of uniformity in the crop, since seeds are produced by plants according to the genetic variances governed by Mendelian rules. Basically, each seed is genetically different and each will grow with its own specific traits. Therefore, it is preferred that the transformed plant be produced such that the regenerated plant has the identical traits and characteristics of the parent transgenic plant. Therefore, it is preferred that the transformed plant be regenerated by micropropagation which provides a rapid, consistent reproduction of the transformed plants.

Micropropagation is a process of growing new generation plants from a single piece of tissue that has been excised from a selected parent plant or cultivar. This process permits the mass reproduction of plants having the preferred tissue expressing the fusion protein. The new generation plants which are produced are genetically identical to, and have all of the characteristics of, the original plant. Micropropagation allows mass production of quality plant material in a short period of time and offers a rapid multiplication of selected cultivars in the preservation of the characteristics of the original transgenic or transformed plant. The advantages of cloning plants are the speed of plant multiplication and the quality and uniformity of plants produced.

Micropropagation is a multi-stage procedure that requires alteration of culture medium or growth conditions between stages. Thus, the micropropagation process involves four basic stages: Stage one, initial tissue culturing; stage two, tissue culture multiplication; stage three, differentiation and plant formation; and stage four, greenhouse culturing and hardening. During stage one, initial tissue culturing, the tissue culture is established and certified contaminant-free. During stage two, the initial tissue culture is multiplied until a sufficient number of tissue samples are produced to meet production goals. During stage three, the tissue samples grown in stage two are divided and grown into individual plantlets. At stage four, the transformed plantlets are transferred to a greenhouse for hardening where the plants' tolerance to light is gradually increased so that it can be grown in the natural environment.

Preferably, mature transformed plants generated as described above are further selected for abiotic stress tolerance. Accordingly, transformed and non-transformed (wild type) plants are exposed to an abiotic stress condition, such as water deprivation, or preferably a salt stress condition. Salt stress can be effected in many ways such as, for example, by irrigating the plants with a hyperosmotic solution, by cultivating the plants hydroponically in a hyperosmotic growth solution (e.g., Hoagland solution), or by culturing the plants in a hyperosmotic growth medium (e.g., MS medium). Since different plants vary considerably in their tolerance to salinity, the salt concentration in the irrigation water, growth solution, or growth medium is preferably adjusted according to the specific characteristics of the specific plant cultivar or variety, so as to inflict a mild or moderate effect on the physiology and/or morphology of the plants (for guidelines as to appropriate concentration please see, Bernstein and Kafkafi, Root Growth Under Salinity Stress In: Plant Roots, The Hidden Half 3rd ed. Waisel Y, Eshel A and Kafkafi U. (editors) Marcel Dekker Inc., New York, 2002, and reference therein). Following exposure to the stress condition the plants are frequently monitored until substantial physiological and/or morphological effects appear in wild type plants. Subsequently, transformed plants not exhibiting substantial physiological and/or morphological effects, or exhibiting higher biomass than wild-type plants, are identified as abiotic stress tolerant plants.

Although stable transformation is presently preferred, transient transformation of leaf cells, meristematic cells or the whole plant is also envisaged by the present invention.

Transient transformation can be effected by any of the direct DNA transfer methods described above or by viral infection using modified plant viruses.

Viruses that have been shown to be useful for the transformation of plant hosts include CaMV, TMV and BV. Transformation of plants using plant viruses is described in U.S. Pat. No. 4,855,237 (BGV), EP-A 67,553 (TMV), Japanese Published Application No. 63-14693 (TMV), EPA 194,809 (BV), EPA 278,667 (BV); and Gluzman, Y. et al., Communications in Molecular Biology: Viral Vectors, Cold Spring Harbor Laboratory, New York, pp. 172-189 (1988). Pseudovirus particles for use in expressing foreign DNA in many hosts, including plants, is described in WO 87/06261.

Preferably, the virus is avirulent and thus is incapable of causing severe symptoms such as reduced growth rate, mosaic, ring spots, leaf roll, yellowing, streaking, pox formation, tumor formation and pitting. A suitable avirulent virus may be a naturally occurring avirulent virus or an artificially attenuated virus. Virus attenuation may be effected by using methods well known in the art including, but not limited to, sub-lethal heating, chemical treatment or by directed mutagenesis techniques such as described, for example, by Kurihara and Watanabe (Molecular Plant Pathology 4:259-269, 2003), Gal-on *et al.* (1992), Atreya *et al.* (1992) and Huet *et al.* (1994).

Suitable virus strains can be obtained from available sources such as, for example, the American Type culture Collection (ATCC) or by isolation from infected plants. Isolation of viruses from infected plant tissues can be effected by techniques well known in the art such as described, for example by Foster and Tatlor, Eds. "Plant Virology Protocols: From Virus Isolation to Transgenic Resistance (Methods in Molecular Biology (Humana Pr), Vol 81)", Humana Press, 1998. Briefly, tissues of an infected plant believed to contain a high concentration of a suitable virus, preferably young leaves and flower petals, are ground in a buffer solution (e.g., phosphate buffer solution) to produce a virus infected sap which can be used in subsequent inoculations.

Construction of plant RNA viruses for the introduction and expression of non-viral exogenous polynucleotide sequences in plants is demonstrated by the above references as well as by Dawson, W. O. et al., Virology (1989) 172:285-292; Takamatsu et al. EMBO J. (1987) 6:307-311; French et al. Science (1986) 231:1294-1297; and Takamatsu et al. FEBS Letters (1990) 269:73-76.

When the virus is a DNA virus, suitable modifications can be made to the virus itself. Alternatively, the virus can first be cloned into a bacterial plasmid for ease of constructing the desired viral vector with the foreign DNA. The virus can then be excised from the plasmid. If the virus is a DNA virus, a bacterial origin of replication can be attached to the viral DNA, which is then replicated by the bacteria. Transcription and translation of this DNA will produce the coat protein which will encapsidate the viral DNA. If the virus is an RNA virus, the virus is generally cloned as a cDNA and inserted into a plasmid. The plasmid is then used to make all of the constructions. The RNA virus is then produced by transcribing the viral sequence of the plasmid and translation of the viral genes to produce the coat protein(s) which encapsidate the viral RNA.

Construction of plant RNA viruses for the introduction and expression in plants of non-viral exogenous polynucleotide sequences such as those included in the construct of the present invention is demonstrated by the above references as well as in U.S. Pat. No. 5,316,931.

In one embodiment, a plant viral polynucleotide is provided in which the native coat protein coding sequence has been deleted from a viral polynucleotide, a non-native plant viral coat protein coding sequence and a non-native promoter, preferably the subgenomic promoter of the non-native coat protein coding sequence, capable of expression in the plant host, packaging of the recombinant plant viral polynucleotide, and ensuring a systemic infection of the host by the recombinant plant viral polynucleotide, has been inserted. Alternatively, the coat protein gene may be inactivated by insertion of the non-native polynucleotide sequence within it, such that a protein is produced. The recombinant plant viral polynucleotide may contain one or more additional non-native subgenomic promoters. Each non-native subgenomic promoter is capable of transcribing or expressing adjacent genes or polynucleotide sequences in the plant host and incapable of recombination with each other and with native subgenomic promoters. Non-native (foreign) polynucleotide sequences may be inserted adjacent the native plant viral subgenomic promoter or the native and a non-native plant viral subgenomic promoters if more than one polynucleotide sequence is included. The non-native polynucleotide sequences are transcribed or expressed in the host plant under control of the subgenomic promoter to produce the desired products.

In a second embodiment, a recombinant plant viral polynucleotide is provided as in the first embodiment except that the native coat protein coding sequence is placed adjacent one of the non-native coat protein subgenomic promoters instead of a non-native coat protein coding sequence.

In a third embodiment, a recombinant plant viral polynucleotide is provided in which the native coat protein gene is adjacent its subgenomic promoter and one or more non-native subgenomic promoters have been inserted into the viral polynucleotide. The inserted non-native subgenomic promoters are capable of transcribing or expressing adjacent genes in a plant host and are incapable of recombination with each other and with native subgenomic promoters. Non-native polynucleotide sequences may be inserted adjacent the non-native subgenomic plant viral promoters such that the sequences are transcribed or expressed in the host plant under control of the subgenomic promoters to produce the desired product.

In a fourth embodiment, a recombinant plant viral polynucleotide is provided as in the third embodiment except that the native coat protein coding sequence is replaced by a non-native coat protein coding sequence.

The viral vectors are encapsidated by the coat proteins encoded by the recombinant plant viral polynucleotide to produce a recombinant plant virus. The recombinant plant viral polynucleotide or recombinant plant virus is used to infect appropriate host plants. The recombinant plant viral polynucleotide is capable of replication in the host, systemic spread in the host, and transcription or expression of foreign gene(s) (exogenous polynucleotide) in the host to produce the desired protein.

Techniques for inoculation of viruses to plants may be found in Foster and Taylor, eds. "Plant Virology Protocols: From Virus Isolation to Transgenic Resistance (Methods in Molecular Biology (Humana Pr), Vol 81)", Humana Press, 1998; Maramorosh and Koprowski, eds. "Methods in Virology" 7 vols, Academic Press, New York 1967-1984; Hill, S.A. "Methods in Plant Virology", Blackwell, Oxford, 1984; Walkey, D.G.A. "Applied Plant Virology", Wiley, New York, 1985; and Kado and Agrawa, eds. "Principles and Techniques in Plant Virology", Van Nostrand-Reinhold, New York.

In addition to the above, the polynucleotide can also be introduced into a chloroplast genome thereby enabling chloroplast expression.

A technique for introducing exogenous polynucleotide sequences to the genome of the chloroplasts is known. This technique involves the following procedures. First, plant cells are chemically treated so as to reduce the number of chloroplasts per cell to about one. Then, the exogenous polynucleotide is introduced via particle bombardment into the cells with the aim of introducing at least one exogenous polynucleotide molecule into the chloroplasts. The exogenous polynucleotides selected such that it is integratable into the chloroplast's genome via homologous recombination which is readily effected by enzymes inherent to the chloroplast. To this end, the exogenous polynucleotide includes, in addition to a gene of interest, at least one polynucleotide stretch which is derived from the chloroplast's genome. In addition, the exogenous polynucleotide includes a selectable marker, which serves by sequential selection procedures to ascertain that all or substantially all of the copies of the chloroplast genomes following such selection will include the exogenous polynucleotide. Further details relating to this technique are found in U.S. Pat. Nos. 4,945,050; and 5,693,507. A polypeptide can thus be produced by the protein expression system of the chloroplast and become integrated into the chloroplast's inner membrane.

Since abiotic stress tolerance in plants can involve multiple genes acting additively or in synergy (see, for example, in Quesda et al., Plant Physiol. 130:951-063, 2002), the present description also envisages expressing a plurality of exogenous polynucleotides in a single host plant to thereby achieve superior abiotic stress tolerance.

Expressing a plurality of exogenous polynucleotides in a single host plant can be effected by co-introducing multiple nucleic acid constructs, each including a different exogenous polynucleotide, into a single plant cell. The transformed cell can than be regenerated into a mature plant using the methods described hereinabove.

Alternatively, expressing a plurality of exogenous polynucleotides in a single host plant can be effected by co-introducing into a single plant-cell a single nucleic-acid construct including a plurality of different exogenous polynucleotides. Such a construct can be designed with a single promoter sequence which can transcribe a polycistronic message including all the different exogenous polynucleotide sequences. To enable co-translation of the different polypeptides encoded by the polycistronic message, the polynucleotide sequences can be inter-linked via an internal ribosome entry site (IRES) sequence which facilitates translation of polynucleotide sequences positioned downstream of the IRES sequence. In this case, a transcribed polycistronic RNA molecule encoding the different polypeptides described above will be translated from both the capped 5' end and the two internal IRES sequences of the polycistronic RNA molecule to thereby produce in the cell all different polypeptides. Alternatively, the construct can include several promoter sequences each linked to a different exogenous polynucleotide sequence.

The plant cell transformed with the construct including a plurality of different exogenous polynucleotides, can be regenerated into a mature plant, using the methods described hereinabove.

Alternatively, expressing a plurality of exogenous polynucleotides in a single host plant can be effected by introducing different nucleic acid constructs, including different exogenous polynucleotides, into a plurality of plants. The regenerated transformed plants can then be cross-bred and resultant progeny selected for superior abiotic stress tolerance and/or biomass traits, using conventional plant breeding techniques.

Hence, the present specification provides methods of utilizing novel abiotic stress-tolerance genes to increase tolerance to abiotic stress in a wide range of economical plants, safely and cost effectively.

Additional objects, advantages, and novel features of the present invention will become apparent to one ordinarily skilled in the art upon examination of the following examples. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

### EXAMPLE 1

### Identifying putative abiotic stress- tolerance genes

Putative abiotic stress-tolerance (ABST) genes were selected from NCBI databases of tomato expressed sequence tags (ESTs) and cDNAs. The database sequences were clustered and assembled using the LEADS^{™} software (Compugen). Clustering resulted in more than 20,000 clusters, each representing a different gene. An expression profile summary was compiled for each cluster by pooling all keywords included in the sequence records comprising the cluster. The clusters were then screened to include polynucleotides originating from libraries identified by keywords relating to ABST. The selected clusters were further filtered to exclude any cluster which included more than 100 ESTs per cluster and/or any cluster in which less than 50% of the sequences are annotated by ABST-related keywords.

Prior art ABST plant genes were identified from the publications of Quesada et al. (Plant Physiol. 130:951-963, 2002); Apse and Blumwald (Curr Opin Biotechnol. 13:146-150, 2002); Rontein et al. (Metab Eng 4:49-56, 2002); and references therein. Known plant ABST genes were aligned with the clustered tomato nucleic-acid sequences, using the BLAST program. The tomato sequences having an e-score value lower than 5 were identified as ABST orthologes. Additional prior art tomato ABST genes were identified by searching the clustered tomato sequence records using the keywords "root", "crown gall", "nutrient", "callus", "disease", "pathogen", "elicitor" and "pseudomonas".

Finally, all identified prior art ABST genes were matched (by sequence alignment using the BLAST software) with the output set of tomato gene clusters, selected as described above. Consequently, about 40% of the genes selected in the output set of clusters which matched with prior art ABST genes proved to be known ABST genes, indicating that the remaining genes of the selected clusters are potentially capable of increasing abiotic stress tolerance in plants.

The selected polynucleotide sequences (Table 1a), were analyzed for presence of ORFs using Vector NTI suite (InforMax, U.K.) version 6 (Hasting Software, Inc: www.generunner.com/). ORFs identified in each of these polynucleotide sequences were compared to Genbank database sequences, using Blast (www.ncbi.nlm.nih.gov/BLAST/); the ORF displaying the highest homology to a GenBank sequence or sequences, was mapped in order to identify an ATG start codon. The position of the ATG start codon of this ORF was then compared with that of the identified polynucleotide sequence in order to verify that each of the eighteen sequences described herein includes a full length ORF and an ATG start codon (thus qualifies as a "putative ABST gene").

**Table 1a**

| ***Putative ABST genes*** | |
|---|---|
| ABST No. | SEQ ID No. |
| 1 | 1 |
| 3 | 2 |
| 5 | 3 |
| 6 | 4 |
| 10 | 5 |
| 11 | 6 |
| 12 | 7 |
| 19 | 8 |
| 22 | 9 |
| 24 | 10 |
| 26 | 11 |
| 27 | 12 |
| 36 | 13 |
| 37 | 14 |
| 39 T0 | 15 |
| 39 T1 | 16 |
| 49 T0 | 17 |
| 49 T1 | 18 |

ABST polypeptide homologues have been identified from the NCBI databases using BLAST software (Table 1b).

**Table 1h**

| ***ABST homologues*** | | | |
|---|---|---|---|
| **ABST Putative Gene SEQ ID No.** | **ABST Polypeptide Homologue NCBI Accession No.** | **ABST Polypeptide Homologue SEQ ID No.** | **Homology (%)** |
| 1 | BAA96366 | 39 | 98 |
| 1 | AAS47510 | 40 | 98 |
| 1 | NP 567151 | 41 | 97 |
| 1 | NP 567104 | 42 | 96 |
| 1 | AAK55664 | 43 | 96 |
| 1 | P46298 | 44 | 97 |
| 1 | T01338 | 45 | 96 |
| 1 | T47888 | 46 | 95 |
| 1 | BAD09465 | 47 | 92 |
| 1 | Q05761 | 48 | 91 |
| 1 | BAD09464 | 49 | 88 |
| 1 | CAA79496 | 50 | 84 |
| 1 | EAJ94592 | 51 | 85 |
| 4 | NP_188036 | 52 | 76 |
| 4 | NP_035977 | 53 | 70 |
| 4 | XP_342608 | 54 | 69 |
| 4 | T09295 | 55 | 60 |
| 4 | NP_564717 | 56 | 59 |
| 4 | AAM63624 | 57 | 59 |
| 9 | P37707 | 58 | 93 |
| 9 | CAD37200 | 59 | 81 |
| 9 | CAA04664 | 60 | 78 |
| 9 | AAM64572 | 61 | 77 |
| 9 | NP_189345 | 62 | 77 |
| 9 | NP_974979 | 63 | 60 |
| 13 | AAC49992 | 64 | 88 |
| 13 | T10804 | 65 | 87 |
| 13 | AAL38357 | 66 | 87 |
| 13 | NP_188245 | 67 | 87 |
| 13 | NP_193465 | 68 | 87 |
| 13 | AAG44945 | 69 | 86 |
| 13 | T07819 | 70 | 86 |
| 13 | T12632 | 71 | 86 |
| 13 | CAC39073 | 72 | 86 |
| 13 | T01648 | 73 | 86 |
| 13 | AAF90121 | 74 | 86 |
| 13 | S48116 | 75 | 86 |
| 13 | AAO86710 | 76 | 86 |
| 13 | T14002 | 77 | 85 |
| 13 | T14001 | 78 | 85 |
| 13 | T48886 | 79 | 85 |
| 13 | T14314 | 80 | 85 |
| 13 | P33560 | 81 | 85 |
| 13 | P21653 | 82 | 85 |
| 13 | T14000 | 83 | 85 |
| 13 | T48884 | 84 | 85 |
| 13 | P24422 | 85 | 85 |
| 13 | AAB53329 | 86 | 85 |
| 14 | NP_200279 | 87 | 67 |
| 14 | AAM64276 | 88 | 67 |
| 14 | AAO72577 | 89 | 66 |
| 14 | NP_175518 | 90 | 64 |
| 14 | BAC78588 | 91 | 64 |
| 14 | BAD03011 | 92 | 62 |

### EXAMPLE 2

### Isolation of putative ABS tolerance genes

RNA was extracted from 4 week-old tomato root and leaf tissues using Tri Reagent (Molecular Research Center, Inc), following the protocol provided by the manufacturer (www.mrcgene.com/tri.htm). Complementary DNA molecules were produced from the extracted mRNA using M-MuLV reverse-transcriptase (RT) enzyme (Roche) and T₁₆NN DNA primer, according to the manufacturer's instructions. The cDNA sequences set forth in SEQ ID NOs: 1,4,8-9 and 12-14, were amplified by PCR using the primers described in Table 2 below, with PFU proof reading DNA polymerase enzyme (Promega-www.promega.com/pnotes/68/7381_07/7381_07.html), following the protocol provided by the manufacturer. Additional restriction endonuclease sites were added to the 5' prime end of each primer to facilitate cloning of the putative ABS tolerance genes in binary vectors.

**Table 2**

| ***PCR primers used for amplifying putative ABS tolerance (ABST) genes*** | | | | |
|---|---|---|---|---|
| **ABST gene SEQ ID No** | **Forward Primer SEQ ID No** | **Reverse Primer SEQ ID No** | **upstream restriction site** | **downstream restriction site** |
| 1 | 21 | 22 | BamH1 | SacI |
| 4 | 23 | 24 | BamH1 | SacI |
| 8 | 25 | 26 | BamH1 | SacI |
| 9 | 27 | 28 | XbaI | SmaI |
| 12 | 29 | 30 | BamH1 | SacI |
| 13 | 31 | 32 | BamH1 | SacI |
| 14 | 33 | 34 | BamH1 | SmaI |

### EXAMPLE 3

### Cloning the putative ABST genes

The resulting PCR blunt ended products were purified using PCR Purification Kit (Qiagen, Germany), digested with the appropriate restriction enzymes (Roche) and then inserted into the binary plasmid vector pPI. The plasmid pPI was constructed by inserting a synthetic poly-(A) signal sequence, originating from pGL3 basic plasmid vector (Promega, Acc No U47295; bp 4658-4811) into the *Hind*III restriction site of the binary vector pBI101.3 (Clontech, Acc. No. U12640).

The resulting pPI plasmid was digested with restriction enzymes (*Bam*HI and *Sac*I; MBI Fermentas) and purified using PCR Purification Kit (Qiagen, Germany). The open pPI construct was then ligated with each of the seven PCR products described hereinabove. The ligation was effected using a ligation mixture containing T4 DNA ligase enzyme (Roche) and was performed according to the manufacturer's instructions.

The pPI constructs harboring putative ABST genes were introduced to *E. coli* DH5 competent cells by electroporation, using a MicroPulser electroporator (Biorad), 0.2 cm cuvettes (Biorad) and EC-2 electroporation program (Biorad). The treated cells were cultured in LB liquid medium at 37°C for 1 hr, then plated over LB agar supplemented with kanamycin (50 mg/L; Sigma) and incubated at 37°C for 16 hrs. Colonies which developed on the selective medium were analyzed by PCR using the primers set forth in SEQ ID NOs: 35-36, which were designed to span the inserted sequence in the pPI plasmid. The resulting PCR products were separated on 1.5% agarose gels and the DNA fragment having the predicted size were isolated and sequenced using the ABI 377 sequencer (Amersham Biosciences Inc) in order to verify that the correct DNA sequences were properly introduced to the *E. coli* cells.

### EXAMPLE 4

### Generating binary vectors comprising putative ABST genes and plant promoters operably linked thereto

***Generating binary vectors comprising the Cauliflower Mosaic Virus 35S promoter:*** The Cauliflower Mosaic Virus 35S promoter sequence (set forth in SEQ ID NO: 19) was inserted upstream of the putative ABST gene in each of the pPI constructs described above. The promoter was isolated from the pBI121 plasmid (Clontech, Accession No. AF485783) using the restriction endonucleases *Hind*III and *Bam*HI (Roche). The isolated promoter was ligated into the pPI constructs digested with the same enzymes. Altogether, seven pPI constructs were generated, each comprising the CaMV 35S promoter positioned upstream of a putative ABST gene having a sequence set forth in SEQ ID NO: 1,4,8,9,12,13 or 14.

***Generating binary vectors comprising the At6669 promoter:*** The At6669 promoter sequence (set forth in SEQ ID NO: 20) was inserted upstream of the putative ABST gene in each of the pPI binary constructs described above. The promoter was isolated from *Arabidopsis thaliana* var Col0 genomic DNA by PCR amplification using the primers set forth in SEQ ID NOs: 37-38. The PCR product was purified (Qiagen, Germany) and digested with the restriction endonucleases *Hind*III and *Bam*HI (Roche). The resulting promoter sequence was introduced into the open binary constructs digested with the same enzymes. Altogether, seven pPI constructs were generated, each comprising the At6669 promoter positioned upstream of a putative ABST gene having a sequence set forth in SEQ ID NO: 1,4,8,9,12,13 or 14.

### EXAMPLE 5

### Confirming At6669 promoter activity in transgenic Arabidopsis thaliana

The capacity of At-6669 promoter to regulate transcription of genes carried by the pPI vector in plants was tested. Accordingly, the promoter At6669 was inserted into the pPI binary vector upstream of a Luciferase reporter gene. The binary vector was introduced to *Arabidopsis thaliana* plants using the procedure as described in Example 6 below. Mature transformed T₂ *Arabidopsis* plants were assayed for bio-illumination in a darkroom using an ultra-low light detection camera (Princeton Instruments Inc., USA) using the procedure described by Meissner *et al.* (Plant J. 22:265, 2000). Illumination indicating positive Luciferase activity was observed in the flower and root meristem tissues of transformed plants (Figure 2).

### EXAMPLE 6

### Transforming Agrobacterium tumefaciens cells with binary vectors harboring putative ABST genes

Each of the binary vectors described in Example 4 above were used to transform *Agrobacterium* cells. Two additional binary constructs, having the Luciferase reporter gene replacing an ABST gene (positioned downstream of the 35S or At6669 promoter), were used as negative controls.

The binary vectors were introduced to *Agrobacterium tumefaciens* GV301, or LB4404 competent cells (about 10⁹ cells/mL) by electroporation. The electroporation was effected by using a MicroPulser electroporator (Biorad), 0.2 cm cuvettes (Biorad) and EC-2 electroporation program (Biorad). The treated cells were cultured in LB liquid medium at 28°C for 3 hr, then plated over LB agar supplemented with gentamycin (50 mg/L; for *Agrobacterium* strains GV301) or streptomycin (300 mg/L; for *Agrobacterium* strain LB4404) and kanamycin (50 mg/L) at 28°C for 48 hrs. *Abrobacterium* colonies which developed on the selective media were analyzed by PCR using the primers set forth in SEQ ID NOs: 35-36, which were designed to span the inserted sequence in the pPI plasmid. The resulting PCR products were isolated and sequenced as described in Example 4 above, to verify that the correct ABST sequences were properly introduced to the *Agrobacterium* cells.

### EXAMPLE 7

### Transformation of Arabidopsis thaliana plants with putative ABSTgenes

*Arabidopsis thaliana* Columbia plants (T₀ plants) were transformed using the Floral Dip procedure described by Clough and Bent (10) and by Desfeux *et al.* (11), with minor modifications. Briefly, T₀ Plants were sown in 250 ml pots filled with wet peat-based growth mix. The pots were covered with aluminum foil and a plastic dome, kept at 4°C for 3-4 days, then uncovered and incubated in a growth chamber at 18-24°C under 16/8 hr light/dark cycles. The T₀ plants were ready for transformation six days before anthesis.

Single colonies of *Agrobacterium* carrying the binary constructs, generated as described in Example 6 above, were cultured in LB medium supplemented with kanamycin (50 mg/L) and gentamycin (50 mg/L). The cultures were incubated at 28°C for 48 hrs under vigorous shaking and then centrifuged at 4000 rpm for 5 minutes. The pellets comprising *Agrobacterium* cells were re-suspended in a transformation medium containing half-strength (2.15 g/L) Murashig-Skoog (Duchefa); 0.044 µM benzylamino purine (Sigma); 112 µg/L B5 Gambourg vitamins (Sigma); 5% sucrose; and 0.2 ml/L Silwet L-77 (OSI Specialists, CT) in double-distilled water, at pH of 5.7.

Transformation of T₀ plants was effected by inverting each plant into an *Agrobacterium* suspension, such that the above ground plant tissue was submerged for 3-5 seconds. Each inoculated T₀ plant was immediately placed in a plastic tray, then covered with clear plastic dome to maintain humidity and was kept in the dark at room temperature for 18 hrs, to facilitate infection and transformation. Transformed (transgenic) plants were then uncovered and transferred to a greenhouse for recovery and maturation. The transgenic T₀ plants were grown in the greenhouse for 3-5 weeks until siliques were brown and dry. Seeds were harvested from plants and kept at room temperature until sowing.

For generating T₁ and T₂ transgenic plants harboring the genes, seeds collected from transgenic T₀ plants were surface-sterilized by soaking in 70% ethanol for 1 minute, followed by soaking in 5% sodium hypochloride and 0.05% triton for 5 minutes. The surface-sterilized seeds were thoroughly washed in sterile distilled water then placed on culture plates containing half-strength Murashig-Skoog (Duchefa); 2% sucrose; 0.8% plant agar; 50 mM kanamycin; and 200 mM carbenicylin (Duchefa). The culture plates were incubated at 4°C for 48 hours then transferred to a growth room at 25°C for an additional week of incubation. Vital T₁ *Arabidopsis* plants were transferred to a fresh culture plates for another week of incubation. Following incubation the T₁ plants were removed from culture plates and planted in growth mix contained in 250 ml pots. The transgenic plants were allowed to grow in a greenhouse to maturity. Seeds harvested from T₁ plants were cultured and grown to maturity as T₂ plants under the same conditions as used for culturing and growing the T₁ plants.

### EXAMPLE 8

### Evaluating growth of transgenic plants cultivated under abiotic stress conditions Methods:

T₁ or T₂ transgenic plants generated as described above were individually transplanted into pots containing a growth mixture of peat and vermiculite (volume ratio 3:2, respectively). The pots were covered for 24 hr period for hardening, then placed in the greenhouse in complete random order and irrigated with tap water (provided from the pots' bottom every 3-5 days) for seven days. Thereafter, half of the plants were irrigated with a salt solution (100 mM NaCl and 5 mM CaCl₂) to induce salinity stress (stress conditions). The other half of the plants were continued to be irrigated with tap water (normal conditions). All plants were grown in the greenhouse at 100% RH for 28 days, then harvested (the above ground tissue) and weighted (immediately or following drying in oven at 50°C for 24 hr).

### Results:

No significant differences in plant fresh weights were observed between T₁ plants transformed with 3 different ABST genes and plants transformed with the Luciferase reporter gene, grown either under normal or stress conditions (Figure 3 and Table 3 below). Yet, T₁ plants transformed with SEQ ID NO: 1 positioned under the regulatory control of the At6669 promoter maintained 71 % of their fresh weight when exposed to stress conditions, while the control plants (carrying Luciferase gene positioned under the regulatory control of the AT6669 promoter) maintained only 61% of their fresh weight under similar stress conditions.

**Table 3**

| ***Fresh weight of T₁ transgenic Arabidopsis plants irrigated with water or salt solution*** | | | | | |
|---|---|---|---|---|---|
| **Transgene (SEQ ID NO)** | **Promoter** | **N Rows¹** | **Irrigation solution (mM NaCl)** | **Mean (g)** | **Std Error** |
| Luciferase | At6669 | 2 | 0 | 0.7925 | 0.0275 |
| Luciferase | At6669 | 2 | 100 | 0.485 | 0.045 |
| 13 | At6669 | 8 | 0 | 0.81625 | 0.020305 |
| 13 | At6669 | 8 | 100 | 0.4725 | 0.029246 |
| 1 | At6669 | 8 | 0 | 0.7875 | 0.026032 |
| 1 | At6669 | 8 | 100 | 0.55875 | 0.044699 |
| 8 | At6669 | 8 | 0 | 0.8575 | 0.023088 |
| 8 | At6669 | 8 | 100 | 0.440625 | 0.011198 |

| | | | | | |
|---|---|---|---|---|---|
| ¹N Rows represent number of independent transformation event plants measured. For each transgene, 3-5 independent transformation events with 1-3 plants per a single transformation event were used. | | | | | |

T₂ plants transformed with SEQ ID NOs: 7 or 14 positioned under the regulatory control of the 35S promoter accumulated significantly higher biomass than control plants, regardless of growth conditions. As shown in Figure 4A and Table 4 below, the mean fresh weight of plants transformed with SEQ ID NOs: 7 and 14, grown under stress conditions, were 15% and 24%, respectively, higher than the mean fresh weight control plants grown under similar stress conditions. Similarly, the mean fresh weight of plants transformed with SEQ ID NOs: 7 or 14, grown under normal conditions, were 21% and 27%, respectively, higher than the mean fresh weight control plants grown under similar normal conditions.

Similar phenomenon was observed with T₂ plants transformed with SEQ ID NO: 4 positioned under the regulatory control of the 35S promoter. Accordingly, as shown in Figure 4A and Table 4 below, the mean fresh weight of plants transformed with SEQ ID NO: 4 was 14% and 7% was higher than the mean fresh weight of control plants grown under stress and normal conditions, respectively. Similarly, T₂ plants transformed with SEQ ID NO: 4 positioned under the regulatory control of the At6669 promoter exhibited 1.3 and 5% higher biomass than control plants grown under stress and normal conditions, respectively. However, these differences were not found statistically different under the experimental conditions.

**Table 4**

| ***Fresh weight of T₂ transgenic Arabidopsis plants irrigated with water or salt solution*** | | | | | |
|---|---|---|---|---|---|
| **Transgene (SEQ ID NO)** | **Promoter** | **N Rows** | **Irrigation solution (mM NaCl)** | **Mean (g)** | **Std Error** |
| Luciferase | CaMV-35S | 11 | 0 | 0.352727 | 0.011208 |
| Luciferase | CaMV-35S | 11 | 100 | 0.280909 | 0.010484 |
| 9 | CaMV-35S | 11 | 0 | 0.426364 | 0.019599 |
| 9 | CaMV-35S | 11 | 100 | 0.322727 | 0.027306 |
| 12 | CaMV-35S | 11 | 0 | 0.374545 | 0.015746 |
| 12 | CaMV-35S | 11 | 100 | 0.249091 | 0.020647 |
| 1 | CaMV-35S | 8 | 0 | 0.36625 | 0.034171 |
| 1 | CaMV-35S | 8 | 100 | 0.265 | 0.031225 |
| 13 | CaMV-35S | 11 | 0 | 0.349091 | 0.013515 |
| 13 | CaMV-35S | 11 | 100 | 0.293636 | 0.019921 |
| 14 | CaMV-35S | 11 | 0 | 0.446364 | 0.025558 |
| 14 | CaMV-35S | 11 | 100 | 0.348182 | 0.023772 |
| 8 | CaMV-35S | 11 | 0 | 0.310909 | 0.015223 |
| 8 | CaMV-35S | 11 | 100 | 0.253636 | 0.01539 |
| 4 | CaMV-35S | 11 | 0 | 0.379091 | 0.010992 |
| 4 | CaMV-35S | 11 | 100 | 0.318182 | 0.013336 |

| | | | | | |
|---|---|---|---|---|---|
| ¹N Rows represent number of independent transformation event plants measured. For each transgene, 3-5 independent transformation events with 1-3 plants per a single transformation event were used. | | | | | |

T₂ plants transformed with SEQ ID NOs: 1 and 13 positioned under the regulatory control of the At6669 promoter and grown under stress conditions, exhibited significantly higher biomass than control plants grown under similar stress conditions. The mean fresh weight of T₂ plants transformed with SEQ ID NOs: 1 and 13 positioned under the regulatory control of the At6669 promoter, and grown under stress conditions, were 37% and 21 %, respectively, higher than the mean fresh weight control plants grown under similar stress conditions (Figure 4B and Table 5 below). No significant increase in biomass over control was observed when these transgenic plants (carrying SEQ ID NOs: 1 and 13 regulated under At6669 promoter) where grown under normal conditions.

**Table 5**

| ***Fresh weight of T₂ transgenic Arabidopsis plants irrigated with water or salt solution*** | | | | | |
|---|---|---|---|---|---|
| **Transgene (SEQ ID NO)** | **Promoter** | **N Rows** | **Irrigation solution (mM NaCl)** | **Mean (g)** | **Std Error** |
| Luciferase | At6669 | 6 | 0 | 0.3 | 0.010328 |
| Luciferase | At6669 | 6 | 100 | 0.125 | 0.009916 |
| 13 | At6669 | 6 | 0 | 0.286667 | 0.024449 |
| 13 | At6669 | 6 | 100 | 0.151667 | 0.007032 |
| 1 | At6669 | 6 | 0 | 0.305 | 0.03423 |
| 1 | At6669 | 6 | 100 | 0.171667 | 0.012225 |
| 4 | At6669 | 6 | 0 | 0.315 | 0.049983 |
| 4 | At6669 | 6 | 100 | 0.126667 | 0.005578 |
| 12 | At6669 | 6 | 0 | 0.263333 | 0.012824 |
| 12 | At6669 | 6 | 100 | 0.098333 | 0.007923 |
| 8 | At6669 | 6 | 0 | 0.228333 | 0.020235 |
| 8 | At6669 | 6 | 100 | 0.121667 | 0.004014 |

| | | | | | |
|---|---|---|---|---|---|
| ¹N Rows represent number of independent transformation event plants measured. For each transgene, 3-5 independent transformation events with 1-3 plants per a single transformation event were used. | | | | | |

The results illustrate that the isolated putative ABST genes, set forth in SEQ ID NOs: 1 and 13, are capable of increasing plant tolerance to abiotic stress, such as a salinity stress. In addition, the isolated putative ABST genes set forth in SEQ ID NOs: 7, 14 (and possibly also 4), are capable of substantially promoting biomass in plants grown under stress, as well as under normal conditions.

Hence, the results clearly indicate that the putative abiotic stress tolerance genes described herein can be readily isolated and utilized to substantially increase tolerance to abiotic stress and/or biomass in plants.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

All sequences are identified by their accession numbers mentioned in this specification. In addition, citation or identification of any reference in this application shall not be construed as an admission that such reference is available as prior art to the present invention.

### REFERENCES CITED

### (Additional references are cited hereinabove)

1. www.fao.org/ag/agl/agll/spush/degrad.htm.
2. www.fao.org/ag/agl/aglw/watermanagement/introduc.stm
3. McCue KF, Hanson AD (1990). Drought and salt tolerance: towards understanding and application. Trends Biotechnol 8: 358-362.
4. Flowers TJ, Yeo Ar (1995). Breeding for salinity resistance in crop plants: where next? Aust J Plant Physiol 22:875-884.
5. Nguyen BD, Brar DS, Bui BC, Nguyen TV, Pham LN, Nguyen HT (2003). Identification and mapping of the QTL for aluminum tolerance introgressed from the new source, ORYZA RUFIPOGON Griff., into indica rice (Oryza sativa L.). Theor Appl Genet. 106:583-93.
6. Sanchez AC, Subudhi PK, Rosenow DT, Nguyen HT (2002). Mapping QTLs associated with drought resistance in sorghum (Sorghum bicolor L. Moench). Plant Mol Biol. 48:713-26.
7. Quesada V, Garcia-Martinez S, Piqueras P, Ponce MR, Micol JL (2002). Genetic architecture of NaCl tolerance in Arabidopsis. Plant Physiol. 130:951-963.
8. Apse MP, Blumwald E (2002). Engineering salt tolerance in plants. Curr Opin Biotechnol. 13:146-150.
9. Rontein D, Basset G, Hanson AD (2002). Metabolic engineering of osmoprotectant accumulation in plants. Metab Eng 4:49-56
10. Clough SJ, Bent AF (1998). Floral dip: a simplified method for Agrobacterium-mediated transformation of Arabidopsis thaliana. Plant J 16:735-43.
11. Desfeux C, Clough SJ, Bent AF (2000). Female reproductive tissues are the primary target of Agrobacterium-mediated transformation by the Arabidopsis floral-dip method. Plant Physiol 123:895-904.

## Claims

1. A method of increasing tolerance of a plant to an abiotic stress, comprising expressing within the plant an exogenous polynucleotide at least 90% homologous to the polynucleotide set forth by SEQ ID NO: 13, wherein said abiotic stress is selected from the group consisting of water deprivation and salinity, thereby increasing the tolerance of the plant to the abiotic stress as compared to a non-transformed plant.

2. The method of claim 1, wherein said expressing is effected by:
(a) transforming a cell of said plant with said exogenous polynucleotide;
(b) generating a mature plant from said cell; and
(c) cultivating said mature plant under conditions suitable for expressing said exogenous polynucleotide within said mature plant.

3. The method of claim 2, wherein said transforming is effected by introducing to said plant cell a nucleic acid construct including said exogenous polynucleotide and at least one promoter capable of directing transcription of said exogenous polynucleotide in said plant cell.

4. The method of claim 3, wherein said at least one promoter is a constitutive promoter.

5. The method of claim 4, wherein said constitutive promoter is CaMV 35S promoter.

6. The method of claim 4, wherein said constitutive promoter is At6669 promoter.

7. The method of claim 3, wherein said at least one promoter is an inducible promoter.

8. The method of claim 7, wherein said inducible promoter is an abiotic stress inducible promoter.

9. The method of claim 1, wherein said expressing is effected by infecting said plant with a virus including said exogenous polynucleotide.

10. The method of claim 9, wherein said virus is an avirulent virus.

11. The method of claim 1, wherein said plant is a dicotyledonous plant.

12. The method of claim 1, wherein said plant is a monocotyledonous plant.

13. A nucleic acid construct, comprising a polynucleotide at least 90% homologous to the nucleotide sequence set forth by SEQ ID NO: 13 and a promoter capable of directing transcription of the polynucleotide in a host cell, said promoter is CaMV 35S promoter, At6669 promoter or an abiotic stress inducible promoter, wherein said polynucleotide is capable of increasing abiotic stress tolerance in a plant transformed with the nucleic acid construct as compared to a non-transformed plant, wherein said abiotic stress is selected from the group consisting of water deprivation and salinity.

14. A nucleic acid construct comprising the polynucleotide set forth in SEQ ID NO: 13 and a promoter capable of directing transcription of the polynucleotide in a host cell.

15. The nucleic acid construct of claim 14, wherein said promoter is a constitutive promoter.

16. The nucleic acid construct of claim 15, wherein said constitutive promoter is CaMV 35S promoter.

17. The nucleic acid construct of claim 15, wherein said constitutive promoter is At6669 promoter.

18. The nucleic acid construct of claim 14, wherein said promoter is an abiotic stress inducible promoter.

19. A plant cell comprising the nucleic acid construct of any of claims 13-18, wherein said polynucleotide is exogenous to said plant cell.

20. The plant cell of claim 19, wherein said plant cell forms a part of a plant.

21. The plant cell of claim 19, wherein said plant is a dicotyledonous plant.

22. The plant cell of claim 19, wherein said plant is a monocotyledonous plant.

## Patentansprüche

1. Verfahren zum Erhöhen der Toleranz einer Pflanze gegen abiotischen Stress, umfassend die Expression eines exogenen Polynukleotids in der Pflanze, das zu mindestens 90% homolog ist zu dem Polynukleotid gemäß SEQ ID NO:13, wobei der abiotische Stress Wasserentzug oder Salzhaltigkeit ist, wodurch die Toleranz der Pflanze gegen den abiotischen Stress im Vergleich zu einer nichttransformierten Pflanze erhöht wird.

2. Verfahren nach Anspruch 1, wobei die Expression bewirkt wird durch:
(a) Transformation einer Zelle der Pflanze mit dem exogenen Polynukleotid;
(b) Erzeugen einer vollentwickelten Pflanze aus der Zelle; und
(c) Kultivieren der vollentwickelten Pflanze unter Bedingungen, die für die Expression des exogenen Polynukleotids in der vollentwickelten Pflanze geeignet sind.

3. Verfahren nach Anspruch 2, wobei die Transformation dadurch bewirkt wird, dass in die Pflanzenzelle ein Nukleinsäurekonstrukt eingeführt wird, das das exogene Polynukleotid und mindestens einen Promotor enthält, der die Transkription des exogenen Polynukleotids in der Pflanzenzelle steuern kann.

4. Verfahren nach Anspruch 3, wobei der mindestens eine Promotor ein konstitutiver Promotor ist.

5. Verfahren nach Anspruch 4, wobei der konstitutive Promotor der CaMV-35S-Promotor ist.

6. Verfahren nach Anspruch 4, wobei der konstitutive Promotor der At6669-Promotor ist.

7. Verfahren nach Anspruch 3, wobei der mindestens eine Promotor ein induzierbarer Promotor ist.

8. Verfahren nach Anspruch 7, wobei der induzierbare Promotor ein durch abiotischen Stress induzierbarer Promotor ist.

9. Verfahren nach Anspruch 1, wobei das Exprimieren dadurch bewirkt wird, dass die Pflanze mit einem Virus infiziert wird, das das exogene Polynukleotid enthält.

10. Verfahren nach Anspruch 9, wobei das Virus ein avirulentes Virus ist.

11. Verfahren nach Anspruch 1, wobei die Pflanze eine zweikeimblättrige Pflanze ist.

12. Verfahren nach Anspruch 1, wobei die Pflanze eine einkeimblättrige Pflanze ist.

13. Nukleinsäurekonstrukt umfassend ein Polynukleotid, das zu mindestens 90% homolog zu der Nukleotidsequenz gemäß SEQ ID NO:13 ist, und einen Promotor, der die Transkription des Polynukleotids in einer Wirtszelle steuern kann, wobei der Promotor der CaMV-35S-Promotor, der At6669-Promotor oder ein durch abiotischen Stress induzierbarer Promotor ist, wobei das Polynukleotid die Toleranz gegen abiotischen Stress in einer Pflanze, die mit dem Nukleinsäurekonstrukt transformiert ist, im Vergleich zu einer nichttransformierten Pflanze erhöhen kann, wobei der abiotische Stress Wasserentzug oder Salzhaltigkeit ist.

14. Nukleinsäurekonstrukt umfassend das Polynukleotid gemäß SEQ ID NO:13 und einen Promotor, der die Transkription des Polynukleotids in einer Wirtszelle steuern kann.

15. Nukleinsäurekonstrukt nach Anspruch 14, wobei der Promotor ein konstitutiver Promotor ist.

16. Nukleinsäurekonstrukt nach Anspruch 15, wobei der konstitutive Promotor der CaMV-35S-Promotor ist.

17. Nukleinsäurekonstrukt nach Anspruch 15, wobei der konstitutive Promotor der At6669-Promotor ist.

18. Nukleinsäurekonstrukt nach Anspruch 14, wobei der Promotor ein durch abiotischen Stress induzierbarer Promotor ist.

19. Pflanzenzelle, umfassend das Nukleinsäurekonstrukt nach einem der Ansprüche 13 bis 18, wobei das Polynukleotid für die Pflanzenzelle exogen ist.

20. Pflanzenzelle nach Anspruch 19, wobei die Pflanzenzelle Teil einer Pflanze ist.

21. Pflanzenzelle nach Anspruch 19, wobei die Pflanze eine zweikeimblättrige Pflanze ist.

22. Pflanzenzelle nach Anspruch 19, wobei die Pflanze eine einkeimblättrige Pflanze ist.

## Revendications

1. Méthode pour accroître la tolérance d'une plante à un stress abiotique, comprenant l'expression au sein de la plante d'un polynucléotide exogène présentant une homologie d'au moins 90 % avec le polynucléotide représenté par SEQ ID No : 13, dans laquelle ledit stress abiotique est choisi dans le groupe constitué par la privation d'eau et la salinité, pour accroître ainsi la tolérance de la plante au stress abiotique comparée à une plante non transformée.

2. Méthode selon la revendication 1, dans laquelle ladite expression est effectuée par :
(a) la transformation d'une cellule de ladite plante avec ledit polynucléotide exogène ;
(b) la génération d'une plante mature à partir de ladite cellule ; et
(c) la culture de ladite plante mature dans des conditions convenables pour exprimer ledit polynucléotide exogène au sein de ladite plante mature.

3. Méthode selon la revendication 2, dans laquelle ladite transformation est effectuée par introduction dans ladite cellule végétale d'une construction d'acide nucléique comprenant ledit polynucléotide exogène et au moins un promoteur capable de diriger la transcription dudit polynucléotide exogène dans ladite cellule végétale.

4. Méthode selon la revendication 3, dans laquelle ledit au moins promoteur est un promoteur constitutif.

5. Méthode selon la revendication 4, dans laquelle ledit promoteur constitutif est le promoteur 35S du CaMV.

6. Méthode selon la revendication 4, dans laquelle ledit promoteur constitutif est le promoteur At6669.

7. Méthode selon la revendication 3, dans laquelle ledit au moins promoteur est un promoteur inductible.

8. Méthode selon la revendication 7, dans laquelle ledit promoteur inductible est un promoteur inductible par un stress abiotique.

9. Méthode selon la revendication 1, dans laquelle ladite expression est effectuée par infection de ladite plante avec un virus comprenant ledit polynucléotide exogène.

10. Méthode selon la revendication 9, dans laquelle ledit virus est un virus avirulent.

11. Méthode selon la revendication 1, dans laquelle ladite plante est une plante dicotylédone.

12. Méthode selon la revendication 1, dans laquelle ladite plante est une plante monocotylédone.

13. Construction d'acide nucléique, comprenant un polynucléotide présentant une homologie d'au moins 90 % avec la séquence de nucléotides représentée par SEQ ID No : 13 et un promoteur capable de diriger la transcription du polynucléotide dans une cellule hôte, ledit promoteur étant le promoteur 35S du CaMV, le promoteur At6669 ou un promoteur inductible par un stress abiotique, dans laquelle ledit polynucléotide est capable d'accroître la tolérance à un stress abiotique chez une plante transformée avec la construction d'acide nucléique comparée à une plante non transformée, dans laquelle ledit stress abiotique est choisi dans le groupe constitué par la privation d'eau et la salinité.

14. Construction d'acide nucléique comprenant le polynucléotide représenté par SEQ ID No : 13 et un promoteur capable de diriger la transcription du polynucléotide dans une cellule hôte.

15. Construction d'acide nucléique selon la revendication 14, dans laquelle ledit promoteur est un promoteur constitutif.

16. Construction d'acide nucléique selon la revendication 15, dans laquelle ledit promoteur constitutif est le promoteur 35S du CaMV.

17. Construction d'acide nucléique selon la revendication 15, dans laquelle ledit promoteur constitutif est le promoteur At6669.

18. Construction d'acide nucléique selon la revendication 14, dans laquelle ledit promoteur est un promoteur inductible par un stress abiotique.

19. Cellule végétale comprenant la construction d'acide nucléique selon l'une quelconque des revendications 13 à 18, dans laquelle ledit polynucléotide est exogène à ladite cellule végétale.

20. Cellule végétale selon la revendication 19, dans laquelle ladite cellule végétale forme une partie de plante.

21. Cellule végétale selon la revendication 19, dans laquelle ladite plante est une plante dicotylédone.

22. Cellule végétale selon la revendication 19, dans laquelle ladite plante est une plante monocotylédone.


### SEQUENCE LISTING

<110> Ronen, Gil
   Golan, Ezekiel
   Karchi, Hagai
   Meissner, Rafael
<120> METHODS OF INCLEASING ABIOTIC STRESS TOLERANCE AND/OR BIOMASS IN PLANTS
<130> 27910
<160> 92
<170> PatentIn version 3.2
<210> 1
   <211> 456
   <212> DNA
   <213> Lycopersicon esculentum
<400> 1
   atgggtcgta tgcacagtcg tggtaagggt atttcagctt ctgctctccc ttacaagaga     60
   actcctccta gttggctcaa gatctctgct ccagatgttg aggacaacat ctgcaagttc    120
   gctaagaaag gattgacccc ttcacagatt ggtgtgattc ttcgtgattc tcatggaatt    180
   gcacaagtga agagtgttac tggtagcaag atcttgcgta tcctcaaggc acatgggctt    240
   gcacctgaga ttccagagga tttgtaccac ctgattaaga aggctgttgc cattaggaag    300
   catttggaga ggaacaggaa ggataaggat tctaagttcc gtttgatttt ggtggagagc    360
   aggattcatc gccttgctcg ttattacaag aaaacaaaaa agctcccacc tgtctggaaa    420
   tacgaatcta ccactgctag cacacttgtg gcatag                              456
<210> 2
   <211> 1386
   <212> DNA
   <213> Lycopersicon esculentum
<400> 2
   atggctattc ctcttcaatt ctctagtata tccactcgca cagatctctc cttgccggag 60
   actagaactt tcaggttacc gaaacctttc tccgtcatca gatgctccgc cggcgaacct 120
   gttccttcct cgtcggctac tgctgagtca gagttcgatg ctaaggtttt ccggaagaat 180
   ctgaccagaa gtgctaatta caatcgtaaa ggttttggac acaaagaagc tactcttgaa 240
   ctcatgaatc gcgaatatac cagtgatatc atcaagaaat tgaaggagaa tgaatttgag 300
   tatacatggg gaaacgtaac cgtaaaactt gcagagtcct atggtttctg ttggggggtt 360
   gagcgtgcag ttcagattgc ttatgaagcg aggaaacagt ttccaacaga gaggatttgg 420
   ataactaatg aaattattca caaccccact gtgaataaga ggctagagga tatggatgtt 480
   aagaacattc cacttgagga agggaagaaa aactttgatg ttgttgacaa ggatgatgtt 540
   gtggttttgc ctgcttttgg ggctgctgtt gatgaaatgt tggttttgag tgataaaaac 600
   gtacaaattg ttgatacaac ctgcccgtgg gtgactaagg tttggaacac ggttgaaaag 660
   cacaagaagg gagaatatac ctccattatc catggtaaat atgctcatga ggaaactgtt 720
   gcgactgcat cctttgctgg gaaatacatc attgtgaaga acatggcaga ggcaacttat 780
   gtctgtgatt atattcttgg aggtaaactt gatggttcta gctcaaccaa agaggcattt 840
   atgcagaaat ttaaatatgc agtttctgaa gggtttgatc cggatgttga ccttgtaaaa 900
   gctggtattg caaaccaaac aactatgttg aagggagaaa cagaagatat tgggaagttg 960
   gtcgagagga ccatgatgca aaaatatggg gtggaaaatg ttaacaacca cttcgtaagt 1020
   ttcaacacta tatgcgatgc cacacaagag cgtcaagatg caatgtataa gctggttgag 1080
   caaaagctgg atcttatgtt agtgattggt ggctggaact caagtaacac ttcacatcta 1140
   caggagattg cagaggaacg tggaattccc tcatactgga ttgacagtga acagagagta 1200
   ggtcctggaa acaaaataag ttacaagtta atgcatggtg agttggttga gaaagagaac 1260
   ttcttaccgg agggtcctat tacagttggg gtgacatctg gtgcatccac ccccgataag 1320
   gttgttgaag atgtccttat caaggtgttt gatatcaagc gcgaggaagc cttacaattg 1380
   gcctaa 1386
<210> 3
   <211> 2418
   <212> DNA
   <213> Lycopersicon esculentum
<400> 3
   atggctcaac gtgttctaac tcgtgttcac agtcttcgtg aacgtcttga tgctactttg     60
   gatgctcatc gcaatgaaat tttgctcttt ctttcaagga tcgaaagcca cgggaaaggg    120
   atcttgaaac ctcaccagct actggctgag tttgaatcaa ttcagaaaga agacaaagac    180
   aaactgaatg atcatgcctt tgaagaagtc ctgaaatcca ctcaggaagc aattgttttg    240
   cccccatggg ttgcacttgc tattcgtttg aggcccggtg tgtgggaata tgtccgtgtg    300
   aatgttaatg ctcttagtgt tgaggagctg actgtgcctg agtttttgca attcaaggaa    360
   gaacttgtta acggaacttc cagtgataac tttgttcttg aattggattt tgagcccttc    420
   actgcatcat ttccaaaacc aaccctcacg aaatcaattg gaaatggagt tgaattcctc    480
   aacaggcacc tctctgctaa aatgttccat gacaaggaaa gcatgacccc tcttctcgag    540
   tttcttcgag ttcaccacta caatggaaag tcaatgatgc tgaatgatag aattcagaat    600
   ttgtatactc tccaaaaagt cctgaggaag gccgaggaat acctcaccac cctttcgcca    660
   gaaacttcat actcctcatt tgagcacaag ttccaagaaa ttggcttgga gagaggttgg    720
   ggtgacaccg cagagcgtgt tctagagatg atctgcatgc tcctggatct ccttgaggct    780
   cctgactcat gtactcttga gaagttcctt agtagaattc ctatggtttt caatgtagtt    840
   atactttcac ctcatggata tttcgcccag gaaaatgtct tgggttaccc cgacactggt    900
   ggtcaggttg tctatatttt ggatcaagtt cctgccttgg agcgtgagat gctcaagcgc    960
   ataaaggagc aaggacttga tatcaaaccg cgtattctta ttgttactcg gcttctccct   1020
   gatgcagttg gtaccacttg tggtcagcga ctcgagaagg tatttggaac tgagcattca   1080
   catattctta gggtcccctt taggactgaa aagggcattg ttcgcaaatg gatctctcgt   1140
   tttgaagtct ggccatacat ggagactttc attgaggatg tggggaaaga aataaccgca   1200
   gaactgcaag ctaagccaga tcttattatt ggaaactata gtgagggaaa ccttgcagcc   1260
   tccttgttgg ctcacaagtt aggtgtaaca cagtgcacca ttgctcatgc attggagaaa   1320
   accaaatatc ctgattctga catttacttg aacaaatttg acgagaaata ccacttctca   1380
   gctcagttca cagctgatct tatagcaatg aatcatactg atttcattat caccagcacc   1440
   ttccaggaga tagcaggaag caaggacact gttggacagt atgagagcca catggccttc   1500
   acaatgcctg gattgtatag agttgttcat ggcattgatg tgttcgaccc caaattcaac    1560
   attgtgtcac caggagctga tgtgaatctc tatttcccat actccgaaaa ggaaaagaga    1620
   ttgacaactt ttcaccctga aattgaagac ttgctgttta gcgatgttga gaacgaagaa    1680
   cacctgtgtg tgttgaagga caggaataag cccatcatat tcaccatggc aagattggac    1740
   cgagtgaaga acttaactgg acttgtcgag tggtatgcta agaatccacg actaagggag    1800
   ttggttaacc ttgtagtggt tggtggagac cgaagaaagg aatccaaaga cttggaagag    1860
   caggcagaga tgaagaagat gtatgaactt ataaagactc acaatttgaa tggccagttc    1920
   cgatggattt cttcccagat gaaccgcgtg aggaatgggg aactctacag gtacattgct    1980
   gacacaaggg gagctttcgt gcagcctgca ttctacgagg ctttcggtct gactgttgtt    2040
   gaggccatga gctgcggttt gcctacattt gcaactaatc aaggtggtcc agctgagatc    2100
   atcgttcatg gaaagtctgg tttccaaatt gatccatacc atggcgagca ggctgctgat    2160
   ctcctcgctg agttcttcga gaaatgtaag gtagaccctt cacattggga agccatttcc    2220
   aagggtggcc ttaagcgtat acaggagaag tacacatggc aaatctactc cgaccggctg    2280
   ttgacactag ctgctgttta cgggttctgg aagcacgttt ccaagcttga tcgtcttgaa    2340
   attcgtcgtt atcttgagat gttttacgct ctcaaattcc gcaagctggc tgaacttgtc    2400
   ccattggctg ttgagtaa                                                  2418
<210> 4
   <211> 522
   <212> DNA
   <213> Lycopersicon esculentum
<400> 4
   atggaagaca aaagcaatga ttattatgca gttttggggt tgaagaagga atgcactgac    60
   acagaactta ggaatgccta taagaagctt gcactgaaat ggcacccaga tcgctgttca   120
   gcatcgggga atttgaagtt tgtagatgaa gcaaagaagc aatttcaggc aattcaagaa   180
   gcatattctg tgttatcgga tgcaaacaaa aagtttttgt acgatgtagg agtttatgac   240
   tctggtgatg atgacgacga aaatggcatg ggtgatttcc tgaatgaaat ggcagctatg   300
   atgagccaaa ataagtccaa tgaaaatcag ggagaagaaa cctttgagga attgcaggat   360
   atgtttaatg aaatgttcaa cagtgataat ggaacgtttt cttcttcttc ttcttcttct   420
   tcttcttctt ggactggaac tccttcaatg tgctctacta catcatctac atcttcaagt   480
   gagacttttt taacctttcc ccaacaagag aagttcaggt ga                      522
<210> 5
   <211> 1434
   <212> DNA
   <213> Lycoperrsicon esculentum
<400> 5
   atggctactg ctactactct ctctcctgct gatgctgaaa agctcaacaa cctcaaatct 60
   gccgtcgccg gtctaaatca aatcagtgaa aatgagaaat ctggatttat taaccttgtc 120
   ggtcgctatc taagtggtga agcacaacac attgactgga gtaagatcca gacgccaact 180
   gatgaagttg tggtgcctta tgacaagtta gcacctcttt ctgaagatcc cgcggaaact 240
   aagaagcttt tggacaaact tgttgtcctg aagctcaatg gaggcttggg aacaacaatg 300
   ggatgcacgg gtcccaaatc agttattgaa gttcgtaatg gtttgacatt ccttgacttg 360
   attgtcaagc aaattgaggc actcaatgcc aagttcggat gcagtgttcc cctgcttttg 420
   atgaattcgt tcaacaccca cgatgataca ctgaagattg ttgaaaaata tgcaaactca 480
   aacattgata ttcatacatt caatcagagc cagtaccctc gcctggttac tgaagacttt 540
   gccccacttc catgcaaagg caattccgga aaagatggat ggtaccctcc aggtcatggt 600
   gatgttttcc cttctttgat gaatagtgga aagcttgatg cactactagc aaagggcaag 660
   gaatatgtct ttgttgcaaa ctctgataat ttgggcgcca ttgttgattt gaaaatctta 720
   aatcatttga tcctaaacaa aaatgagtac tgcatggagg ttactcccaa aactttagct 780
   gatgtcaaag gtggcacctt aatctcatat gaaggaaaag tacagctatt ggaaatagca 840
   caagtccctg atgaacatgt caatgaattc aagtcaattg aaaaattcaa aattttcaac 900
   accaacaact tgtgggtgag tcttagtgct attaaaagac ttgtagaagc agatgcactc 960
   aagatggaga ttattcccaa cccaaaggaa gtagacggag ttaaagttct tcaacttgaa 1020
   actgctgccg gtgctgcgat taagtttttc gaccgggcaa ttggtgctaa tgttcctcga 1080
   tctcgtttcc ttcccgtgaa agcaacttca gatttgctcc ttgttcagtc tgatctttac 1140
   accttgactg atgagggcta tgtcatccga aacccggcca ggtctaatcc gtccaaccca 1200
   tccatcgagt taggacctga attcaagaag gtggccaact tcttaggccg tttcaagtcc 1260
   attcccagca tcattgatct aggtagcttg aaggtgaccg gtgatgtatg gtttggatct 1320
   agcgttaccc taaaggggaa agtgactgtt gcagccaaat ccggagtgaa gctagaaatt 1380
   ccagatggtg ctgtgattgc aaacaaggac atcaatggac ctgaggatat atag 1434
<210> 6
   <211> 1485
   <212> DNA
   <213> Lycopersicon esculentum
<400> 6
   atggcaaaga gtggcatttt ggtaattgtt tcagctcttg ttgttcttgc agtttgtggt     60
   gtttttgctg aggagaacga atatgtgttg actttggacc attctaacct cactgagact    120
   gttgctaagc acaacttcat tgttgttgaa ttctatgcac cttggtgtgg acactgtaag    180
   agtcttgctc ctgagtatga aaaagctgcc tcagagctga gtagtcatga ccctccaatt    240
   gttctagcta agtatgatgc aaatgatgaa gccaatagag aactttcaaa acagtacgag    300
   atccagggtt tcccaactat taagatattg agagatggag gaaagaaagt tcaagactat    360
   aacggtcctc gtgaagcagc tggtattgta tcctacttga agaaacaagt gggtcctgca    420
   tctgctgaaa tcaagtcgaa ggaagatgcc acaaacctta ttgatgagaa aagtatcttt    480
   gttgttggta tatttccaga cccctccgga gagaaattcg agaactattt aacgctagct    540
   gaaaaactgc gaggcgagtt cgattttgct cacactgttg atgctaaaca cctccctcgg    600
   ggtggaccag tcaacaagcc cactcttcgt cttctaaagc catttgatga actctttgtt    660
   gattttgagg actttgatgt cgatgcaatg gagaagttca tctcagaatc tagtattcct    720
   gttgttacta tttttgacaa tgacccaaac aaccatcctt atgttaacaa gttcttcgaa    780
   ggcaccaacg ccaaggcatt gctatttgtg aactttagct ctgaatttga tgcttttaag    840
   tccaagtaca acgatgttgc tgtgatttac aaaggggatg ggtgagcttt tctcttgggt    900
   gatgttgagg ctggtcaagg tgcttttgag tacttcggac tgaagccgga acaggcacct    960
   gtgatcatca taatggacgc tgatgaacaa aagtatatta aggaccatgt ggaacctgat   1020
   gccattgctg cttacttgaa ggattacaag gaaggaaaac tgaagccaca tgtgaagtca   1080
   gagcccatcc ctgaagtcaa tgacgaacct gttaaggtgg ttgttaggga taccctccag   1140
   gatatggttt acaaatcggg aaaaaatgtg ctgttagagt tctatgcacc ttggtgtggc   1200
   cactgcaaga gtctggctcc aattttggat gaagtggctg tatcatttga aagcgatcct   1260
   gatgttctca ttgcaaaact ggacgcaacc gcaaatgatc tcccgaaagg tgactttgat   1320
   gttcagggat tccctactat gtacttcaga tccgcctctg gtaacttgtc acagtacaat   1380
   ggtgagagaa caaaagaggc tatcatcgaa ttcatcgaga agaatcgtgg caagcctgct   1440
   cagtcagact ctgccaaagt cgattcagca aaggatgaac tttag                   1485
<210> 7
   <211> 250
   <212> DNA
   <213> Lycopersicon esculentum
<400> 7
   atgccgggtg cgctcgcatg gcaggtcaca aaacctaata aagatgcgat tttgtgttcc      60
   ggaggggaga tggtacgggg ttttttgccc gactctcctc tctgtgtctg tctttggcct    120
   cctttctttt ctagaactgt ctgcaagatc tattcagata aacgtctcat caaatttggc    180
   gttaatgtcc ccgaacgatg gtttcgatat ccgcaaagtt atagcagcgc tctacctttt    240
   caatggatag                                                           250
<210> 8
   <211> 717
   <212> DNA
   <213> Lycopersicon esculentum
<400> 8
   atgaattctc agatttgcag atctgctaca agagcagcta agtcactcct ttctgcttca      60
   tctaagcaga cttctcgtgc tttttcagga ggacgagcag cagctgcagc agccacagtt     120
   tcattgagag gagtggtgcc ttctctagcc tcatatggca ggaatgaatc tggaaatgca     180
   tctagagctt ggatttctgg tgtgcttgcc cttcctgcag cagcttacat gctccaggag     240
   caagaagcac atgctgccga gatggagcgc acctttattg ccatcaagcc agatggagta     300
   cagagaggcc tgatttcaga aatcgtatca cggtttgagc gcaagggctt caagctggtt     360
   gcaatcaaag ttgtgattcc ttccaaggaa tttgcaaaga agcactatca tgacttgagt     420
   gagcgaccat tctttaacgg cttgtgcgac ttccttagct ctggccctgt cttagcaatg     480
   gtttgggaag gtgaaggtgt aatcagatat ggaaggaagc ttatcggagc caccgatcca     540
   cagaaatctg aacctggaac catcagaggc gatttagctg ttgtagtagg aaggaacatc     600
   atccatggca gcgatggccc cgagaccgca aaggatgaga tcaacctatg gtttaaacca     660
   gaggagttgg ttaattacac cagcaactct gagaagtggc tatatggtga taactaa        717
<210> 9
   <211> 897
   <212> DNA
   <213> Lycopersicon esculentum
<400> 9
   atggagaata tgcagagcta ttggcaattt ggcgacgagc ttcgaggaca atcaaaagcc      60
   tcagaggatc ataaatggtc aacagctgct ataaaattat ctgaacagat gaagtacaaa     120
   ggtgaacgta ggaataacct tgacctttca aagagctctg ctgaaattag gcccaggggt     180
   aatcatatgt ttcaggaaga taacaagtgg gaaagcctta acttcaatat gttaaatttg     240
   gaaagcaaga tgactgaaaa tatgagcaag aatcgcatta tggatagcat tttcaatgca     300
   aatccagttt atcttaagcc caattttaac agcttgggaa attcatcttt aagcaagttc     360
   aatgctagca actataccaa ggaacctagc aagaataaca ataacaacgt tgagagcaca     420
   aatggaaata actccgttga caaaaggttt aagactctgc ctgctgctga aacactgccg     480
   aagaatgagg ttcttggtgg atatatattt gtttgtaaca atgacacaat gcaggaagac     540
   ctaaagcgcc tgctctttgg ccttcctcct agatacagag attccgtgag ggcaataaca     600
   ccagggttgc ccttgttcct atataattac actactcacc agttgcatgg tatctttgag     660
   gcatcgagtt ttggaggttc caacattgat ccaactgcct gggaggataa aaagtgtaaa     720
   ggagagtcaa ggttccctgc tcaggtgagg atccgtgtcc ggaaagtctg taatcctttg     780
   gaggaagatg ctttcagacc agttttacat cattatgatg gccccaagtt ccgtctggag     840
   ctctccattc ctgagacttt ggacttacta gatctctgtg aaaaagccgg tgtgtag        897
<210> 10
   <211> 489
   <212> DNA
   <213> Lycopersicon esculentum
<400> 10
   atgtcacacc ccaccgcagc tccgctatca ggaccacgcc tctcacaacc ctcgtcgtcg     60
   gcgatgtctc ccttgtacaa acagaaatct tggtcaccgg acacgtttcg cgacgaggcg    120
   tggcagcggc ggaagggtac ccacggaagc tgcctcaaac ggcggagcaa gagcgttacc    180
   gatgaggact ttgatgagat taaggcctgt atcgaattag ggtttggatt tgattcgcca    240
   gaaatggatc agcgattgtc tgatactttt ccggcgtatg acctgtttta cgccgtgaat    300
   aaacaataca ccgacactct ttcaaagact tcctctgtat catcggtcat ctccaattgc    360
   gagtcaaccc ttcctcccgt cagtccccac accattgtct ttccaggaga taatccacag    420
   gcagtgaaga caaggttgcg gcaatgggca caggtggttg cgtgtgtggt gcgtcaatct    480
   tcgtattaa                                                            489
<210> 11
   <211> 489
   <212> DNA
   <213> Lycopersicon esculentum
<400> 11
   atgtcacacc ccaccgcagc tccgctatca ggaccacgcc tctcacaacc ctcgtcgtcg     60
   gcgatgtctc ccttgtacaa acagaaatct tggtcaccgg acacgtttcg cgacgaggcg    120
   tggcagcggc ggaagggtac ccacggaagc tgcctcaaac ggcggagcaa gagcgttacc    180
   gatgaggact ttgatgagat taaggcctgt atcgaattag ggtttggatt tgattcgcca    240
   gaaatggatc agcgattgtc tgatactttt ccggcgtatg acctgtttta cgccgtgaat    300
   aaacaataca ccgacactct ttcaaagact tcctctgtat catcggtcat ctccaattgc    360
   gagtcaaccc ttcctcccgt cagtccccac accattgtct ttccaggaga taatccacag    420
   gcagtgaaga caaggttgcg gcaatgggca caggtggttg cgtgtgtggt gcgtcaatct    480
   tcgtattaa                                                            489
<210> 12
   <211> 606
   <212> DNA
   <213> Lycopersicon esculentum
<400> 12
   atgggatact ggaaagcaaa ggttcttcca aagatcaagc agatctttga taaaaatgga 60
   cccaagaaaa ctgctgctgc tgaggcatgc aagacttttg atcaagctaa ggaggaatat 120
   agcaaggagt ttgaagagaa gaagactgag cttcaaccca aagttgttga aatttatgaa 180
   gctgctgcag ttgagatcaa gagcttagtg aaggaaccaa agggtgcagg gctgaagaaa 240
   aactcagatg gggttcagaa attccttgat gaccttgtca agattgaatt tccgggatca 300
   aaagctgtta gcgaagcaag ttcaaacttt gggccttcct atgtatcggg cccaattatt 360
   tttgtgttcg aaaaagtttc cactttcata gtcacagaag ataagaagga agaggaaccc 420
   gcggctgctg atgacgtgca tgcaccagcc gccacgtcaa ctgaagaggt ggaggtgaag 480
   gtgaaggaga aggagaagga gatagttatt gagtctggag aaccaaacaa ggaagaagca 540
   cctgctactg ttgttgctga cgtggcacct gcaactaagg tggaagaagc accaaaggtg 600
   gtttaa 606
<210> 13
   <211> 750
   <212> DNA
   <213> Lycopersicon esculentum
<400> 13
   atggctggcg gcgtagctat tggaagtttt agtgattcat tcagcgttgt ctctcttaag 60
   tcctatcttg ccgaattcat ctccacactc atctttgtct tcgccggagt tggttccgcc 120
   attgcttacg gcaagttgac aacaaatgct gcacttgatc cggctgggct tgtagctatt 180
   gcagtttgcc atggatttgc tctattcgta gccgtttcga tttccgctaa catctccggt 240
   ggtcatgtta accctgcggt cacctgtgga ttaaccttcg gcggacatat tacctttatc 300
   actggctcct tctacatgct tgctcaactt accggcgccg ctgtagcttg cttcctcctc 360
   aaattcgtca ccggaggatg tgctattcca acccatggag tgggagctgg tgtgagcata 420
   ctagaaggac tcgtgatgga aataataatc acatttggtt tagtttatac tgtgttcgca 480
   accgccgctg acccgaagaa gggttcattg ggcacaattg caccgattgc aattggtctc 540
   attgttggag ctaatatttt ggctgccgga ccattctccg gtggatcaat gaacccagct 600
   cgttcatttg gacctgcaat ggttagtggt aactttgagg gtttctggat ctactggatt 660
   ggtccattag ttggtggtag tttggctggt cttatttaca caaatgtgtt catgacacaa 720
   gaacatgctc ctttatccaa tgagttctaa 750
<210> 14
   <211> 735
   <212> DNA
   <213> Lycopersicon esculentum
<400> 14
   atggaggtcg attctagtgg gaatcctaat tggttatttg attatgagtt gatgacggat 60
   attacttctg ctgcatctgt taccgtcgct gagtttcagt ctccggctac tattgatttc 120
   agctggcctg ctcaaacgat ctatgcttct tctaatctca ttactgaaac agattacaca 180
   tttgcggatt cagaagttag caaggaggca agctcacgaa agcggttaaa aagtgaatgt 240
   tgcagctctc cgagatctaa ggcatgcaga gagaaattgc ggagggacag actgaatgag 300
   aggttcctcg cattgagctc tgtccttgat cctggaaggc caccaaaaac tgagaaagtt 360
   gcaattctaa gtgatgctca aaggatgctg attgagctgc gaactgaaac ccagaagctg 420
   aaggagtcaa atgaggagct gcaagagaag ataaaagaac ttaaggcaga gaagaatgag 480
   ctccgagatg aaaagcaaag gctaaaggaa gaaaaggata atttggagca gcaggttaaa 540
   agcttagctt ctaaagcagg atttctctcc catccttctg ccatgggagc tgcatttact 600
   gcacaaggac aagttgctgc aggcaacaaa ttgatgcctt tcattggtta tcccagygty 660
   gcgatgtggc rattcatgca acctgctgtt gttgacacat ctcaagatca tgtgctccgt 720
   cctccagttg cttaa 735
<210> 15
   <211> 645
   <212> DNA
   <213> Lycopersicon esculentum
<400> 15
   cccttcaaac ctaaaaaaaa gagaaaatca acaaaaaaaa tgggagcttg tgcaacgaag 60
   ccaaaggatt tgaaaggaga tgcaccggaa accgcaccgg aaaatgttcc ggcgactgaa 120
   atcgccacca aggatgcggc ggaagtagcc gtcgccgcca aggatgtggt ggttgtggcg 180
   gaagtagaag tgaaaaagga aattgaggcc gatgctgctg ctgcagatga cgacgatgct 240
   gaaaaacgcc gatctctatc taacttgttc aaagagaacg aagaatgtaa ggggtcagag 300
   caagtaaacg aggaggcatc caagatcaca ccatcagaag ctaagccaga agaagttgag 360
   aaggttgttg atgctcctgt aacttcagag atagaaaaag cactagaagt ggcctcaatt 420
   actgctgagg ctcccaaggt ggagccttcc gaggagaaga agatagatga agtgaaatca 480
   gaacctaaga ctcctgctga gaagaaagta gaggaagcaa aaccggcagt agagactcct 540
   gcagagaaga aaatagaaga ggcaccagtt gctccaactc acgtggagac gaaagccgaa 600
   gatgctccaa aggtaactgt agtagaagaa aagaagtcga gctag 645
<210> 16
   <211> 630
   <212> DNA
   <213> Lycopersicon esculentum
<400> 16
   cccttcaaac ctaaaaaaaa gagaaaatca acaaaaaaaa tgggagcttg tgcaacgaag      60
   ccaaaggatt tgaaaggaga tgcaccggaa accgcaccgg aaaatgttcc ggcgactgaa     120
   atcgccacca aggatgcggc ggaagtagcc gtcgccgcca aggatgtggt ggttgtggcg     180
   gaagtagaag tgaaaaagga aattgaggcc gatgctgctg ctgcagatga cgacgatgct     240
   gaaaaacgcc gatctctatc taacttgttc aaagagaacg aagaatgtaa ggggtcagag     300
   caagtaaacg aggaggcatc caagatcaca ccatcagaag ctaagccaga agaagttgag     360
   aaggttgttg atgctcctgt aacttcagag atagaaaaag cactagaagt ggcctcaatt     420
   actgctgagg ctcccaaggt ggagccttcc gaggagaaga agatagatga agtgaaatca     480
   gaacctaaga ctcctgctga gaagaaagta gaggaagcaa aaccggcagt agagactcct     540
   gcagagaaga aaatagaaga ggcaccagtt gctccaactc acgtggagac gaaagccgaa     600
   gattgcatgg tgaaaaacgc gtggaaatga                                      630
<210> 17
   <211> 216
   <212> DNA
   <213> Lycopersicon esculentum
<400> 17
   atggagataa catgtgcact tgtctgtgag atcttactag caatcttgct tcctcctctt      60
   ggtgtttgcc ttcgcaatgg ttgctgcact gtggagttct tgatttgctt ggtattgacc     120
   atattgggct atgttcctgg aattatctat gctctctatg caatcctctt tgttgaacgt     180
   gaaccaagaa gggattatta tgacactctt gcttga                               216
<210> 18
   <211> 216
   <212> DNA
   <213> Lycopersicon esculentum
<400> 18
   atggactcaa gatgtgcaat tctgtgtgaa gttttactag caatcttgct tcctcctctt      60
   ggtgtttgcc ttcgcaatgg ttgctgcact gtggagttct tgatttgctt ggtattgacc     120
   atattgggct atgttcctgg aattatctat gctctttatg caatcctctg tattcaaagt     180
   gaaccacact accatcatta ccattctctt gcttga                               216
<210> 19
   <211> 877
   <212> DNA
   <213> Cauliflower mosaic virus
<400> 19
   aagcttgcat gcctgcaggt ccccagatta gccttttcaa tttcagaaag aatgctaacc      60
   cacagatggt tagagaggct tacgcagcag gtctcatcaa gacgatctac ccgagcaata     120
   atctccagga aatcaaatac cttcccaaga aggttaaaga tgcagtcaaa agattcagga     180
   ctaactgcat caagaacaca gagaaagata tatttctcaa gatcagaagt actattccag     240
   tatggacgat tcaaggcttg cttcacaaac caaggcaagt aatagagatt ggagtctcta     300
   aaaaggtagt tcccactgaa tcaaaggcca tggagtcaaa gattcaaata gaggacctaa     360
   cagaactcgc cgtaaagact ggcgaacagt tcatacagag tctcttacga ctcaatgaca     420
   agaagaaaat cttcgtcaac atggtggagc acgacacact tgtctactcc aaaaatatca     480
   aagatacagt ctcagaagac caaagggcaa ttgagacttt tcaacaaagg gtaatatccg     540
   gaaacctcct cggattccat tgcccagcta tctgtcactt tattgtgaag atagtggaaa     600
   aggaaggtgg ctcctacaaa tgccatcatt gcgataaagg aaaggccatc gttgaagatg     660
   cctctgccga cagtggtccc aaagatggac ccccacccac gaggagcatc gtggaaaaag     720
   aagacgttcc aaccacgtct tcaaagcaag tggattgatg tgatatctcc actgacgtaa     780
   gggatgacgc acaatcccac tatccttcgc aagacccttc ctctatataa ggaagttcat     840
   ttcatttgga gagaacacgg gggactctag aggatcc                             877
<210> 20
   <211a 2322
   cm DNA
   <213> Arabidopsis thaliana
<400> 20
<210> 21
   <211> 31
   <212> DNA
   <213> Artificial sequence
<220>
   <223> Single strand DNA oligonucleotide
<400> 21
   gggaaggatc catgggtcgt atgcacagtc g 31
<210> 22
   <211> 32
   <212> DNA
   <213> Artificial sequence
<220>
   <223> single strand DNA oligonucleotide
<400> 22
   cgacggagct cctatgccac aagtgtgcta gc 32
<210> 23
   <211> 35
   <212> DNA
   <213> Artificial sequence
<220>
   <223> Single strand DNA oligonucleotide
<400> 23
   gggaaggatc cggtggtatt gaagttatgg aagac 35
<210> 24
   <211> 30
   <212> DNA
   <213> Artificial sequence
<220>
   <223> Single strand DNA oligonucleotide
<400> 24
   gctgcgagct cctaatgctt ccgtccactc 30
<210> 2S
   <211> 34
   <212> DNA
   <213> Artificial sequence
<220>
   <223> Single strand DNA oligonucleotide
<400> 25
   gggaaggatc catgaattct cagatttgca gatc 34
<210> 26
   <211> 36
   <212> DNA
   <213> Artificial sequence
<220>
   <223> Single strand DNA oligonucleotide
<400> 26
   gaggagagct cttagttatc accatatagc cacttc 36
<210> 27
   <211> 33
   <212> DNA
   <213> Artificial sequence
<220>
   <223> Single strand DNA oligonucleotide
<400> 27
   gggaatctag acaacatgga gaatatgcag age 33
<210> 28
   <211> 31
   <212> DNA
   <213> Artificial sequence
<220>
   <223> Single strand DNA oligonucleotide
<400> 28
   agaagcccgg gcacatagca cctacacacc g 31
<210> 29
   <211> 33
   <212> DNA
   <213> Artificial sequence
<220>
   <223> Single strand DNA oligonucleotide
<600> 29
   gggaaggatc cgatgggata ctggaaagca aag 33
<210> 30
   <211> 32
   <212> DNA
   <213> Artificial sequence
<220>
   <223> Single strand DNA oligonucleotide
<400> 30
   agccggagct cttaaaccac ctttggtgct tc 32
<210> 31
   <211> 29
   <212> DNA
   <213> Artificial sequence
<220>
   <223> Single strand DNA oligonucleotide
<400> 31
   gggaaggatc caaatggctg gcggcgtag 29
<210> 32
   <211> 34
   <212> DNA
   <213> Artificial sequence
<220>
   <223> single strand DNA oligonucleotide
<400> 32
   agaaggagct ctagaactca ttggataaag gagc 34
<210> 33
   <211> 32
   <212> DNA
   <213> Artificial sequence
<220>
   <223> Single strand DNA oligonucleotide
<400> 33
   gggaaggatc catggaggtc gattctagtg gg 32
<210> 34
   <211> 32
   <212> DNA
   <213> Artificial sequence
<220>
   <223> Single strand DNA oligonucleotide
<400> 34
   agaagcccgg gttaagcaac tggaggacgg ag 32
<210> 35
   <211> 22
   <212> DNA
   <213> Artificial sequence
<220>
   <223> Single strand DNA oligonucleotide
<400> 35
   ggtggctcct acaaatgcca tc 22
<210> 36
   <211> 20
   <212> DNA
   <213> Artificial sequence
<220>
   <223> Single strand DNA oligonucleotide
<400> 36
   aagttgggta acgccagggt 20
<210> 37
   <211> 22
   <212> DNA
   <213> Artificial sequence
<220>
   <223> Single strand DNA oligonucleotide
<400> 37
   tagtttggtc agatgggaaa cg 22
<210> 38
   <211> 25
   <212> DNA
   <213> Artificial sequence
<220>
   <223> Single strand DNA oligonucleotide
<400> 38
   aaatattgga tcctttgggg ttctc 25
<210> 39
   <211> 151
   <212> PRT
   <213> Panax ginseng
<400> 39
   Met Gly Arg Met His Ser Arg Gly Lys Gly Ile Ser Ala Ser Ala Leu
   1 5 10 15
   Pro Tyr Lys Arg Thr Pro Pro Ser Trp Leu Lys Ile Thr Pro Gln Asp
   20 25 30
   Val Asp Asp Asn Ile Cys Lys Phe Ala Lys Lys Gly Leu Thr Pro Ser
   35 40 45
   Gln Ile Gly Val Ile Leu Arg Asp Ser His Gly Ile Ala Gln Val Lys
   50 55 60
   Ser Val Thr Gly Ser Lys Ile Leu Arg Ile Leu Lys Ala His Gly Leu
   65 70 75 80
   Ala Pro Glu Ile Pro Glu Asp Leu Tyr His Leu Ile Lys Lys Ala Val
   85 90 95
   Ala Ile Arg Lys His Leu Glu Arg Asn Arg Lys Asp Lys Asp Ser Lys
   100 105 110
   Phe Arg Leu Ile Leu Val Glu Ser Arg Ile His Arg Leu Ala Arg Tyr
   115 120 125
   Tyr Lys Lys Thr Lys Lys Leu Pro Pro Val Trp Lys Tyr Glu Ser Thr
   130 135 140
   Thr Ala Ser Thr Leu Val Ala
   145 150
<210> 40
   <211> 151
   <212> PRT
   <213> Glycine max
<400> 40
   Met Gly Arg Met His Ser Arg Gly Lys Gly Ile Ser Ser Ser Ala Leu
   1 5 10 15
   Pro Tyr Lys Arg Thr Pro Pro Ser Trp Leu Lys Ile Ser Ser Gln Asp
   20 25 30
   Val Glu Glu Asn Ile Cys Lys Phe Ala Lys Lys Gly Leu Thr Pro Ser
   35 40 45
   Gln Ile Gly Val Ile Leu Arg Asp Ser His Gly Ile Ala Gln Val Asn
   50 55 60
   Ser Val Thr Gly Ser Lys Ile Leu Arg Ile Leu Lys Ala His Gly Leu
   65 70 75 80
   Ala Pro Glu Ile Pro Glu Asp Leu Tyr His Leu Ile Lys Lys Ala Val
   85 90 95
   Ser Ile Arg Lys His Leu Glu Arg Asn Arg Lys Asp Lys Asp Ser Lys
   100 105 110
   Phe Arg Leu Ile Leu Val Glu Ser Arg Ile His Arg Leu Ala Arg Tyr
   115 120 125
   Tyr Lys Lys Thr Lys Lys Leu Pro Pro Val Trp Lys Tyr Glu Ser Thr
   130 135 140
   Thr Ala Ser Thr Leu Val Ala
   145 150
<210> 41
   <211> 151
   <212> PRT
   <213> Arabidopsis thaliana
<400> 41
<210> 42
   <211> 151
   <212> PRT
   <213> Arabidopsis thaliana
<400> 42
   Met Gly Arg Met His Ser Arg Gly Lys Gly Ile Ser Ala Ser Ala Leu
   1 5 10 15
   Pro Tyr Lys Arg Ser Ser Pro Ser Trp Leu Lys Thr Thr Pro Gln Asp
   20 25 30
   Val Asp Glu Ser Ile Cys Lys Phe Ala Lys Lys Gly Leu Thr Pro Ser
   35 40 45
   Gln Ile Gly Val Ile Leu Arg Asp Ser His Gly Ile Pro Gln Val Lys
   50 55 60
   Ser Val Thr Gly Ser Lys Ile Leu Arg Ile Leu Lys Ala His Gly Leu
   65 70 75 80
   Ala Pro Glu Ile Pro Glu Asp Leu Tyr His Leu Ile Lys Lys Ala Val
   85 90 95
   Ala Ile Arg Lys His Leu Glu Arg Asn Arg Lys Asp Lys Asp Ser Lys
   100 105 110
   Phe Arg Leu Ile Leu Val Glu Ser Arg Ile His Arg Leu Ala Arg Tyr
   115 120 125
   Tyr Lys Lys Thr Lys Lys Leu Pro Pro Val Trp Lys Tyr Glu Ser Thr
   130 135 140
   Thr Ala Ser Thr Leu Val Ala
   145 150
<210> 43
   <211> 151
   <212> PRT
   <213> Arabidopsis thaliana
<400> 43
   Met Gly Arg Met His Ser Arg Gly Lys Gly Ile Ser Ala Ser Ala Leu
   1               5                   10                  15
   Pro His Lys Arg Ser Ser Pro Ser Trp Leu Lys Thr Thr Pro Gln Asp
               20                  25                  30
   Val Asp Glu Ser Ile Cys Lys Phe Ala Lys Lys Gly Leu Thr Pro Ser
           35                  40                  45
   Gln Ile Gly Val Ile Leu Arg Asp Ser His Gly Ile Pro Gln Val Lys
       50                  55                  60
   Ser Val Thr Gly Ser Lys Ile Leu Arg Ile Leu Lys Ala His Gly Leu
   65                  70                  75                  80
   Ala Pro Glu Ile Pro Glu Asp Leu Tyr His Leu Ile Lys Lys Ala Val
                   85                  90                  95
   Ala Ile Arg Lys His Leu Glu Arg Asn Arg Lys Asp Lys Asp Ser Lys
               100                 105                 110
   Phe Arg Leu Ile Leu Val Glu Ser Arg Ile His Arg Leu Ala Arg Tyr
           115                 120                 125
   Tyr Lys Lys Thr Lys Lys Leu Pro Pro Val Trp Lys Tyr Glu Ser Thr
       130                 135                 140
   Thr Ala Ser Thr Leu Val Ala
   145                 150
<210> 44
   <211> 151
   <212> PRT
   <213> Pisum sativum
<400> 44
   Met Gly Arg Leu His Ser Lys Gly Lys Gly Ile Ser Ser Ser Ala Leu
   1               5                   10                  15
   Pro Tyr Arg Arg Thr Ala Pro Ser Trp Leu Lys Ile Ser Ser Gln Asp
               20                  25                  30
   Val Asp Glu Thr Ile Cys Lys Phe Ala Lys Lys Gly Leu Thr Pro Ser
           35                  40                  45
   Gln Ile Gly Val Ile Leu Arg Asp Ser His Gly Ile Ala Gln Val Lys
       50                  55                  60
   Ser Val Thr Gly Ser Lys Ile Leu Arg Ile Leu Lys Ala His Gly Leu
   65                  70                  75                  80
   Ala Pro Glu Ile Pro Glu Asp Leu Tyr His Leu Ile Lys Lys Ala Val
                   85                  90                  95
   Ser Ile Arg Lys His Leu Glu Arg Phe Arg Lys Asp Lys Asp Ser Lys
               100                 105                 110
   Phe Arg Leu Ile Leu Val Glu Ser Arg Ile His Arg Leu Ala Arg Tyr
           115                 120                 125
   Tyr Lys Lys Thr Lys Lys Leu Pro Pro Val Trp Lys Tyr Glu Ser Thr
       130                 135                 140
   Thr Ala Ser Thr Leu Val Ala
   145                 150
<210> 45
   <211> 150
   <212> PRT
   <213> Arabidopsis thaliana
<400> 45
   Met Gly Arg Met His Ser Arg Gly Lys Gly Ile Ser Ala Ser Ala Leu
   1               5                   10                  15
   Pro Tyr Lys Arg Ser Ser Pro Ser Trp Leu Lys Thr Thr Ser Gln Asp
               20                  25                  30
   Val Asp Glu Ser Ile Cys Lys Phe Ala Lys Lys Gly Leu Thr Pro Ser
           35                  40                  45
   Gln Ile Gly Val Ile Leu Arg Asp Ser His Gly Ile Pro Gln Val Lys
       50                  55                  60
   Ser Val Thr Gly Ser Lys Ile Leu Arg Ile Leu Lys Ala His Gly Leu
   65                  70                  75                  80
   Ala Pro Glu Ile Pro Glu Asp Leu Tyr His Leu Ile Lys Lys Ala Val
                   85                  90                  95
   Ala Ile Arg Lys His Leu Glu Arg Asn Arg Lys Asp Lys Asp Ser Lys
               100                 105                 110
   Phe Arg Leu Ile Leu Val Glu Ser Arg Ile His Arg Leu Ala Arg Tyr
           115                 120                 125
   Tyr Lys Lys Thr Lys Lys Leu Pro Pro Val Trp Asn Glu Ser Thr Thr
       130                 135                 140
   Ala Ser Thr Leu Val Ala
   145                 150
<210> 46
   <211> 150
   <212> PRT
   <213> Arabidopsis thaliana
<400> 46
   Met Gly Arg Met His Ser Arg Gly Lys Gly Ile Ser Ala Ser Ala Leu
   1               5                   10                  15
   Pro Tyr Lys Arg Ser Ser Pro Ser Trp Leu Lys Thr Thr Pro Gln Asp
               20                  25                  30
   Val Asp Glu Ser Ile Cys Lys Phe Ala Lys Lys Gly Leu Thr Pro Ser
           35                  40                  45
   Gln Ile Gly Val Ile Leu Arg Asp Ser His Gly Ile Pro Gln Val Lys
       50                  55                  60
   Ser Val Thr Gly Ser Lys Ile Leu Arg Ile Leu Lys Ala His Gly Leu
   65                  70                  75                  80
   Ala Pro Glu Ile Pro Glu Asp Leu Tyr His Leu Ile Lys Lys Ala Val
                   85                  90                  95
   Ala Ile Arg Lys His Leu Glu Arg Asn Arg Lys Asp Lys Asp Ser Lys
               100                 105                 110
   Phe Arg Leu Ile Leu Val Glu Ser Arg Ile His Arg Leu Ala Arg Tyr
           115                 120                 125
   Tyr Lys Lys Thr Lys Lys Leu Pro Pro Val Trp Asn Glu Ser Thr Thr
       130                 135                 140
   Ala Ser Thr Leu Val Ala
   145                 150
<210> 47
   <211> 151
   <212> PRT
   <213> Oryza sativa
<400> 47
   Met Gly Arg Met His Ser Arg Gly Lys Gly Ile Ser Ser Ser Ala Leu
   1               5                   10                  15
   Pro Tyr Lys Arg Thr Pro Pro Ser Trp Leu Lys Thr Ala Ala Ser Asp
               20                  25                  30
   Val Glu Glu Met Ile Met Lys Ala Ala Lys Lys Gly Gln Met Pro Ser
           35                  40                  45
   Gln Ile Gly Val Val Leu Arg Asp Gln His Gly Ile Pro Leu Val Lys
       50                  55                  60
   Ser Val Thr Gly Ser Lys Ile Leu Arg Ile Leu Lys Ala His Gly Leu
   65                  70                  75                  80
   Ala Pro Glu Ile Pro Glu Asp Leu Tyr Phe Leu Ile Lys Lys Ala Val
                   85                  90                  95
   Ala Ile Arg Lys His Leu Glu Arg Asn Arg Lys Asp Lys Asp Ser Lys
               100                 105                 110
   Phe Arg Leu Ile Leu Val Glu Ser Arg Ile His Arg Leu Ala Arg Tyr
           115                 120                 125
   Tyr Lys Arg Thr Lys Lys Leu Pro Pro Thr Trp Lys Tyr Glu Ser Thr
       130                 135                 140
   Thr Ala Ser Thr Leu Val Ala
   145                 150
<210> 48
   <211> 151
   <212> PRT
   <213> Zea mays
<400> 48
   Met Gly Ala Met His Ser Arg Gly Lys Gly Ile Ser Ser Ser Ala Leu
   1               5                   10                  15
   Pro Tyr Lys Arg Thr Pro Pro Thr Trp Leu Lys Thr Ala Ala Ser Asp
               20                  25                  30
   Val Glu Glu Met Ile Thr Lys Ala Ala Lys Lys Gly Gln Met Pro Ser
           35                  40                  45
   Gln Ile Gly Val Leu Leu Arg Asp Gln His Gly Ile Pro Leu Val Lys
       50                  55                  60
   Ser Val Thr Gly Ser Lys Ile Leu Arg Ile Leu Lys Ala His Gly Leu
   65                  70                  75                  80
   Ala Pro Glu Ile Pro Glu Asp Leu Tyr Phe Leu Ile Lys Lys Ala Val
   85 90 95
   Ala Ile Arg Lys His Leu Glu Arg Asn Arg Lys Asp Lys Asp Ser Lys
   100 105 110
   Phe Arg Leu Ile Leu Val Glu Ser Arg Ile His Arg Leu Ala Arg Tyr
   115 120 125
   Tyr Lys Arg Thr Lys Lys Leu Pro Pro Thr Trp Lys Tyr Glu Ser Thr
   130 135 140
   Thr Ala Ser Thr Leu Val Ala
   145 150
<210> 49
   <211> 156
   e212> PRT
   <213> Oryza sativa
<400> 49
   Met Gly Arg Met His Ser Arg Gly Lys Gly Ile Ser Ser Ser Ala Ile
   1 5 10 15
   Pro Tyr Lys Arg Thr Pro Pro Ser Trp Val Lys Thr Ala Ala Ala Asp
   20 25 30
   Val Glu Glu Met Ile Met Lys Ala Ala Lys Lys Gly Gln Met Pro Ser
   35 40 45
   Gln Ile Gly Val Val Leu Arg Asp Gln His Gly Ile Pro Leu Val Lys
   50 55 60
   Ser Val Thr Gly Ser Lys Ile Leu Arg Ile Leu Lys Ala His Gly Leu
   65 70 75 80
   Ala Pro Glu Ile Pro Glu Asp Leu Tyr Phe Leu Ile Lys Lys Ala Val
   85 90 95
   Ala Ile Arg Lys His Leu Glu Arg Asn Arg Lys Asp Lys Asp Ser Lys
   100 105 110
   Phe Arg Leu Ile Leu Val Glu Ser Arg Ile His Arg Leu Ala Arg Tyr
   115 120 125
   Tyr Lys Arg Thr Lys Lys Leu Pro Pro Thr Trp Lys Thr Thr Tyr Gln
   130 135 140
   Leu Ser Ala Val Cys Leu Asn Tyr Phe Gln Ala Pro
   145 150 155
<210> 50
   <211> 151
   <212> PRT
   <213> Drosophila melanogaster
<400> 50
   Met Gly Arg Met His Ala Pro Gly Lys Gly Ile Ser Gln Ser Ala Leu
   1               5                   10                  15
   Pro Tyr Arg Arg Thr Val Pro Ser Trp Leu Lys Leu Asn Ala Asp Asp
               20                  25                  30
   Val Lys Glu Gln Ile Lys Lys Ala Gly Gln Glu Gly Ser Asp Ser Leu
           35                  40                  45
   Gln Ile Gly Ile Ile Leu Arg Asp Ser His Gly Val Ala Gln Val Arg
       50                  55                  60
   Phe Val Asn Gly Asn Lys Ile Leu Arg Ile Met Lys Ser Val Gly Leu
   65                  70                  75                  80
   Lys Pro Asp Ile Pro Glu Asp Leu Tyr His Met Ile Lys Lys Ala Val
                   85                  90                  95
   Ala Ile Arg Lys His Leu Glu Arg Asn Arg Lys Asp Lys Asp Gly Lys
               100                 105                 110
   Phe Arg Leu Ile Leu Val Glu Ser Arg Ile His Arg Leu Ala Arg Tyr
           115                 120                 125
   Tyr Lys Thr Lys Ser Val Leu Pro Pro Asn Trp Lys Tyr Glu Ser Ser
       130                 135                 140
   Thr Ala Ser Ala Leu Val Ala
   145                 150
<210> 51
   <211> 155
   <212> PRT
   <213> Unknown
<220>
   <223> environmental sequence
<400> 51
   Met Gly Arg Met His Thr Pro Gly Lys Gly Met Ser Gly Ser Ala Leu
   1 5 10 15
   Pro Tyr Lys Arg Ser Ala Pro Ser Trp Leu Lys Ile Thr Pro Thr Glu
   20 25 30
   Val Thr Glu Met Ile Val Lys Met Ala Lys Lys Gly Met Thr Pro Ser
   35 40 45
   Gln Ile Gly Val Met Leu Arg Asp Asn Gln Gly Ile Ala Gln Val Ser
   50 55 60
   Thr Val Thr Asn Ser Lys Ile Leu Arg Ile Leu Arg Gly Gln Gly Leu
   65 70 75 80
   Ala Pro Ser Leu Pro Glu Asp Leu Tyr Cys Leu Ile Lys Lys Ala Val
   85 90 95
   Ser Val Arg Lys His Leu Glu Arg Asn Arg Lys Asp Met Asp Ser Lys
   100 105 110
   Phe Arg Leu Ile Leu Ile Glu Ser Arg Ile His Arg Leu Ala Arg Tyr
   115 120 125
   Tyr Lys Leu Ala Lys Lys Leu Glu Pro Thr Phe Lys Tyr Asp Ser Ala
   130 135 140
   Thr Ala Ser Thr Leu Leu Thr Ala Ala Gly Lys
   145 150 155
<210> 52
   <211> 230
   <212> PRT
   <213> Arabidopsis thaliana
<400> 52
   Met Ala Ser Ser Asn Ser Glu Lys Ile Asn Glu Asn Leu Tyr Ala Val
   1 5 10 15
   Leu Gly Leu Lys Lys Glu Cys Ser Lys Thr Glu Leu Arg Ser Ala Tyr
   20 25 30
   Lys Lys Leu Ala Leu Arg Trp His Pro Asp Arg Cys Ser Ser Met Glu
   35 40 45
   Phe Val Glu Glu Ala Lys Lys Lys Phe Gln Ala Ile Gln Glu Ala Tyr
   50 55 60
   Ser Val Leu Ser Asp Ser Asn Lys Arg Phe Leu Tyr Asp Val Gly Ala
   65 70 75 80
   Tyr Asn Thr Asp Asp Asp Asp Asp Gln Asn Gly Met Gly Asp Phe Leu
   85 90 95
   Asn Glu Met Ala Thr Met Met Asn Gln Ser Lys Pro Ser Asp Asn Asn
   100 105 110
   Thr Gly Asp Ser Phe Glu Gln Leu Gln Asp Leu Phe Asn Glu Met Phe
   115 120 125
   Gln Gly Asp Ala Ala Ala Phe Pro Ser Ser Ser Ser Cys Ser Thr Ser
   130 135 140
   Asn Phe Thr Ser Ser Arg Ser Phe Val Phe Asp Thr Asn Ser Gln Arg
   145 150 155 160
   Ser Ser Ser Phe Ala Thr Ser Ser Met Gly Met Asn Asn Asp Pro Phe
   165 170 175
   Gly Tyr Asp Pro Arg Ala His Ser Phe Ser Leu Gly Val Asp His Gln
   180 185 190
   Gln Glu Phe Lys Lys Gly Lys Asn Asn Gly Gly Arg Arg Asn Arg Arg
   195 200 205
   Lys Asn Asn Val Pro Ser Ala Gly His Glu Thr Ser Ser Ser Asn Asn
   210 215 220
   Tyr Gly Val Pro Thr Ser
   225 230
<210> 53
   <211> 242
   <212> PRT
   <213> Muc musculus
<400> 53
   Met Val Asp Tyr Tyr Glu Val Leu Gly Val Gln Arg His Ala Ser Pro
   1               5                   10                  15
   Glu Asp Ile Lys Lys Ala Tyr Arg Lys Gln Ala Leu Lys Trp His Pro
               20                  25                  30
   Asp Lys Asn Pro Glu Asn Lys Glu Glu Ala Glu Arg Lys Phe Lys Gln
           35                  40                  45
   Val Ala Glu Ala Tyr Glu Val Leu Ser Asp Ala Lys Lys Arg Asp Ile
       50                  55                  60
   Tyr Asp Lys Tyr Gly Lys Glu Gly Leu Asn Gly Gly Gly Gly Gly Gly
   65                  70                  75                  80
   Gly Ile His Phe Asp Ser Pro Phe Glu Phe Gly Phe Thr Phe Arg Asn
                   85                  90                  95
   Pro Asp Asp Val Phe Arg Glu Phe Phe Gly Gly Arg Asp Pro Phe Ser
               100                 105                 110
   Phe Asp Phe Phe Glu Asp Pro Phe Asp Asp Phe Phe Gly Asn Arg Arg
           115                 120                 125
   Gly Pro Arg Gly Asn Arg Ser Arg Gly Ala Ala Pro Phe Phe Ser Thr
       130                 135                 140
   Phe Ser Gly Phe Pro Ser Phe Gly Ser Gly Phe Pro Ala Phe Asp Thr
   145                 150                 155                 160
   Gly Phe Thr Pro Phe Gly Ser Leu Gly His Gly Gly Leu Thr Ser Phe
                   165                 170                 175
   Ser Ser Thr Ser Phe Gly Gly Ser Gly Met Gly Asn Phe Lys Ser Ile
               180                 185                 190
   Ser Thr Ser Thr Lys Ile Val Asn Gly Lys Lys Ile Thr Thr Lys Arg
           195                 200                 205
   Ile Val Glu Asn Gly Gln Glu Arg Val Glu Val Glu Glu Asp Gly Gln
       210                 215                 220
   Leu Lys Pro Leu Thr Ile Asn Gly Lys Glu His Leu Leu Arg Leu Asp
   225                 230                 235                 240
   Asn Lys
<210> 54
   <211> 242
   <212> PRT
   <213> Rattus norvegicus
<400> 54
   Met Val Asp Tyr Tyr Glu Val Leu Gly Val Gln Arg His Ala Ser Pro
   1               5                   10                  15
   Glu Asp Ile Lys Lys Ala Tyr Arg Lys Gln Ala Leu Lys Trp His Pro
               20                  25                  30
   Asp Lys Asn Pro Glu Asn Lys Glu Glu Ala Glu Arg Lys Phe Lys Gln
           35                  40                  45
   Val Ala Glu Ala Tyr Glu Val Leu Ser Asp Ala Lys Lys Arg Asp Ile
       50                  55                  60
   Tyr Asp Lys Tyr Gly Lys Glu Gly Leu Asn Gly Gly Gly Gly Gly Gly
   65                  70                  75                  80
   Gly Ser His Phe Asp Ser Pro Phe Glu Phe Gly Phe Thr Phe Arg Asn
                   85                  90                  95
   Pro Asp Asp Val Phe Arg Glu Phe Phe Gly Gly Arg Asp Pro Phe Ser
               100                 105                 110
   Phe Asp Phe Phe Glu Asp Pro Phe Asp Asp Phe Phe Gly Asn Arg Arg
           115                 120                 125
   Gly Pro Arg Gly Ser Arg Ser Arg Gly Ala Gly Ser Phe Phe Ser Ala
       130                 135                 140
   Phe Ser Gly Phe Pro Ser Phe Gly Ser Gly Phe Pro Ala Phe Asp Thr
   145                 150                 155                 160
   Gly Phe Thr Pro Phe Gly Ser Leu Gly His Gly Gly Leu Thr Ser Phe
                   165                 170                 175
   Ser Ser Ala Ser Phe Gly Gly Ser Gly Met Gly Asn Phe Lys Ser Ile
               180                 185                 190
   Ser Thr Ser Thr Lys Ile Val Asn Gly Lys Lys Ile Thr Thr Lys Arg
           195                 200                 205
   Ile Val Glu Asn Gly Gln Glu Arg Val Glu Val Glu Glu Asp Gly Gln
       210                 215                 220
   Leu Lys Ser Leu Thr Ile Asn Gly Lys Glu His Leu Leu Arg Leu Asp
   225                 230                 235                 240
   Asn Lys
<210> 55
   <211> 158
   <212> PRT
   <213> Picea glauca
<400> 55
   Met Glu Thr Thr Phe Tyr Ser Ile Leu Gly Val Asn Lys Asp Ser Ser
   1 5 10 15
   Ser Ala Glu Ile Arg Ser Ala Tyr Arg Lys Leu Ala Met Lys Trp His
   20 25 30
   Pro Asp Lys Trp Ser Thr Asp Pro Ser Ser Ser Glu Thr Ala Lys Leu
   35 40 45
   Arg Phe Gln Gln Ile Gln Glu Ala Tyr Ser Val Leu Ser Asp Asp Thr
   50 55 60
   Lys Arg Ala Leu Tyr Asp Ala Gly Met Tyr Glu Pro Ser Glu Asp Met
   65 70 75 80
   Asp Ala Phe Cys Asp Phe Leu Asp Glu Leu Ser Ser Leu Ile Ala Thr
   85 90 95
   Val Lys Val Gln Ser Asn Lys Asp Asp Glu Leu Leu Gln Leu Gln Glu
   100 105 110
   Met Phe Thr Lys Met Leu Glu Glu Asp Trp Phe Ser Thr Asp Asn Phe
   115 120 125
   Glu Ala Phe Lys Glu Ile Ser Ser Gln His Ser Asp Asp Lys Pro Glu
   130 135 140
   Asn Gly Gln Asp His Glu Pro Tyr Gly Ser Val Asp Asp Leu
   145 150 155
<210> 56
   <211> 156
   <212> PRT
   <213> Arabidopsis thaliana
<400> 56
   Met Ala Cys Glu Gly Gly Gly Ser Asn Val Arg Ser Ser Tyr Tyr Thr
   1 5 10 15
   Ile Leu Gly Ile Arg Lys Asp Ala Ser Val Ser Asp Ile Arg Thr Ala
   20 25 30
   Tyr Arg Lys Leu Ala Met Lys Trp His Pro Asp Arg Tyr Ala Arg Asn
   35 40 45
   Pro Gly Val Ala Gly Glu Ala Lys Arg Arg Phe Gln Gln Ile Gln Glu
   50 55 60
   Ala Tyr Ser Val Leu Asn Asp Glu Asn Lys Arg Ser Met Tyr Asp Val
   65 70 75 80
   Gly Leu Tyr Asp Pro His Glu Asp Asp Asp Asp Asp Phe Cys Asp Phe
   85 90 95
   Met Gln Glu Met Ile Ser Met Met Asn Asn Val Lys Asp Ala Gly Glu
   100 105 110
   Ser Leu Glu Asp Leu Gln Arg Met Phe Thr Asp Met Val Gly Gly Asp
   115 120 125
   Gly Val Ser Tyr Asp Cys Asn Asn Asn Pro Lys Gly Asn Lys Arg Pro
   130 135 140
   Arg Val Asn Ile Ser Arg Ser Ser Ala Ala Met Arg
   145 150 155
<210> 57
   <211> 156
   <212> PRT
   <213> Arabidopsis thaliana
<400> 57
   Met Ala Cys Glu Gly Gly Gly Ser Asn Val Arg Ser Ser Tyr Tyr Thr
   1 5 10 15
   Val Leu Gly Ile Arg Lys Asp Ala Ser Val Ser Asp Ile Arg Thr Ala
   20 25 30
   Tyr Arg Lys Leu Ala Met Lys Trp His Pro Asp Arg Tyr Ala Arg Asn
   35 40 45
   Pro Gly Val Ala Gly Glu Ala Lys Arg Arg Phe Gln Gln Ile Gln Glu
   50 55 60
   Ala Tyr Ser Val Leu Asn Asp Glu Asn Lys Arg Ser Met Tyr Asp Val
   65 70 75 80
   Gly Leu Tyr Asp Pro His Glu Asp Asp Asp Asp Asp Phe Cys Asp Phe
   85 90 95
   Met Gln Glu Met Ile Ser Met Met Asn Asn Val Lys Asp Glu Gly Glu
   100 105 110
   Ser Leu Glu Asp Leu Gln Arg Met Phe Thr Asp Met Val Gly Gly Asp
   115 120 125
   Gly Val Ser Tyr Asp Cys Asn Asn Asn Pro Lys Gly Ser Lys Arg Pro
   130 135 140
   Arg Val Asn Val Ser Arg Ser Ser Ala Ala Met Arg
   145 150 155
<210> 58
   <211> 207
   <212> PRT
   <213> Daucus carota
<400> 58
   Met Ile Asp Gln Glu Glu Ser Asn Phe Asn Phe Asn Phe Asn Gln Pro
   1               5                   10                  15
   Gln Gln Pro Gln Gln Gln Gln Phe His Gly Lys Ser Val Lys Lys Asn
               20                  25                  30
   Lys Asn Lys Asn Asn Asn Asn Asn Ser Glu Ser Gly Asn Lys Asn Gly
           35                  40                  45
   Gly Glu Asn Lys Asn Gly Val Glu Lys Arg Phe Lys Thr Leu Pro Pro
       50                  55                  60
   Ala Glu Ser Leu Pro Arg Asn Glu Thr Val Gly Gly Tyr Ile Phe Val
   65                  70                  75                  80
   Cys Asn Asn Asp Thr Met Gln Glu Asn Leu Lys Arg Gln Leu Phe Gly
                   85                  90                  95
   Leu Pro Pro Arg Tyr Arg Asp Ser Val Arg Ala Ile Thr Pro Gly Leu
               100                 105                 110
   Pro Leu Phe Leu Tyr Asn Tyr Ser Thr His Gln Leu His Gly Val Phe
           115                 120                 125
   Glu Ala Ala Ser Phe Gly Gly Thr Asn Ile Asp Pro Thr Ala Trp Glu
       130                 135                 140
   Asp Lys Lys Asn Gln Gly Glu Ser Arg Phe Pro Ala Gln Val Arg Val
   145                 150                 155                 160
   Met Thr Arg Lys Ile Cys Glu Pro Leu Glu Glu Asp Ser Phe Arg Pro
                   165                 170                 175
   Ile Leu His His Tyr Asp Gly Pro Lys Phe Arg Leu Glu Leu Asn Ile
               180                 185                 190
   Pro Glu Ala Ile Ser Leu Leu Asp Ile Phe Glu Glu Thr Lys Ala
           195                 200                 205
<210> 59
   <211> 213
   <212> PRT
   <213> Pisum sativum
<400> 59
   Met Asn Lys Asn Ser Leu Arg Asn Gly Val Tyr Asn Met Asn Ala Val
   1 5 10 15
   Tyr Gln Lys Ser Asn Ala Asn Phe Val Gly Asn Met Asn Ser Asn Lys
   20 25 30
   Tyr Ser Gly Asn Val Gln Leu Asn Lys Asp Pro His Ser Asn Asn Asn
   35 40 45
   Asn Asn Asn Asn Glu Asn Asn Thr Asn Ala Thr Asp Lys Arg Phe Lys
   50 55 60
   Thr Leu Pro Ala Ala Glu Thr Leu Pro Arg Asn Glu Val Leu Gly Gly
   65 70 75 80
   Tyr Ile Phe Val Cys Asn Asn Asp Thr Met Gln Glu Asp Leu Lys Arg
   85 90 95
   Gln Leu Phe Gly Leu Pro Pro Arg Tyr Arg Asp Ser Val Arg Ala Ile
   100 105 110
   Thr Pro Gly Leu Pro Leu Phe Leu Tyr Asn Tyr Thr Thr His Gln Leu
   115 120 125
   His Gly Ile Phe Glu Ala Thr Cys Phe Gly Gly Ser Asn Ile Asp Pro
   130 135 140
   Thr Ala Trp Glu Asp Lys Lys Cys Lys Gly Glu Ser Arg Phe Pro Ala
   145 150 155 160
   Gln Val Arg Ile Arg Val Arg Lys Ile Cys Lys Ala Leu Glu Glu Asp
   165 170 175
   Ser Phe Arg Pro Val Leu His His Tyr Asp Gly Pro Lys Phe Arg Leu
   180 185 190
   Glu Leu Ser Val Pro Glu Thr Leu Asp Leu Met Asp Leu Cys Glu Gln
   195 200 205
   Ala Gly Ser Ala Ala
   210
<210> 60
   <211> 305
   <212> PRT
   <213> Citrus X paradisi
<400> 60
   Met Asp Asn Met His Ser Phe Trp Gln Leu Gly Asp Glu Leu Arg Gly
   1               5                   10                  15
   Gln Ser Arg Thr Ser Glu Asp Gln Ser Trp Leu Arg Ala Ala Ser Arg
               20                  25                  30
   Leu Ala Glu Gln Thr Arg Phe Lys Gly Glu Arg Met Asn Asn Leu Asp
           35                  40                  45
   Leu Ser Lys Gly Met Thr Glu Ile Arg Pro Arg Asp Lys Ile Met Tyr
       50                  55                  60
   His Glu Asp Asn Asn Phe Glu Ser Phe Asn Phe Asn Phe Asn Met Met
   65                  70                  75                  80
   Asn Leu Asp Asn Lys Val Val Glu Asn Val Thr Lys Ser Ser Leu Arg
                   85                  90                  95
   Asn Gly Ile Tyr Asn Met Asn Ala Val Tyr Gln Lys Asn Ser Gly His
               100                 105                 110
   Asn Met Gly Asn Leu Met Val Asn Lys Tyr Gly Gly Asn Asn Leu Ser
           115                 120                 125
   Val Lys Glu Ala Glu Asn Asn Asn Asn Asn Asn Asn Asn Asn Asn Asp
   130 135 140
   Ser Asn Ala Asn Ser Ala Leu Asp Lys Arg Phe Lys Thr Leu Pro Ala
   145 150 155 160
   Thr Glu Thr Leu Pro Arg Asn Glu Val Leu Gly Gly Tyr Ile Phe Val
   165 170 175
   Cys Asn Asn Asp Thr Met Gln Glu Asp Leu Lys Arg Gln Leu Phe Gly
   180 185 190
   Leu Pro Pro Arg Tyr Arg Asp Ser Val Arg Ala Ile Thr Pro Gly Leu
   195 200 205
   Pro Leu Phe Leu Tyr Asn Tyr Thr Thr His Gln Leu His Gly Ile Phe
   210 215 220
   Glu Ala Thr Gly Phe Gly Gly Ser Asn Ile Asp Pro Thr Ala Trp Glu
   225 230 235 240
   Asp Lys Lys Cys Lys Gly Glu Ser Arg Phe Pro Ala Gln Val Arg Ile
   245 250 255
   Arg Val Arg Lys Leu Cys Lys Ala Leu Glu Glu Asp Ala Phe Arg Pro
   260 265 270
   Val Leu His His Tyr Asp Gly Pro Lys Phe Arg Leu Glu Leu Ser Val
   275 280 285
   Pro Glu Thr Leu Asp Leu Met Asp Leu Cys Glu Gln Ala Gly Ser Ala
   290 295 300
   Ala
   305
<210> 61
   <211> 296
   <212> PRT
   <213> Arabidopsis thaliana
<400> 61
   Met Asp Ser Phe Trp Gln Leu Gly Asp Glu Leu Arg Gly Gln Thr Arg
   1 5 10 15
   Ala Ser Glu Asp His Lys Trp Ser Thr Val Ala Thr Lys Leu Ala Glu
   20 25 30
   Gln Thr Arg Met Lys Gly Glu Arg Met Asn Asn Leu Asp Leu Ser Lys
   35 40 45
   Gly Tyr Thr Glu Phe Arg Pro Ser Glu Lys Phe Ser Phe Gln Glu Asn
   50 55 60
   Asn Leu Asn Phe Asn Met Leu Asn Leu Asp Gly Lys Phe Gly Glu Ser
   65 70 75 80
   Ile Met Gly Lys Thr Ser Met Gln Ser Asn Val Tyr Asn Met Asn Thr
   85 90 95
   Val Phe Gln Lys Asn Asp Phe Lys Ser Gly Gly Asn Met Lys Val Asn
   100 105 110
   Lys Tyr Asn Gly Asn Ile Val Ala Asn Lys Glu Met Ser Asn Asn Lys
   115 120 125
   His Asn Asn Asn Cys Asn Asp Asn Gly Asn Met Asn Leu Ala Val Asp
   130 135 140
   Lys Arg Phe Lys Thr Leu Pro Ala Ser Glu Thr Leu Pro Arg Asn Glu
   145 150 155 160
   Val Leu Gly Gly Tyr Ile Phe Val Cys Asn Asn Asp Thr Met Gln Glu
   165 170 175
   Asp Met Lys Arg His Leu Phe Gly Leu Pro Pro Arg Tyr Arg Asp Ser
   180 185 190
   Val Arg Ala Ile Thr Pro Gly Leu Pro Leu Phe Leu Tyr Asn Tyr Thr
   195 200 205
   Thr His Gln Leu His Gly Ile Phe Glu Ala Thr Thr Phe Gly Gly Thr
   210 215 220
   Asn Ile Asp Ala Thr Ala Trp Glu Asp Lys Lys Cys Lys Gly Glu Ser
   225 230 235 240
   Arg Phe Pro Ala Gln Val Arg Ile Arg Val Arg Lys Ile Cys Lys Ala
   245 250 255
   Leu Glu Glu Asp Ser Phe Arg Pro Val Leu His His Tyr Asp Gly Pro
   260 265 270
   Lys Phe Arg Leu Glu Leu Ser Val Pro Glu Thr Leu Asp Leu Leu Asp
   275 280 285
   Leu Cys Glu Gln Ala Gly Ser Ala
   290 295
<210> 62
   <211> 296
   <212> PRT
   <213> Arabidopsis thaliana
<100> 62
<210> 63
   <211> 742
   <212> PRT
   <213> Arabidopsis thaliana
<400> 63
   Met Ala Glu Ala Met Glu Thr Glu Met Asp Phe Ser Asp Gly Glu Gln
   1 5 10 15
   Thr Asn Gly Asn Ser His Val Thr Ala Ser Gln Tyr Phe Ala Pro Pro
   20 25 30
   Gly Tyr Asn Arg Ser Leu Val Ala Ala Tyr Gly Asn Gly Asn Thr Thr
   35 40 45
   Ile Gly Leu Glu Lys Gly Ile Glu Arg Arg Leu Asp His His Glu Gln
   50 55 60
   Leu Pro Gly Tyr Ile Phe Met Cys Asn Gly Arg Thr Lys Thr Asp Cys
   65 70 75 80
   Tyr Arg Tyr Arg Val Phe Gly Ile Pro Arg Gly Gly Lys Asp Val Val
   85 90 95
   Glu Ser Ile Lys Pro Gly Met Lys Leu Phe Leu Tyr Asp Phe Glu Lys
   100 105 110
   Arg Leu Leu Tyr Gly Val Tyr Glu Ala Thr Val Gly Gly Arg Leu Asp
   115 120 125
   Ile Glu Pro Glu Ala Phe Glu Gly Lys Tyr Pro Ala Gln Val Gly Phe
   130 135 140
   Arg Ile Val Met Asn Cys Leu Pro Leu Thr Glu Asn Thr Phe Lys Ser
   145 150 155 160
   Ala Ile Tyr Glu Asn Tyr Lys Gly Ser Lys Phe Lys Gln Glu Leu Ser
   165 170 175
   Pro His Gln Val Met Ser Leu Leu Ser Leu Phe Arg Ser Phe Thr Ser
   180 185 190
   Pro Glu Leu Asp Leu Leu Pro His Arg Leu Ala Ser Arg Ala Ser Ala
   195 200 205
   Pro Arg Thr Leu Ser Phe Glu Glu Arg Phe Ile Ala Ala Thr His Leu
   210 215 220
   Arg Asn Ala Ser Ser Val Leu Asp Pro Leu Ser Ala Arg His Val Glu
   225 230 235 240
   Pro Arg Leu Gly Ser Val Met Ala His Gln Pro Val Pro Arg Thr Ser
   245 250 255
   Leu Leu Gln His Ser Tyr Phe Arg Gln Asp Asp Tyr Thr Thr Pro Pro
   260 265 270
   Arg Glu Ser Leu Ser Asn Leu Asn Gln Pro Tyr Tyr Pro Thr Glu Ala
   275 280 285
   Arg Gln Leu Arg Leu Leu Gly Asp Pro Ser Arg Ser Asp Ser Pro Arg
   290 295 300
   Ser Glu Pro Pro Arg Ser Ser Ile Gln Asp Pro Gln Leu Lys Tyr Leu
   305 310 315 320
   Thr Ile Leu Ser Asn Ile Arg Arg Tyr Gly Ser Ala Ser Asp Arg Leu
   325 330 335
   Ala Ser Glu Asn Glu Tyr His Pro Ala Thr Pro Ser Glu Lys Asp Gln
   340 345 350
   Phe Ala Val Pro Tyr Ser Asp Asn Lys Asn Tyr Pro Ser Thr Leu Ser
   355 360 365
   Gly Ser Glu His Pro Ser Ala Ser Ala Ala Asn Gly Ser Val Tyr Arg
   370 375 380
   Ser Glu Phe Tyr Asn Ser Ala Ser Gln Lys Glu Gly Glu Ala Ser Gln
   385 390 395 400
   Gln His Glu Ile Pro Ala Gly Thr Tyr His His Pro Glu Ala Ser Thr
   405 410 415
   Val Ser Asn Thr Thr Lys Ser Met Gln Pro Asp Met Gln Ala Val Ser
   420 425 430
   Val Ala Gln Ser His Thr Glu Thr Ala Gly Tyr Pro Thr Pro Ala His
   435 440 445
   Gly Glu Ala Ser Gln Pro Pro Ala Gly Ala Ile Gly Tyr Thr His Gln
   450 455 460
   Pro Gln Ser Val Ala Gly Asn Tyr Ser Thr His Ser Gln Pro Gly Asn
   465 470 475 480
   Val Glu Glu Ser Thr Gln Ser Tyr Ala Gly Thr Asp Ser Tyr Ser Gln
   485 490 495
   Gln Gln Tyr Tyr Ala Ala Met Gly Pro Thr Thr Gln Leu His Ala Gly
   500 505 510
   Gly Tyr Ile Gln Lys Pro His Glu Ile Gly Tyr Ser Gln Gln Pro His
   515 520 525
   Asp Ala Ala Thr Gly Tyr Ser Gln Gln Pro His Asp Ala Ala Thr Gly
   530 535 540
   Tyr Ser Gln Gln Pro His Asp Ala Ala Thr Gly Tyr Ser Gln Gln Pro
   545 550 555 560
   His Ala Ala Ser Thr Gly Tyr Ser Gln Gln Thr Tyr Ala Ala Ala Thr
   565 570 575
   Gly Tyr Thr Gln Gln Pro His Ala Ala Ala Ala Gly Tyr Thr Gln Gln
   580 585 590
   Pro His Ala Ala Ala Thr Gly Tyr Ser Gln Gln Pro His Ala Ala Ala
   595 600 605
   Thr Ala His Ala Gln Gln Pro Tyr Ala Ala Ala Thr Ala His Ala Gln
   610 615 620
   Gln Leu His Ala Val Ala Thr Gly Tyr Ala Leu Gln Leu His Ala Ala
   625 630 635 640
   Ala Thr Gly Tyr Ala Gln Gln Pro His Ala Ala Ala Thr Gly Tyr Ala
   645 650 655
   Leu Gln Pro His Ala Gln Ala Val Glu Tyr Thr Met Gln Pro His Ala
   660 665 670
   Gln Ala Val Gly Tyr Met Pro Gln Tyr His Ala His Ala Val Val Tyr
   675 680 685
   Ser Gln Gln Gly Val Thr Gln Gly Ser Val Pro Arg Ala Pro Gly Thr
   690 695 700
   Thr Asp Cys Asn Ala Ala Asn Gln Ala Tyr Ser Ala Thr Gly Asp Trp
   705 710 715 720
   Asn Ala Val His Gln Ser Tyr Tyr Pro Gln Thr Ala Asp Ala Thr Thr
   725 730 735
   Thr Tyr Tyr Gln Thr Ser
   740
<210> 64
   <211> 195
   <212> PRT
   <213> Arabidopsis thaliana
<400> 64
   Met Ala Gly Val Ala Phe Gly Ser Phe Asp Asp Ser Phe Ser Leu Ala
   1               5                   10                  15
   Ser Leu Arg Ala Tyr Leu Ala Glu Phe Ile Ser Thr Leu Leu Phe Val
               20                  25                  30
   Phe Ala Gly Val Gly Ser Ala Ile Ala Tyr Ala Lys Leu Thr Ser Asp
           35                  40                  45
   Ala Ala Leu Asp Thr Pro Gly Leu Val Ala Ile Ala Val Cys His Gly
       50                  55                  60
   Phe Ala Leu Phe Val Ala Val Ala Ile Gly Ala Asn Ile Ser Gly Gly
   65                  70                  75                  80
   His Val Asn Pro Ala Val Thr Phe Gly Leu Ala Val Gly Gly Gln Ile
                   85                  90                  95
   Thr Val Ile Thr Gly Val Phe Tyr Trp Ile Ala Gln Leu Leu Gly Ser
               100                 105                 110
   Thr Ala Ala Cys Phe Leu Leu Lys Tyr Val Thr Gly Gly Leu Ala Val
           115                 120                 125
   Pro Thr His Ser Val Ala Ala Gly Leu Gly Ser Ile Glu Gly Val Val
       130                 135                 140
   Met Glu Ile Ile Ile Thr Phe Ala Leu Val Tyr Thr Val Tyr Ala Thr
   145                 150                 155                 160
   Ala Ala Asp Pro Lys Lys Gly Ser Leu Gly Thr Ile Ala Pro Leu Ala
                   165                 170                 175
   Ile Gly Leu Ile Val Gly Ala Asn Ile Leu Ala Ala Gly Pro Phe Ser
               180                 185                 190
   Gly Gly Ser
           195
<210> 65
   <211> 248
   <212> PRT
   <213> Gossypium hirsutum
<400> 65
   Met Ala Gly Ile Ala Phe Gly Arg Phe Asp Asp Ser Phe Ser Leu Gly
   1               5                   10                  15
   Thr Val Lys Ala Tyr Leu Ala Glu Phe Ile Ser Thr Leu Val Phe Val
               20                  25                  30
   Phe Ala Gly Val Gly Ser Ala Ile Ala Tyr Asn Lys Leu Thr Thr Asp
           35                  40                  45
   Ala Ala Leu Asp Pro Asp Gly Leu Val Ala Ile Ala Val Cys His Gly
       50                  55                  60
   Phe Ala Leu Phe Val Ala Val Ala Ile Gly Ala Asn Ile Ser Gly Gly
   65                  70                  75                  80
   His Val Asn Pro Ala Val Thr Phe Gly Leu Ala Leu Gly Gly Gln Ile
                   85                  90                  95
   Thr Ile Leu Thr Gly Ile Phe Tyr Trp Ile Ala Gln Leu Leu Gly Ser
               100                 105                 110
   Ile Val Ala Cys Phe Leu Leu Lys Ala Val Thr Gly Gly Leu Thr Val
           115                 120                 125
   Pro Ile His Gly Leu Gly Ala Gly Val Gly Ala Ile Gln Gly Val Val
       130                 135                 140
   Met Glu Ile Ile Ile Thr Phe Ala Leu Val Tyr Thr Val Tyr Ala Thr
   145                 150                 155                 160
   Ala Ala Asp Pro Lys Lys Gly Ser Leu Gly Thr Ile Ala Pro Ile Ala
                   165                 170                 175
   Ile Gly Phe Ile Val Gly Ala Asn Ile Leu Ala Ala Gly Pro Phe Ser
               180                 185                 190
   Gly Gly Ser Met Asn Pro Ala Arg Ser Phe Gly Pro Ala Val Ala Ser
           195                 200                 205
   Gly Asp Phe Asn Gly Ile Trp Ile Tyr Trp Val Gly Pro Leu Ile Gly
       210                 215                 220
   Gly Gly Leu Ser Gly Leu Ile Tyr Gly Asn Val Phe Met Asn Ser Asp
   225                 230                 235                 240
   His Ala Pro Leu Ser Asn Asp Phe
                   245
<210> 66
   <211> 250
   <212> PRT
   <213> Arabidopsis thaliana
<400> 66
   Met Ala Gly Val Ala Phe Gly Ser Phe Asp Asp Ser Phe Ser Leu Ala
   1               5                   10                  15
   Ser Leu Arg Ala Tyr Leu Ala Glu Phe Ile Ser Thr Leu Leu Phe Val
               20                  25                  30
   Phe Ala Gly Val Gly Ser Ala Ile Ala Tyr Ala Lys Leu Thr Ser Asp
           35                  40                  45
   Ala Ala Leu Asp Thr Pro Gly Leu Val Ala Ile Ala Val Cys His Gly
       50                  55                  60
   Phe Ala Leu Phe Val Ala Val Ala Ile Gly Ala Asn Ile Ser Gly Gly
   65                  70                  75                  80
   His Val Asn Pro Ala Val Thr Phe Gly Leu Ala Val Gly Gly Gln Ile
                   85                  90                  95
   Thr Val Ile Thr Gly Val Phe Tyr Trp Ile Ala Gln Leu Leu Gly Ser
               100                 105                 110
   Thr Ala Ala Cys Phe Leu Leu Lys Tyr Val Thr Gly Gly Leu Ala Val
           115                 120                 125
   Pro Thr His Ser Val Ala Ala Gly Leu Gly Ser Ile Glu Gly Val Val
       130                 135                 140
   Met Glu Ile Ile Ile Thr Phe Ala Leu Val Tyr Thr Val Tyr Ala Thr
   145                 150                 155                 160
   Ala Ala Asp Pro Lys Lys Gly Ser Leu Gly Thr Ile Ala Pro Leu Ala
                   165                 170                 175
   Ile Gly Leu Ile Val Gly Ala Asn Ile Leu Ala Ala Gly Pro Phe Ser
               180                 185                 190
   Gly Gly Ser Met Asn Pro Ala Arg Ser Phe Gly Pro Ala Val Ala Ala
           195                 200                 205
   Gly Asp Phe Ser Gly His Trp Val Tyr Trp Val Gly Pro Leu Ile Gly
       210                 215                 220
   Gly Glu Leu Ala Gly Leu Ile Tyr Gly Asn Val Phe Met Gly Ser Ser
   225                 230                 235                 240
   Glu His Val Pro Leu Ala Ser Ala Asp Phe
                   245                 250
<210> 67
   <211> 250
   <212> PRT
   <213> Arabidopsis thaliana
<400> 67
   Met Ala Gly Val Ala Phe Gly Ser Phe Asp Asp Ser Phe Ser Leu Ala
   1 5 10 15
   Ser Leu Arg Ala Tyr Leu Ala Glu Phe Ile Ser Thr Leu Leu Phe Val
   20 25 30
   Phe Ala Gly Val Gly Ser Ala Ile Ala Tyr Ala Lys Leu Thr Ser Asp
   35 40 45
   Ala Ala Leu Asp Thr Pro Gly Leu Val Ala Ile Ala Val Cys His Gly
   50 55 60
   Phe Ala Leu Phe Val Ala Val Ala Ile Gly Ala Asn Ile Ser Gly Gly
   65 70 75 80
   His Val Asn Pro Ala Val Thr Phe Gly Leu Ala Val Gly Gly Gln Ile
   85 90 95
   Thr Val Ile Thr Gly Val Phe Tyr Trp Ile Ala Gln Leu Leu Gly Ser
   100 105 110
   Thr Ala Ala Cys Phe Leu Leu Lys Tyr Val Thr Gly Gly Leu Ala Val
   115 120 125
   Pro Thr His Ser Val Ala Ala Gly Leu Gly Ser Ile Glu Gly Val Val
   130 135 140
   Met Glu Ile Ile Ile Thr Phe Ala Leu Val Tyr Thr Val Tyr Ala Thr
   145 150 155 160
   Ala Ala Asp Pro Lys Lys Gly Ser Leu Gly Thr Ile Ala Pro Leu Ala
   165 170 175
   Ile Gly Leu Ile Val Gly Ala Asn Ile Leu Ala Ala Gly Pro Phe Ser
   180 185 190
   Gly Gly Ser Met Asn Pro Ala Arg Ser Phe Gly Pro Ala Val Ala Ala
   195 200 205
   Gly Asp Phe Ser Gly His Trp Val Tyr Trp Val Gly Pro Leu Ile Gly
   210 215 220
   Gly Gly Leu Ala Gly Leu Ile Tyr Gly Asn Val Phe Met Gly Ser Ser
   225 230 235 240
   Glu His Val Pro Leu Ala Ser Ala Asp Phe
   245 250
<210> 68
   <211> 250
   <212> PRT
   <213> Arabidopsis thaliana
<400> 68
   Met Val Lys Ile Glu Ile Gly Ser Val Gly Asp Ser Phe Ser Val Ala
   1 5 10 15
   Ser Leu Lys Ala Tyr Leu Ser Glu Phe Ile Ala Thr Leu Leu Phe Val
   20 25 30
   Phe Ala Gly Val Gly Ser Ala Leu Ala Phe Ala Lys Leu Thr Ser Asp
   35 40 45
   Ala Ala Leu Asp Pro Ala Gly Leu Val Ala Val Ala Val Ala His Ala
   50 55 60
   Phe Ala Leu Phe Val Gly Val Ser Ile Ala Ala Asn Ile Ser Gly Gly
   65 70 75 80
   His Leu Asn Pro Ala Val Thr Leu Gly Leu Ala Val Gly Gly Asn Ile
   85 90 95
   Thr Val Ile Thr Gly Phe Phe Tyr Trp Ile Ala Gln Cys Leu Gly Ser
   100 105 110
   Ile Val Ala Cys Leu Leu Leu Val Phe Val Thr Asn Gly Glu Ser Val
   115 120 125
   Pro Thr His Gly Val Ala Ala Gly Leu Gly Ala Ile Glu Gly Val Val
   130 135 140
   Met Glu Ile Val Val Thr Phe Ala Leu Val Tyr Thr Val Tyr Ala Thr
   145 150 155 160
   Ala Ala Asp Pro Lys Lys Gly Ser Leu Gly Thr Ile Ala Pro Ile Ala
   165 170 175
   Ile Gly Phe Ile Val Gly Ala Asn Ile Leu Ala Ala Gly Pro Phe Ser
   180 185 190
   Gly Gly Ser Met Asn Pro Ala Arg Ser Phe Gly Pro Ala Val Val Ser
   195 200 205
   Gly Asp Phe Ser Gln Ile Trp Ile Tyr Trp Val Gly Pro Leu Val Gly
   210 215 220
   Gly Ala Leu Ala Gly Leu Ile Tyr Gly Asp Val Phe Ile Gly Ser Tyr
   225 230 235 240
   Ala Pro Ala Pro Thr Thr Glu Ser Tyr Pro
   245 250
<210> 69
   <211> 246
   <212> PRT
   <213> Nicotiana glauca
<400> 69
<210> 70
   <211> 248
   <212> PRT
   <213> Raphanus sativus
<400> 70
   Met Ala Gly Val Ala Phe Gly Ser Phe Asp Asp Ser Phe Ser Leu Ala
   1 5 10 15
   Ser Leu Arg Ala Tyr Leu Ala Glu Phe Ile Ser Thr Leu Leu Phe Val
   20 25 30
   Phe Ala Gly Val Gly Ser Ala Ile Ala Tyr Ala Lys Leu Thr Ser Asp
   35 40 45
   Ala Ala Leu Asp Thr Pro Gly Leu Val Ala Ile Ala Val Cys His Gly
   50 55 60
   Phe Ala Leu Phe Val Ala Val Ala Ile Gly Ala Asn Ile Ser Gly Gly
   65 70 75 80
   His Val Asn Pro Ala Val Thr Phe Gly Leu Ala Val Gly Gly Gln Ile
   85 90 95
   Thr Leu Ile Thr Gly Val Phe Tyr Trp Val Ala Gln Leu Leu Gly Ser
   100 105 110
   Thr Ala Ala Cys Phe Leu Leu Lys Tyr Val Thr Gly Gly Leu Ala Val
   115 120 125
   Pro Thr His Ser Val Ala Ala Gly Val Gly Ala Ile Glu Gly Val Val
   130 135 140
   Met Glu Ile Ile Ile Thr Phe Ser Leu Val Tyr Thr Val Tyr Pro Thr
   145 150 155 160
   Ala Ala Asp Pro Lys Lys Gly Ser Leu Gly Thr Ile Ala Pro Leu Ala
   165 170 175
   Ile Gly Leu Ile Val Gly Ala Asn Ile Leu Ala Ala Gly Pro Phe Ser
   180 185 190
   Gly Gly Ser Met Asn Pro Ala Arg Ser Phe Gly Pro Ala Val Ala Ala
   195 200 205
   Gly Asp Phe Ser Gly His Trp Val Tyr Trp Val Gly Pro Leu Ile Gly
   210 215 220
   Gly Gly Leu Ala Gly Val Thr Tyr Gly Asn Val Phe Met Thr Ser Glu
   225 230 235 240
   His Val Pro Leu Ala Ser Glu Phe
   245
<210> 71
   <211> 248
   <212> PRT
   <213> Helianthus annuus
<400> 71
   Met Pro Gly Ile Ala Phe Gly Ser Phe Asp Asp Ser Phe Ser Leu Ser
   1 5 10 15
   Ser Ile Lys Ser Tyr Val Ala Glu Phe Ile Ser Thr Leu Leu Phe Val
   20 25 30
   Phe Ala Gly Val Gly Ser Ala Ile Ala Phe Ala Lys Leu Thr Ala Asp
   35 40 45
   Ala Asp Leu Asp Pro Ala Gly Leu Val Ala Ile Ala Val Cys His Gly
   50 55 60
   Leu Ala Leu Phe Val Ala Val Ser Ile Ala Ala Asn Ile Ser Gly Gly
   65 70 75 80
   His Val Asn Pro Ala Val Thr Phe Gly Leu Ala Val Gly Gly Gln Ile
   85 90 95
   Thr Ile Leu Thr Gly Ile Phe Tyr Trp Ile Ala Gln Cys Ile Gly Ser
   100 105 110
   Ile Ala Ala Cys Tyr Leu Leu Ser Phe Val Thr Gly Gly Leu Ala Val
   115 120 125
   Pro Thr His Ala Val Ala Ala Gly Val Gly Ala Ile Gln Gly Val Val
   130 135 140
   Met Glu Ile Ile Ile Thr Phe Ala Leu Val Tyr Asn Val Tyr Ala Thr
   145 150 155 160
   Ala Val Asp Pro Lys Lys Gly Asp Leu Gly Thr Ile Ala Pro Leu Ala
   165 170 175
   Ile Gly Leu Ile Val Gly Ala Asn Ile Leu Ala Ala Gly Pro Phe Ser
   180 185 190
   Gly Gly Ser Met Asn Pro Ala Arg Ser Phe Gly Pro Ala Val Ala Ala
   195 200 205
   Gly Asp Phe Ser Gly His Trp Ile Tyr Trp Val Gly Pro Leu Val Gly
   210 215 220
   Gly Gly Leu Ala Gly Ala Ile Tyr Ser Asn Val Phe Ile Ser Asn Glu
   225 230 235 240
   His Ala Pro Leu Ser Ser Glu Phe
   245
<210> 72
   <211> 248
   <212> PRT
   <213> Oryza sativa
<400> 72
   Met Val Lys Leu Ala Phe Gly Ser Leu Gly Asp Ser Phe Ser Ala Thr
   1 5 10 15
   Ser Val Lys Ala Tyr Val Ala Glu Phe Ile Ala Thr Leu Leu Phe Val
   20 25 30
   Phe Ala Gly Val Gly Ser Ala Ile Ala Tyr Gly Gln Leu Thr Asn Gly
   35 40 45
   Gly Ala Leu Asp Pro Ala Gly Leu Val Ala Ile Ala Ile Ala His Ala
   50 55 60
   Leu Ala Leu Phe Val Gly Val Ser Val Ala Ala Asn Ile Ser Gly Gly
   65 70 75 80
   His Leu Asn Pro Ala Val Thr Phe Gly Leu Ala Val Gly Gly His Ile
   85 90 95
   Thr Ile Leu Thr Gly Leu Phe Tyr Trp Ile Ala Gln Leu Leu Gly Ala
   100 105 110
   Ser Ile Ala Cys Leu Leu Leu Lys Phe Val Thr His Gly Lys Ala Ile
   115 120 125
   Pro Thr His Gly Val Ala Gly Ile Ser Glu Leu Glu Gly Val Val Met
   130 135 140
   Glu Ile Val Ile Thr Phe Ala Leu Val Tyr Thr Val Tyr Ala Thr Ala
   145 150 155 160
   Ala Asp Pro Lys Lys Gly Ser Leu Gly Thr Ile Ala Pro Ile Ala Ile
   165 170 175
   Gly Phe Ile Val Gly Ala Asn Ile Leu Ala Ala Gly Pro Phe Ser Gly
   180 185 190
   Gly Ser Met Asn Pro Ala Arg Ser Phe Gly Pro Ala Val Ala Ala Gly
   195 200 205
   Asn Phe Ala Gly Asn Trp Val Tyr Trp Val Gly Pro Leu Ile Gly Gly
   210 215 220
   Gly Leu Ala Gly Leu Val Tyr Gly Asp Val Phe Ile Gly Ser Tyr Gln
   225 230 235 240
   Pro Val Ala Asp Gln Asp Tyr Ala
   245
<210> 73
   <211> 248
   <212> PRT
   <213> Zea mays
<400> 73
   Met Val Lys Leu Ala Phe Gly Ser Phe Arg Asp Ser Leu Ser Ala Ala
   1 5 10 15
   Ser Leu Lys Ala Tyr Val Ala Glu Phe Ile Ala Thr Leu Leu Phe Val
   20 25 30
   Phe Ala Gly Val Gly Ser Ala Ile Ala Tyr Ser Gln Leu Thr Lys Gly
   35 40 45
   Gly Ala Leu Asp Pro Ala Gly Leu Val Ala Ile Ala Ile Ala His Ala
   50 55 60
   Phe Ala Leu Phe Val Gly Val Ser Met Ala Ala Asn Ile Ser Gly Gly
   65 70 75 80
   His Leu Asn Pro Ala Val Thr Phe Gly Leu Ala Val Gly Gly His Ile
   85 90 95
   Thr Ile Leu Thr Gly Ile Leu Tyr Trp Val Ala Gln Leu Leu Gly Ala
   100 105 110
   Ser Val Ala Cys Phe Leu Leu Gln Tyr Val Thr His Gly Gln Ala Ile
   115 120 125
   Pro Thr His Gly Val Ser Gly Ile Ser Glu Ile Glu Gly Val Val Met
   130 135 140
   Glu Ile Val Ile Thr Phe Ala Leu Val Tyr Thr Val Tyr Ala Thr Ala
   145 150 155 160
   Ala Asp Pro Lys Lys Gly Ser Leu Gly Thr Ile Ala Pro Met Ala Ile
   165 170 175
   Gly Phe Ile Val Gly Ala Asn Ile Leu Ala Ala Gly Pro Phe Ser Gly
   180 185 190
   Gly Ser Met Asn Pro Ala Arg Ser Phe Gly Pro Ala Val Ala Ala Gly
   195 200 205
   Asn Phe Ala Gly Asn Trp Val Tyr Trp Val Gly Pro Leu Val Gly Gly
   210 215 220
   Gly Leu Ala Gly Leu Val Tyr Gly Asp Val Phe Ile Ala Ser Tyr Gln
   225 230 235 240
   Pro Val Gly Gln Gln Glu Tyr Pro
   245
<210> 74
   <211> 249
   <212> PRT
   <213> Hordeum vulgare
<400> 74
   Met Val Lys Leu Ala Phe Gly Ser Phe Gly Asp Ser Phe Ser Ala Thr
   1 5 10 15
   Ser Ile Arg Ser Tyr Val Ala Glu Phe Ile Ala Thr Leu Leu Phe Val
   20 25 30
   Phe Ala Gly Val Gly Ser Ala Ile Ser Tyr Gly Gln Leu Thr Gln Gly
   35 40 45
   Gly Ala Leu Asp Pro Ala Gly Leu Val Ala Ile Ala Ile Ala His Ala
   50 55 60
   Phe Ala Leu Phe Val Gly Val Ala Met Ala Ala Asn Ile Ser Gly Gly
   65 70 75 80
   His Leu Asn Pro Ala Val Thr Phe Gly Leu Ala Val Gly Gly His Val
   85 90 95
   Thr Ile Leu Thr Gly Leu Phe Tyr Trp Val Ala Gln Leu Leu Gly Ala
   100 105 110
   Ser Val Ala Cys Leu Leu Leu Gln Phe Val Thr His Ala Gln Ala Met
   115 120 125
   Pro Thr His Ala Val Ser Gly Ile Ser Glu Val Glu Gly Val Val Met
   130 135 140
   Glu Ile Val Ile Thr Phe Ala Leu Val Tyr Thr Val Tyr Ala Thr Ala
   145 150 155 160
   Ala Asp Pro Lys Lys Gly Ser Leu Gly Thr Ile Ala Pro Met Ala Ile
   165 170 175
   Gly Phe Ile Val Gly Ala Asn Ile Leu Ala Ala Gly Pro Phe Ser Gly
   180 185 190
   Gly Ser Met Asn Pro Ala Arg Ser Phe Gly Pro Ala Val Ala Ala Gly
   195 200 205
   Asn Phe Ser Gly His Trp Val Tyr Trp Val Gly Pro Leu Ile Gly Gly
   210 215 220
   Gly Leu Ala Gly Leu Val Tyr Gly Asp Val Phe Ile Ala Ser Tyr Gln
   225 230 235 240
   Pro Val Gly His Gln Gln Glu Tyr Pro
   245
<210> 75
   <211> 228
   <212> PRT
   <213> Antirrhinum majus
<400> 75
   Phe Gly Ser Ile Gly Asp Ser Phe Ser Val Ala Ser Ile Lys Ala Tyr
   1 5 10 15
   Val Ala Glu Phe Ile Ala Thr Leu Leu Phe Val Phe Ala Gly Val Gly
   20 25 30
   Ser Ala Ile Ala Tyr Asn Lys Leu Thr Ser Asp Ala Ala Leu Asp Pro
   35 40 45
   Ala Gly Leu Val Ala Val Ala Val Ala His Ala Phe Ala Leu Phe Val
   50 55 60
   Gly Val Ser Met Ala Ala Asn Val Ser Gly Gly His Leu Asn Pro Ala
   65 70 75 80
   Val Thr Leu Gly Leu Ala Val Gly Gly Asn Ile Thr Ile Leu Thr Gly
   85 90 95
   Leu Phe Tyr Trp Ile Ala Gln Cys Leu Gly Ser Thr Val Ala Cys Leu
   100 105 110
   Leu Leu Lys Phe Val Thr Asn Gly Leu Ser Val Pro Thr His Gly Val
   115 120 125
   Ala Ala Gly Met Asp Ala Ile Gln Gly Val Val Met Glu Ile Ile Ile
   130 135 140
   Thr Phe Ala Leu Val Tyr Thr Val Tyr Ala Thr Ala Ala Asp Pro Lys
   145 150 155 160
   Lys Gly Ser Leu Gly Val Ile Ala Pro Ile Ala Ile Gly Phe Ile Val
   165 170 175
   Gly Ala Asn Ile Leu Ala Ala Gly Pro Phe Ser Gly Gly Ser Met Asn
   180 185 190
   Pro Ala Arg Ser Phe Gly Pro Ala Val Ala Ser Gly Asp Phe Ser Gln
   195 200 205
   Asn Trp Ile Tyr Trp Ala Gly Pro Leu Ile Gly Gly Ala Leu Ala Gly
   210 215 220
   Phe Ile Tyr Gly
   225
<210> 76
   <211> 248
   <212> PRT
   <213> Zea mays
<400> 76
   Met Val Lys Leu Ala Phe Gly Ser Phe Arg Asp Ser Leu Ser Ala Ala
   1 5 10 15
   Ser Leu Lys Ala Tyr Val Ala Glu Phe Ile Ala Thr Leu Leu Phe Val
   20 25 30
   Phe Ala Gly Val Gly Ser Ala Ile Ala Tyr Ser Gln Leu Thr Lys Gly
   35 40 45
   Gly Ala Leu Asp Pro Ala Gly Leu Val Ala Ile Ala Ile Ala His Ala
   50 55 60
   Phe Ala Leu Phe Val Gly Val Ser Met Ala Ala Asn Ile Ser Gly Gly
   65 70 75 80
   His Leu Asn Pro Ala Val Thr Phe Gly Leu Ala Val Gly Gly His Ile
   85 90 95
   Thr Ile Leu Thr Gly Ile Leu Tyr Trp Val Ala Gln Leu Leu Gly Ala
   100 105 110
   Ser Val Ala Cys Phe Leu Leu Gln Tyr Val Thr His Gly Gln Ala Ile
   115 120 125
   Pro Thr His Gly Val Ser Gly Ile Ser Glu Ile Glu Gly Val Val Met
   130 135 140
   Glu Ile Val Ile Thr Phe Ala Leu Val Tyr Thr Val Tyr Ala Thr Ala
   145 150 155 160
   Ala Asp Pro Lys Lys Gly Ser Leu Gly Thr Ile Ala Pro Met Ala Ile
   165 170 175
   Gly Phe Ile Val Gly Ala Asn Ile Leu Ala Ala Gly Pro Phe Ser Gly
   180 185 190
   Gly Ser Met Asn Pro Ala Arg Ser Phe Gly Pro Ala Val Ala Ala Gly
   195 200 205
   Asn Phe Ala Gly Asn Trp Val Tyr Trp Val Gly Pro Leu Val Gly Gly
   210 215 220
   Gly Leu Ala Gly Pro Val Tyr Gly Asp Val Phe Ile Ala Ser Tyr Gln
   225 230 235 240
   Pro Val Gly Gln Gln Glu Tyr Pro
   245
<210> 77
   <211> 248
   <212> PRT
   <213> Helianthus annuus
<400> 77
   Met Pro Gly Ile Ala Phe Gly Ser Phe Asp Asp Ser Phe Ser Ser Ser
   1 5 10 15
   Ser Ile Lys Ala Tyr Ile Ala Glu Phe Ile Ser Thr Leu Leu Phe Val
   20 25 30
   Phe Ala Gly Val Gly Ser Ala Ile Ala Tyr Ala Lys Leu Thr Ala Asp
   35 40 45
   Ala Ala Leu Asp Pro Pro Gly Leu Val Ala Val Ala Val Cys His Gly
   50 55 60
   Phe Ala Leu Phe Val Ala Val Cys Ile Ala Ala Asn Ile Cys Gly Gly
   65 70 75 80
   His Val Asn Pro Ala Val Thr Leu Gly Leu Ala Val Gly Gly Gln Ile
   85 90 95
   Thr Phe Leu Thr Gly Leu Phe Tyr Trp Ile Ala Gln Leu Leu Gly Ser
   100 105 110
   Thr Val Ala Cys Phe Leu Leu Ser Phe Val Thr Gly Gly Leu Ala Val
   115 120 125
   Pro Thr His Gly Val Ala Glu Gly Val Gly Thr Ile Gln Gly Val Val
   130 135 140
   Phe Glu Ile Ile Ile Thr Phe Ala Met Val Tyr Thr Val Tyr Ala Thr
   145 150 155 160
   Ala Cys Asp Pro Lys Lys Gly Ala Leu Gly Thr Ile Ala Pro Ile Ala
   165 170 175
   Ile Gly Phe Ile Val Gly Ala Asn Ile Leu Ala Ala Gly Pro Phe Ser
   180 185 190
   Gly Gly Ser Met Asn Pro Ala Arg Ser Phe Gly Pro Ala Val Ala Ser
   195 200 205
   Phe Asp Phe Ser Gly His Trp Ile Tyr Trp Val Gly Pro Leu Val Gly
   210 215 220
   Gly Gly Leu Ala Gly Leu Ile Tyr Pro Asn Val Phe Ile Ser Asn Glu
   225 230 235 240
   His Ile Pro Leu Thr Asn Glu Tyr
   245
<210> 78
   <211> 248
   <212> PRT
   <213> Helianthus annuus
<400> 78
<210a 79
   <211a 248
   <212a PRT
   <213> Vernicia fordii
<400> 79
<210> 80
   <211> 248
   <212> PRT
   <213> Daucus carota
<400> 80
   Met Val Lys Leu Ala Ile Gly Ser Val Gly Asp Ser Phe Ser Ala Val
   1               5                   10                  15
   Ser Leu Lys Ser Tyr Leu Ala Glu Phe Ile Ala Thr Leu Leu Phe Val
               20                  25                  30
   Phe Ala Gly Val Gly Ser Ala Ile Ala Tyr Gly His Leu Thr Ala Asp
           35                  40                  45
   Ala Ala Leu Asp Pro Ala Gly Leu Val Ala Ile Ala Ile Ala His Ala
       50                  55                  60
   Phe Ala Leu Phe Val Gly Val Ser Met Ala Ala Asn Ile Ser Gly Gly
   65                  70                  75                  80
   His Leu Asn Pro Ala Val Thr Leu Gly Leu Ala Ile Gly Gly Asn Ile
                   85                  90                  95
   Thr Ile Ile Thr Gly Leu Phe Tyr Trp Ile Ala Gln Cys Leu Gly Ser
               100                 105                 110
   Thr Val Ala Cys Phe Leu Leu Lys Phe Val Thr Ala Gly Lys Ala Ile
           115                 120                 125
   Pro Thr His Gly Val Gly Ala Gly Leu Gly Ala Ala Glu Gly Val Val
       130                 135                 140
   Phe Glu Ile Val Ile Thr Phe Ala Leu Val Tyr Thr Val Tyr Ala Thr
   145                 150                 155                 160
   Ala Ala Asp Pro Lys Lys Gly Ser Leu Gly Thr Ile Ala Pro Ile Ala
                   165                 170                 175
   Ile Gly Phe Ile Val Gly Ala Asn Ile Leu Ala Ala Gly Pro Phe Ser
               180                 185                 190
   Gly Gly Ser Met Asn Pro Ala Arg Ser Phe Gly Pro Ala Val Ala Ser
           195                 200                 205
   Phe Asp Phe Ser Gly His Trp Ile Tyr Trp Val Gly Pro Leu Ile Gly
       210                 215                 220
   Gly Gly Leu Ala Gly Leu Val Tyr Gly Asp Val Phe Ile Gly Ser Tyr
   225                 230                 235                 240
   Ala Pro Ile Ala Glu Asp Tyr Ala
                   245
<210> 81
   <211> 250
   <212> PRT
   <213> Antirrhinum majus
<400> 81
   Met Val Lys Ile Ala Phe Gly Ser Ile Gly Asp Ser Phe Ser Val Ala
   1 5 10 15
   Ser Ile Lys Ala Tyr Val Ala Glu Phe Ile Ala Thr Leu Leu Phe Val
   20 25 30
   Phe Ala Gly Val Gly Ser Ala Ile Ala Tyr Asn Lys Leu Thr Ser Asp
   35 40 45
   Ala Ala Leu Asp Pro Ala Gly Leu Val Ala Val Ala Val Ala His Ala
   50 55 60
   Phe Ala Leu Phe Val Gly Val Ser Met Ala Ala Asn Val Ser Gly Gly
   65 70 75 80
   His Leu Asn Pro Ala Val Thr Leu Gly Leu Ala Val Gly Gly Asn Ile
   85 90 95
   Thr Ile Leu Thr Gly Leu Phe Tyr Trp Ile Ala Gln Cys Leu Gly Ser
   100 105 110
   Thr Val Ala Cys Leu Leu Leu Lys Phe Val Thr Asn Gly Leu Ser Val
   115 120 125
   Pro Thr His Gly Val Ala Ala Gly Met Asp Ala Ile Gln Gly Val Val
   130 135 140
   Met Glu Ile Ile Ile Thr Phe Ala Leu Val Tyr Thr Val Tyr Ala Thr
   145 150 155 160
   Ala Ala Asp Pro Lys Lys Gly Ser Leu Gly Val Ile Ala Pro Ile Ala
   165 170 175
   Ile Gly Phe Ile Val Gly Ala Asn Ile Leu Ala Ala Gly Pro Phe Ser
   180 185 190
   Gly Gly Ser Met Asn Pro Ala Arg Ser Phe Gly Pro Ala Val Ala Ser
   195 200 205
   Gly Asp Phe Ser Gln Asn Trp Ile Tyr Trp Ala Gly Pro Leu Ile Gly
   210 215 220
   Gly Ala Leu Ala Gly Phe Ile Tyr Gly Asp Val Phe Ile Thr Ala His
   225 230 235 240
   Ala Pro Leu Pro Thr Ser Glu Asp Tyr Ala
   245 250
<210> 82
   <211> 250
   <212> PRT
   <213> Nicotiana tabacum
<400> 82
   Met Val Arg Ile Ala Phe Gly Ser Ile Gly Asp Ser Phe Ser Val Gly
   1 5 10 15
   Ser Leu Lys Ala Tyr Val Ala Glu Phe Ile Ala Thr Leu Leu Phe Val
   20 25 30
   Phe Ala Gly Val Gly Ser Ala Ile Ala Tyr Asn Lys Leu Thr Ala Asp
   35 40 45
   Ala Ala Leu Asp Pro Ala Gly Leu Val Ala Val Ala Val Ala His Ala
   50 55 60
   Phe Ala Leu Phe Val Gly Val Ser Ile Ala Ala Asn Ile Ser Gly Gly
   65 70 75 80
   His Leu Asn Pro Ala Val Thr Leu Gly Leu Ala Val Gly Gly Asn Ile
   85 90 95
   Thr Ile Leu Thr Gly Phe Phe Tyr Trp Ile Ala Gln Leu Leu Gly Ser
   100 105 110
   Thr Val Ala Cys Leu Leu Leu Lys Tyr Val Thr Asn Gly Leu Ala Val
   115 120 125
   Pro Thr His Gly Val Ala Ala Gly Leu Asn Gly Leu Gln Gly Val Val
   130 135 140
   Met Glu Ile Ile Ile Thr Phe Ala Leu Val Tyr Thr Val Tyr Ala Thr
   145 150 155 160
   Ala Ala Asp Pro Lys Lys Gly Ser Leu Gly Thr Ile Ala Pro Ile Ala
   165 170 175
   Ile Gly Phe Ile Val Gly Ala Asn Ile Leu Ala Ala Gly Pro Phe Ser
   180 185 190
   Gly Gly Ser Met Asn Pro Ala Arg Ser Phe Gly Pro Ala Val Val Ala
   195 200 205
   Gly Asp Phe Ser Gln Asn Trp Ile Tyr Trp Ala Gly Pro Leu Ile Gly
   210 215 220
   Gly Gly Leu Ala Gly Phe Ile Tyr Gly Asp Val Phe Ile Gly Cys His
   225 230 235 240
   Thr Pro Leu Pro Thr Ser Glu Asp Tyr Ala
   245 250
<210> 83
   <211> 250
   <212> PRT
   <213> Helianthus annuus
<400> 83
   Met Val Lys Leu Ala Ile Gly Ser Ile Gly Asp Ser Leu Ser Ala Gly
   1               5                   10                  15
   Ser Ile Lys Ser Tyr Leu Ala Glu Phe Ile Ala Thr Leu Leu Phe Val
               20                  25                  30
   Phe Ala Gly Val Gly Ser Ala Ile Ala Tyr Gly Lys Leu Thr Thr Asp
           35                  40                  45
   Ala Ala Leu Asp Pro Ala Gly Leu Val Ala Ile Ala Ile Ala His Ala
       50                  55                  60
   Phe Ala Leu Phe Val Gly Val Ser Met Ala Ala Asn Ile Ser Gly Gly
   65                  70                  75                  80
   His Leu Asn Pro Ala Val Thr Phe Gly Leu Ala Ile Gly Gly Asn Ile
                   85                  90                  95
   Thr Ile Ile Thr Gly Leu Phe Tyr Trp Ile Ala Gln Cys Leu Gly Ser
               100                 105                 110
   Ile Val Ala Cys Phe Leu Leu Gln Phe Val Thr Gly Gly Leu Ala Val
           115                 120                 125
   Pro Thr His Gly Val Ala Asp Gly Met Asn Gly Val Gln Gly Val Val
       130                 135                 140
   Met Glu Ile Ile Ile Thr Phe Ala Leu Val Tyr Thr Val Tyr Ala Thr
   145                 150                 155                 160
   Ala Val Asp Pro Lys Lys Gly Ser Leu Gly Thr Ile Ala Pro Met Ala
                   165                 170                 175
   Ile Gly Phe Ile Val Gly Ala Asn Ile Leu Ala Ala Gly Pro Phe Ser
               180                 185                 190
   Gly Gly Ser Met Asn Pro Ala Arg Ser Phe Gly Pro Ala Val Val Ser
           195                 200                 205
   Gly Asp Phe Ser Gln Asn Trp Ile Tyr Trp Val Gly Pro Leu Ile Gly
       210                 215                 220
   Gly Gly Leu Ala Gly Phe Ile Phe Gly Asp Val Phe Ile Gly Ser Tyr
   225                 230                 235                 240
   Glu Thr Leu Pro Asp Ser Gly Asp Tyr Ala
                   245                 250
<210> 84
   <211> 250
   <212> PRT
   *<213> Solanum tuberosum
<400> 84
   Met Val Arg Ile Ala Phe Gly Ser Ile Gly Asp Ser Leu Ser Val Gly
   1 5 10 15
   Ser Leu Lys Ala Tyr Leu Ala Glu Phe Ile Ala Thr Leu Leu Phe Val
   20 25 30
   Phe Ala Gly Val Gly Ser Ala Ile Ala Tyr Asn Lys Leu Thr Ser Asp
   35 40 45
   Ala Ala Leu Asp Pro Ala Gly Leu Val Ala Ile Ala Val Ala His Ala
   50 55 60
   Phe Ala Leu Phe Val Gly Val Ser Met Ala Ala Asn Ile Ser Gly Gly
   65 70 75 80
   His Leu Asn Pro Ala Val Thr Leu Gly Leu Ala Val Gly Gly Asn Ile
   85 90 95
   Thr Thr Leu Thr Gly Leu Phe Tyr Trp Val Ala Gln Leu Leu Gly Ser
   100 105 110
   Thr Val Ala Cys Leu Leu Leu Lys Tyr Val Thr Asn Gly Leu Ala Val
   115 120 125
   Pro Thr His Gly Val Ala Ala Gly Met Asn Gly Ala Glu Gly Val Val
   130 135 140
   Met Glu Ile Val Ile Thr Phe Ala Leu Val Tyr Thr Val Tyr Ala Thr
   145 150 155 160
   Ala Ala Asp Pro Lys Lys Gly Ser Leu Gly Thr Ile Ala Pro Ile Ala
   165 170 175
   Ile Gly Phe Ile Val Gly Ala Asn Ile Leu Ala Ala Gly Pro Phe Ser
   180 185 190
   Gly Gly Ser Met Asn Pro Ala Arg Ser Phe Gly Pro Ala Val Val Ala
   195 200 205
   Gly Asp Phe Ser Gln Asn Trp Ile Tyr Trp Val Gly Pro Leu Ile Gly
   210 215 220
   Gly Gly Leu Ala Gly Phe Ile Tyr Gly Asp Val Phe Ile Gly Ser His
   225 230 235 240
   Thr Pro Leu Pro Thr Ser Glu Asp Tyr Ala
   245 250
<210> 85
   <211> 250
   <212> PRT
   <213> Nicotiana tabacum
<400> 85
   Met Val Leu Ile Ala Phe Gly Ser Ile Gly Asp Ser Phe Ser Val Gly
   1 5 10 15
   Ser Leu Lys Ala Tyr Val Ala Glu Phe Ile Ala Thr Leu Leu Phe Val
   20 25 30
   Phe Ala Gly Val Gly Ser Ala Ile Ala Tyr Asn Lys Leu Thr Ala Asp
   35 40 45
   Ala Ala Leu Asp Pro Ala Gly Leu Val Ala Val Ala Val Ala His Ala
   50 55 60
   Phe Ala Leu Phe Val Gly Val Ser Ile Ala Ala Asn Ile Ser Gly Gly
   65 70 75 80
   His Leu Asn Pro Ala Val Thr Leu Gly Leu Ala Val Gly Gly Asn Ile
   85 90 95
   Thr Ile Leu Thr Gly Phe Phe Tyr Trp Ile Ala Gln Leu Leu Gly Ser
   100 105 110
   Thr Val Ala Cys Leu Leu Leu Lys Tyr Val Thr Asn Gly Leu Ala Val
   115 120 125
   Pro Thr His Gly Val Ala Ala Gly Leu Asn Gly Phe Gln Gly Val Val
   130 135 140
   Met Glu Ile Ile Ile Thr Phe Ala Leu Val Tyr Thr Val Tyr Ala Thr
   145 150 155 160
   Ala Ala Asp Pro Lys Lys Gly Ser Leu Gly Thr Ile Ala Pro Ile Ala
   165 170 175
   Ile Gly Phe Ile Val Gly Ala Asn Ile Leu Ala Ala Gly Pro Phe Ser
   180 185 190
   Gly Gly Ser Met Asn Pro Ala Arg Ser Phe Gly Pro Ala Val Val Ala
   195 200 205
   Gly Asp Phe Ser Gln Asn Trp Ile Tyr Trp Ala Gly Pro Leu Ile Gly
   210 215 220
   Gly Gly Leu Ala Gly Phe Ile Tyr Gly Asp Val Phe Ile Gly Cys His
   225 230 235 240
   Thr Pro Leu Pro Thr Ser Glu Asp Tyr Ala
   245 250
<210> 86
   <211> 250
   <212> PRT
   <213> Lycopersicon esculentum
<400> 86
   Met Val Arg Ile Ala Phe Gly Ser Ile Gly Asp Ser Leu Ser Val Gly
   1               5                   10                  15
   Ser Leu Lys Ala Tyr Leu Ala Glu Phe Ile Ala Thr Leu Leu Phe Val
               20                  25                  30
   Phe Ala Gly Val Gly Ser Ala Ile Ala Phe Asn Lys Leu Thr Ser Gly
           35                  40                  45
   Ala Ala Leu Asp Pro Ala Gly Leu Val Ala Ile Ala Val Ala His Ala
       50                  55                  60
   Phe Ala Leu Phe Val Gly Val Ser Met Ala Ala Asn Ile Ser Gly Gly
   65                  70                  75                  80
   His Leu Asn Pro Ala Val Thr Leu Gly Leu Ala Val Gly Gly Asn Ile
                   85                  90                  95
   Thr Ile Leu Thr Gly Leu Phe Tyr Trp Val Ala Gln Leu Leu Gly Ser
               100                 105                 110
   Thr Val Ala Cys Leu Leu Leu Lys Tyr Val Thr Asn Gly Leu Ala Val
           115                 120                 125
   Pro Thr His Gly Val Ala Ala Gly Met Ser Gly Ala Glu Gly Val Val
       130                 135                 140
   Met Glu Ile Val Ile Thr Phe Ala Leu Val Tyr Thr Val Tyr Ala Thr
   145                 150                 155                 160
   Ala Ala Asp Pro Lys Lys Gly Ser Leu Gly Thr Ile Ala Pro Ile Ala
                   165                 170                 175
   Ile Gly Phe Ile Val Gly Ala Asn Ile Leu Ala Ala Gly Pro Phe Ser
               180                 185                 190
   Gly Gly Ser Met Asn Pro Ala Arg Ser Phe Gly Pro Ala Val Val Ala
           195                 200                 205
   Gly Asp Phe Ser Gln Asn Trp Ile Tyr Trp Val Gly Pro Leu Ile Gly
       210                 215                 220
   Gly Gly Leu Ala Gly Phe Ile Tyr Gly Asp Val Phe Ile Gly Cys His
   225                 230                 235                 240
   Thr Pro Leu Pro Thr Ser Glu Asp Tyr Ala
                   245                 250
<210> 87
   <211> 234
   <212> PRT
   <213> Arabidopsis thaliana
<210> 88
   <211> 234
   <212> PRT
   <213> Arabidopsis thaliana
<400> 88
   Met Val Ser Pro Glu Asn Ala Asn Trp Ile Cys Asp Leu Ile Asp Ala
   1               5                   10                  15
   Asp Tyr Gly Ser Phe Thr Ile Gln Gly Pro Gly Phe Ser Trp Pro Val
               20                  25                  30
   Gln Gln Pro Ile Gly Val Ser Ser Asn Ser Ser Ala Gly Val Asp Gly
           35                  40                  45
   Ser Ala Gly Asn Ser Glu Ala Ser Lys Glu Pro Gly Ser Lys Lys Arg
       50                  55                  60
   Gly Arg Cys Glu Ser Ser Ser Ala Thr Ser Ser Lys Ala Cys Arg Glu
   65                  70                  75                  80
   Lys Gln Arg Arg Asp Arg Leu Asn Asp Lys Phe Met Glu Leu Gly Ala
                   85                  90                  95
   Ile Leu Glu Pro Gly Asn Pro Pro Lys Thr Asp Lys Ala Ala Ile Leu
               100                 105                 110
   Val Asp Ala Val Arg Met Val Thr Gln Leu Arg Gly Glu Ala Gln Lys
           115                 120                 125
   Leu Lys Asp Ser Asn Ser Ser Leu Gln Asp Lys Ile Lys Glu Leu Lys
       130                 135                 140
   Thr Glu Lys Asn Glu Leu Arg Asp Glu Lys Gln Arg Leu Lys Thr Glu
   145                 150                 155                 160
   Lys Glu Lys Leu Glu Gln Gln Leu Lys Ala Ile Asn Ala Pro Gln Pro
                   165                 170                 175
   Ser Phe Phe Pro Ala Pro Pro Met Met Pro Thr Ala Phe Ala Ser Ala
               180                 185                 190
   Gln Gly Gln Ala Pro Gly Asn Lys Met Val Pro Ile Ile Ser Tyr Pro
           195                 200                 205
   Gly Val Ala Met Trp Gln Phe Met Pro Pro Ala Ser Val Asp Thr Ser
       210                 215                 220
   Gln Asp His Val Leu Arg Pro Pro Val Ala
   225                 230
<210> 89
   <211> 216
   <212> PRT
   <213> Oryza sativa
<400> 89
   Cys Gly Ala Phe Pro Trp Asp Ala Ser Pro Ser Cys Ser Asn Pro Ser
   1               5                   10                  15
   Val Glu Val Ser Ser Tyr Val Asn Thr Thr Ser Tyr Val Leu Lys Glu
               20                  25                  30
   Pro Gly Ser Asn Lys Arg Val Arg Ser Gly Ser Cys Gly Arg Pro Thr
   35 40 45
   Ser Lys Ala Ser Arg Glu Lys Ile Arg Arg Asp Lys Met Asn Asp Arg
   50 55 60
   Phe Leu Glu Leu Gly Thr Thr Leu Glu Pro Gly Lys Pro Val Lys Ser
   65 70 75 80
   Asp Lys Ala Ala Ile Leu Ser Asp Ala Thr Arg Met Val Ile Gln Leu
   85 90 95
   Arg Ala Glu Ala Lys Gln Leu Lys Asp Thr Asn Glu Ser Leu Glu Asp
   100 105 110
   Lys Ile Lys Glu Leu Lys Ala Glu Lys Asp Glu Leu Arg Asp Glu Lys
   115 120 125
   Gln Lys Leu Lys Val Glu Lys Glu Thr Leu Glu Gln Gln Val Lys Ile
   130 135 140
   Leu Thr Ala Thr Pro Ala Tyr Met Pro His Pro Thr Leu Met Pro Ala
   145 150 155 160
   Pro Tyr Pro Gln Ala Pro Leu Ala Pro Phe His His Ala Gln Gly Gln
   165 170 175
   Ala Ala Gly Gln Lys Leu Met Met Pro Phe Val Gly Tyr Pro Gly Tyr
   180 185 190
   Pro Met Trp Gln Phe Met Pro Pro Ser Glu Val Asp Thr Ser Lys Asp
   195 200 205
   Ser Glu Ala Cys Pro Pro Val Ala
   210 215
<210> 90
   <211> 226
   <212> PRT
   <213> Arabidopsis thaliana
<400> 90
   Met Val Ser Pro Glu Asn Thr Asn Trp Leu Ser Asp Tyr Pro Leu Ile
   1 5 10 15
   Glu Gly Ala Phe Ser Asp Gln Asn Pro Thr Phe Pro Trp Gln Ile Asp
   20 25 30
   Gly Ser Ala Thr Val Ser Val Glu Val Asp Gly Phe Leu Cys Asp Ala
   35 40 45
   Asp Val Ile Lys Glu Pro Ser Ser Arg Lys Arg Ile Lys Thr Glu Ser
   50 55 60
   Cys Thr Gly Ser Asn Ser Lys Ala Cys Arg Glu Lys Gln Arg Arg Asp
   65 70 75 80
   Arg Leu Asn Asp Lys Phe Thr Glu Leu Ser Ser Val Leu Glu Pro Gly
   85 90 95
   Arg Thr Pro Lys Thr Asp Lys Val Ala Ile Ile Asn Asp Ala Ile Arg
   100 105 110
   Met Val Asn Gln Ala Arg Asp Glu Ala Gln Lys Leu Lys Asp Leu Asn
   115 120 125
   Ser Ser Leu Gln Glu Lys Ile Lys Glu Leu Lys Asp Glu Lys Asn Glu
   130 135 140
   Leu Arg Asp Glu Lys Gln Lys Leu Lys Val Glu Lys Glu Arg Ile Asp
   145 150 155 160
   Gln Gln Leu Lys Ala Ile Lys Thr Gln Pro Gln Pro Gln Pro Cys Phe
   165 170 175
   Leu Pro Asn Pro Gln Thr Leu Ser Gln Ala Gln Ala Pro Gly Ser Lys
   180 185 190
   Leu Val Pro Phe Thr Thr Tyr Pro Gly Phe Ala Met Trp Gln Phe Met
   195 200 205
   Pro Pro Ala Ala Val Asp Thr Ser Gln Asp His Val Leu Arg Pro Pro
   210 215 220
   Val Ala
   225
<210> 91
   <211> 256
   <212> PRT
   <213> Oryza sativa
<400> 91
   Met Ala Ser Pro Glu Gly Ser Thr Trp Val Phe Asp Cys Pro Leu Met
   1               5                   10                  15
   Asp Asp Leu Ala Ala Ala Ala Gly Phe Asp Ala Ala Pro Ala Gly Gly
               20                  25                  30
   Phe Tyr Trp Thr Thr Pro Ala Pro Pro Gln Ala Ala Leu Gln Pro Pro
           35                  40                  45
   Pro Pro Gln Gln Gln Pro Val Ala Pro Ala Thr Ala Ala Pro Asn Ala
       50                  55                  60
   Cys Ala Glu Ile Asn Gly Ser Val Asp Cys Glu His Gly Lys Glu Gln
   65                  70                  75                  80
   Pro Thr Asn Lys Arg Pro Arg Ser Glu Ser Gly Thr Arg Pro Ser Ser
                   85                  90                  95
   Lys Ala Cys Arg Glu Lys Val Arg Arg Asp Lys Leu Asn Glu Arg Phe
               100                 105                 110
   Leu Glu Leu Gly Ala Val Leu Glu Pro Gly Lys Thr Pro Lys Met Asp
           115                 120                 125
   Lys Ser Ser Ile Leu Asn Asp Ala Ile Arg Val Met Ala Glu Leu Arg
       130                 135                 140
   Ser Glu Ala Gln Lys Leu Lys Glu Ser Asn Glu Ser Leu Gln Glu Lys
   145                 150                 155                 160
   Ile Lys Glu Leu Lys Ala Glu Lys Asn Glu Leu Arg Asp Glu Lys Gln
                   165                 170                 175
   Lys Leu Lys Ala Glu Lys Glu Ser Leu Glu Gln Gln Ile Lys Phe Leu
               180                 185                 190
   Asn Ala Arg Pro Ser Phe Val Pro His Pro Pro Val Ile Pro Ala Ser
           195                 200                 205
   Ala Phe Thr Ala Pro Gln Gly Gln Ala Ala Gly Gln Lys Leu Met Met
       210                 215                 220
   Pro Val Ile Gly Tyr Pro Gly Phe Pro Met Trp Gln Phe Met Pro Pro
   225                 230                 235                 240
   Ser Asp Val Asp Thr Thr Asp Asp Thr Lys Ser Cys Pro Pro Val Ala
                   245                 250                 255
<210> 92
   <211> 253
   <212> PRT
   <213> Oryza sativa
<400> 92
   Met Ser Gly Thr Pro Ala Asp Gly Gly Gly Gly Gly Gly Gly Gly Gly
   1 5 10 15
   Gly Gly Ser Gly Asp Asp Trp Phe Leu Asp Cys Gly Ile Leu Glu Asp
   20 25 30
   Leu Pro Ala Ala Ala Cys Gly Ala Phe Pro Trp Asp Ala Ser Pro Ser
   35 40 45
   Cys Ser Asn Pro Ser Val Glu Val Ser Ser Tyr Val Asn Thr Thr Ser
   50 55 60
   Tyr Val Leu Lys Glu Pro Gly Ser Asn Lys Arg Val Arg Ser Gly Ser
   65 70 75 80
   Cys Gly Arg Pro Thr Ser Lys Ala Ser Arg Glu Lys Ile Arg Arg Asp
   85 90 95
   Lys Met Asn Asp Arg Phe Leu Glu Leu Gly Thr Thr Leu Glu Pro Gly
   100 105 110
   Lys Pro Val Lys Ser Asp Lys Ala Ala Ile Leu Ser Asp Ala Thr Arg
   115 120 125
   Met Val Ile Gln Leu Arg Ala Glu Ala Lys Gln Leu Lys Asp Thr Asn
   130 135 140
   Glu Ser Leu Glu Asp Lys Ile Lys Glu Leu Lys Ala Glu Lys Asp Glu
   145 150 155 160
   Leu Arg Asp Glu Lys Gln Lys Leu Lys Val Glu Lys Glu Thr Leu Glu
   165 170 175
   Gln Gln Val Lys Ile Leu Thr Ala Thr Pro Ala Tyr Met Pro His Pro
   180 185 190
   Thr Leu Met Pro Ala Pro Tyr Pro Gln Ala Pro Leu Ala Pro Phe His
   195 200 205
   His Ala Gln Gly Gln Ala Ala Gly Gln Lys Leu Met Met Pro Phe Val
   210 215 220
   Gly Tyr Pro Gly Tyr Pro Met Trp Gln Phe Met Pro Pro Ser Glu Val
   225 230 235 240
   Asp Thr Ser Lys Asp Ser Glu Ala Cys Pro Pro Val Ala
   245 250